# EUROPEAN PATENT APPLICATION

(11) **EP 1 921 142 A1**
(43) Date of publication of application: **14.05.2008**
(21) Application number: 06123620.4
(22) Date of filing: 07.11.2006
(51) Int. Cl.: C12N 15/10, C07K 16/00, C12N 15/86, C07K 16/08, C07K 16/10

(54) **Selection of human monoclonal antibodies by eukaryotic cell display**

(71) Applicant: Cytos Biotechnology AG, 8952 Schlieren (CH)
(72) Inventor: Bachmann, Martin, Dr., 8472 Seuzach (CH); Bauer, Monika, Dr., 8048 Zürich (CH); Beerli, Roger, Dr., 8106 Adlikon b. Regensdorf (CH)
(74) Representative: Sperrle, Martin

(57) **Abstract**

The application provides a method of isolating a eukaryotic cell expressing an antibody of desired specificity, preferably a monoclonal single chain antibody (scFv). The application further provides methods which allow to clone the variable regions of said antibody from that isolated eukaryotic cell and to recombinantly produce antibodies comprising said variable regions as fusion protein with a purification tag, eg. as Fc-fusion, as a Fab fragment, or as whole antibodies, such as IgG, IgE, IgD, IgA and IgM. Said methods also allow to recombinantly produce antibodies with desired specificity in a fully species specific form, preferably as fully human antibodies.

SEQUENCE LISTING

<110>  Cytos Biotechnology AG
       Bachmann, Martin
       Bauer, Monika
       Beerli, Roger

<120>  SELECTION OF HUMAN MONOCLONAL ANTIBODIES BY EUKARYOTIC CELL
       DISPLAY

<130>  P1060US00

<160>  124

<170>  PatentIn version 3.3

<210>  1
<211>  285
<212>  DNA
<213>  artificial sequence

<220>
<223>  chemically synthesized

<400>  1
gagtctagag ccaccatgga gacagacaca ctcctgctat gggtactgct gctctgggtt      60

ccaggttcca ctggtgacta tgaggcccag gcggccggta ccgctagcgg ccaggccggc     120

cgcaatgctg tgggccagga cacgcaggag gtcatcgtgg tgccacactc cttgcccttt     180

aaggtggtgg tgatctcagc catcctggcc ctggtggtgc tcaccatcat ctcccttatc     240

atcctcatca tgctttggca gaagaagcca cgttaggggc ccgag                     285


<210>  2
<211>  339
<212>  PRT
<213>  artificial sequence

<220>
<223>  chemically synthesized

<400>  2

<210>  3
<211>  335
<212>  PRT
<213>  artificial sequence

<220>
<223>  chemically synthesized

<400>  3

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Leu Thr
            20                  25                  30

Gln Pro Pro Ser Val Ser Gly Ala Pro Gly Gln Arg Val Thr Ile Ser
        35                  40                  45

Cys Thr Gly Ser Ser Ser Asn Ile Gly Ala Gly Tyr Asp Val His Trp
    50                  55                  60

Tyr Gln Gln Leu Pro Gly Thr Ala Pro Gln Leu Leu Ile Tyr Gly Asn
65                  70                  75                  80

Ile Asn Arg Pro Ser Gly Val Pro Asp Arg Ser Ser Gly Ser Lys Ser
                85                  90                  95

Gly Thr Ser Ala Ser Leu Ala Ile Thr Gly Leu Arg Ala Glu Asp Glu
            100                 105                 110

Val Asp Tyr Tyr Cys Gln Ser Tyr Asp Arg Thr Leu Ser Gly Val Ile
        115                 120                 125

Phe Gly Gly Gly Thr Gln Leu Thr Val Leu Gly Gly Gly Ser Ser Arg
    130                 135                 140

Ser Ser Ser Ser Gly Gly Gly Gly Ser Gly Gly Gly Gly Gln Ile Thr
145                 150                 155                 160

Leu Lys Glu Ser Gly Gly Gly Val Val Gln Pro Gly Ser Ser Arg Thr
                165                 170                 175

Leu Ser Cys Glu Ala Ser Gly Phe Ser Phe Ser Thr Tyr Trp Met Thr
            180                 185                 190

Trp Val Arg Gln Ala Pro Gly Lys Gly Leu Glu Trp Val Ala Asn Ile
        195                 200                 205

Lys Gln Asp Gly Ser Glu Lys Tyr Tyr Val Asp Ser Val Lys Gly Arg
    210                 215                 220

Phe Thr Ile Ser Arg Asp Asn Ala Lys Asn Ser Leu Tyr Leu Gln Met
225                 230                 235                 240

Asn Ser Leu Arg Ala Glu Asp Thr Ala Val Tyr Tyr Cys Ser Arg Gly
                245                 250                 255

Phe Phe Tyr Trp Gly Gln Gly Ala Leu Val Thr Val Ser Ser Ala Ser
            260                 265                 270

Thr Lys Gly Pro Ser Val Thr Ser Gly Gln Ala Gly Arg Asn Ala Val
        275                 280                 285

Gly Gln Asp Thr Gln Glu Val Ile Val Val Pro His Ser Leu Pro Phe
    290                 295                 300

Lys Val Val Val Ile Ser Ala Ile Leu Ala Leu Val Val Leu Thr Ile
305                 310                 315                 320

Ile Ser Leu Ile Ile Leu Ile Met Leu Trp Gln Lys Lys Pro Arg
                325                 330                 335

<210>  4
<211>  349
<212>  PRT
<213>  artificial sequence

<220>
<223>  chemically synthesized

<400>  4

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Pro Ser Gln
            20                  25                  30

Ser Pro Ser Val Ser Gly Ser Pro Gly Gln Ser Ile Thr Ile Ser Cys
        35                  40                  45

Thr Gly Thr Ser Ser Asp Phe Gly Gly Tyr Lys Phe Val Ser Trp Tyr
    50                  55                  60

Gln Gln His Pro Gly Lys Ala Pro Lys Leu Ile Ile Phe Asp Val Ser
65                  70                  75                  80

Arg Arg Pro Ala Gly Val Ser Asn Arg Phe Ser Gly Ser Lys Ser Gly
                85                  90                  95

Asn Thr Ala Ser Leu Thr Ile Ser Gly Leu Gln Ala Asp Asp Glu Ala
            100                 105                 110

Glu Tyr Tyr Cys Ser Ser Tyr Lys Ser Gly Thr Thr Leu Tyr Val Phe
        115                 120                 125

Gly Thr Gly Thr Glu Leu Thr Val Leu Gly Gly Gly Ser Ser Arg Ser
    130                 135                 140

Ser Ser Ser Gly Gly Gly Gly Ser Gly Gly Gly Gly Glu Val Gln Leu
145                 150                 155                 160

Val Gln Ser Gly Pro Gly Leu Leu Lys Pro Ser Glu Thr Leu Ser Leu
                165                 170                 175

Thr Cys Ser Val Ser Gly Gly Ser Val Ala Ser Ser Ser Tyr Tyr Trp
            180                 185                 190

Ser Trp Ile Arg Gln Ser Pro Arg Lys Gly Leu Glu Trp Ile Gly His
        195                 200                 205

Ile Phe Tyr Ser Gly Ala Ala Lys Tyr Ser Pro Ser Leu Arg Ser Arg
    210                 215                 220

Ala Thr Ile Ser Val Asp Thr Ser Arg Asn Gln Phe Asn Leu Lys Leu
225                 230                 235                 240

Ser Ser Val Thr Ala Ala Asp Thr Ala Thr Tyr Tyr Cys Ala Arg Asp
                245                 250                 255

Ala His Leu Ile Val Val Pro Ile Ala Gly Ala Leu Gly Ala Phe Asp
            260                 265                 270

Val Trp Gly Gln Gly Thr Val Val Ala Val Ser Ser Ala Ser Thr Lys
        275                 280                 285

Gly Pro Ser Val Thr Ser Gly Gln Ala Gly Arg Asn Ala Val Gly Gln
    290                 295                 300

Asp Thr Gln Glu Val Ile Val Val Pro His Ser Leu Pro Phe Lys Val
305                 310                 315                 320

Val Val Ile Ser Ala Ile Leu Ala Leu Val Val Leu Thr Ile Ile Ser
                325                 330                 335

Leu Ile Ile Leu Ile Met Leu Trp Gln Lys Lys Pro Arg
            340                 345

<210>  5
<211>  349
<212>  PRT
<213>  artificial sequence

<220>
<223>  chemically synthesized

<400>  5

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Leu Leu Thr
            20                  25                  30

Gln Pro Pro Ser Val Ser Gly Ala Pro Gly Gln Arg Ala Thr Ile Ser
        35                  40                  45

Cys Thr Gly Ser Ser Ser Asn Ile Gly Ala Gly Tyr Gly Val Gln Trp
    50                  55                  60

Tyr Gln Gln Leu Pro Gly Thr Ala Pro Lys Leu Leu Ile Phe Gly Asn
65                  70                  75                  80

Asn Asn Arg Pro Ser Gly Val Pro Ala Arg Phe Ser Ala Ser Lys Ser
                85                  90                  95

Gly Thr Ser Ala Ser Leu Thr Ile Thr Gly Leu Gln Ala Glu Asp Glu
            100                 105                 110

Ala Asp Tyr Tyr Cys Arg Ser Tyr Arg Ser Gly Val Ser Leu Ser Val
        115                 120                 125

Phe Gly Thr Gly Thr Lys Leu Thr Val Leu Gly Gly Gly Ser Ser Arg
    130                 135                 140

Ser Ser Ser Ser Gly Gly Gly Gly Ser Gly Gly Gly Gly Gln Val Gln
145                 150                 155                 160

Leu Val Gln Ser Gly Pro Gly Leu Val Lys Pro Ser Glu Thr Leu Ser
                165                 170                 175

Leu Thr Cys Ser Val Ser Gly Gly Ser Val Ser Asp Ala Ser Tyr Cys
            180                 185                 190

Trp Thr Trp Ile Arg Gln Pro Pro Gly Lys Gly Leu Glu Trp Ile Gly
        195                 200                 205

His Thr Ile Tyr Ser Gly Lys Thr Ser Tyr Asn Pro Ser Leu Lys Ser
    210                 215                 220

Arg Val Ala Ile Ser Leu Asp Thr Ser Gln Asn His Phe Ser Leu Arg
225                 230                 235                 240

Leu Thr Ser Val Thr Ala Ala Asp Thr Ala Val Tyr Tyr Cys Ala Arg
                245                 250                 255

Gly Ala Cys Tyr Arg Ser Asn Trp Tyr Pro Leu Lys His Phe Phe Asp
            260                 265                 270

Tyr Trp Gly Gln Gly Ala Leu Val Ala Val Ser Ser Ala Ser Thr Lys
        275                 280                 285

Gly Pro Ser Val Thr Ser Gly Gln Ala Gly Arg Asn Ala Val Gly Gln
    290                 295                 300

Asp Thr Gln Glu Val Ile Val Val Pro His Ser Leu Pro Phe Lys Val
305                 310                 315                 320

Val Val Ile Ser Ala Ile Leu Ala Leu Val Val Leu Thr Ile Ile Ser
                325                 330                 335

Leu Ile Ile Leu Ile Met Leu Trp Gln Lys Lys Pro Arg
            340                 345

<210>  6
<211>  199
<212>  PRT

<213>  artificial sequence

<220>
<223>  chemically synthesized

<400>  6

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Thr Leu Thr
            20                  25                  30

Gln Ser Pro Ala Thr Leu Ser Val Ser Pro Gly Glu Ser Ala Thr Leu
        35                  40                  45

Ser Cys Arg Ala Ser Gln Ser Val Arg Arg Asn Leu Ala Trp Tyr Gln
    50                  55                  60

Gln Arg Pro Gly Gln Ala Pro Arg Leu Leu Ile Tyr Gly Ala Thr Thr
65                  70                  75                  80

Arg Ala Thr Gly Val Pro Val Arg Ile Ser Gly Ser Gly Ser Gly Thr
                85                  90                  95

Glu Phe Thr Leu Thr Ile Ser Ser Leu Gln Ser Glu Asp Phe Val Val
            100                 105                 110

Tyr Tyr Cys Gln Gln Tyr Asn Asp Trp Pro Gly Thr Phe Gly Gln Gly
        115                 120                 125

Thr Lys Val Asp Ile Lys Gly Gly Ser Ser Arg Ser Ser Ser Ser Gly
    130                 135                 140

Gly Gly Gly Ser Gly Gly Gly Gly Glu Val Gln Leu Val Glu Ser Gly
145                 150                 155                 160

Pro Gly Leu Val Lys Pro Ser Gly Thr Leu Ser Leu Thr Cys Ala Val
                165                 170                 175

Ser Gly Val Ser Ile Thr Ser Ser Asn Trp Trp Ser Trp Val Arg Gln
            180                 185                 190

Pro Pro Gly Lys Gly Pro Glu
        195

<210>  7
<211>  347

<212>  PRT
<213>  artificial sequence

<220>
<223>  chemically synthesized

<400>  7

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Gln Met Thr
            20                  25                  30

Gln Ser Pro Ser Thr Leu Ser Ala Ser Val Gly Asp Arg Val Thr Ile
        35                  40                  45

Thr Cys Arg Ala Ser Gln Gly Ile Ser Ser Tyr Leu Val Trp Tyr Gln
    50                  55                  60

Gln Lys Pro Gly Lys Ala Pro Lys Leu Leu Ile Tyr Asp Ser Ser Thr
65                  70                  75                  80

Leu Gln Ser Gly Val Pro Ser Arg Phe Ser Gly Ser Gly Ser Gly Thr
                85                  90                  95

Asp Phe Thr Leu Thr Ile Ser Ser Leu Gln Pro Glu Asp Val Ala Ala
            100                 105                 110

Tyr Phe Cys Gln Gln Val Tyr Ser Tyr Pro Arg Thr Phe Gly Gln Gly
        115                 120                 125

Thr Lys Val Asp Ile Lys Gly Gly Ser Ser Arg Ser Ser Ser Ser Gly
    130                 135                 140

Gly Gly Gly Ser Gly Gly Gly Gly Gln Ile Thr Leu Lys Glu Ser Gly
145                 150                 155                 160

Gly Gly Leu Val Lys Pro Gly Gly Ser Leu Arg Leu Ser Cys Val Ala
                165                 170                 175

Ser Gly Leu Ser Phe Lys Asp Ala Trp Met Ser Trp Val Arg Gln Ala
            180                 185                 190

Pro Gly Lys Gly Leu Glu Trp Val Gly Arg Met Lys Ser Arg Ala Ser
        195                 200                 205

Gly Gly Thr Thr Glu Tyr Gly Gly Leu Ala Asn Gly Arg Phe Thr Ile

210                 215                 220

Ser Arg Asp Asp Ser Lys Asn Thr Leu Phe Leu Gln Ile Asn Arg Leu
225                 230                 235                 240

Glu Thr Glu Asp Thr Ala Val Tyr Tyr Cys Thr Phe Ala Phe Cys Ser
                245                 250                 255

Gly Thr Ser Cys Tyr Gly Gln Tyr Thr Tyr Tyr Gly Leu Asp Val Trp
            260                 265                 270

Gly Gln Gly Thr Thr Val Ile Val Ser Ser Ala Ser Thr Lys Gly Pro
        275                 280                 285

Ser Val Thr Ser Gly Gln Ala Gly Arg Asn Ala Val Gly Gln Asp Thr
    290                 295                 300

Gln Glu Val Ile Val Val Pro His Ser Leu Pro Phe Lys Val Val Val
305                 310                 315                 320

Ile Ser Ala Ile Leu Ala Leu Val Val Leu Thr Ile Ile Ser Leu Ile
                325                 330                 335

Ile Leu Ile Met Leu Trp Gln Lys Lys Pro Arg
            340                 345

<210>  8
<211>  348
<212>  PRT
<213>  artificial sequence

<220>
<223>  chemically synthesized

<400>  8

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Gly Leu Thr
            20                  25                  30

Gln Pro Pro Ser Val Ser Gly Ala Pro Gly Gln Arg Val Thr Ile Ser
        35                  40                  45

Cys Thr Gly Ser Ser Ser Asn Ile Gly Arg Phe Asp Val His Trp Tyr
    50                  55                  60

Gln Gln Leu Pro Gly Thr Ala Pro Lys Leu Leu Ile Tyr Gly Asn Thr
65                  70                  75                  80

Asn Arg Pro Ser Gly Val Pro Asp Arg Phe Ser Gly Ser Lys Ser Gly
                85                  90                  95

Ser Ser Ala Ser Leu Ala Ile Thr Gly Leu Gln Ala Glu Asp Glu Ala
            100                 105                 110

Asp Tyr Tyr Cys Gln Ser Tyr Asp Arg Ser Leu Ser Gly Val Val Phe
        115                 120                 125

Gly Gly Gly Thr Gln Leu Thr Val Leu Gly Gly Gly Ser Ser Arg Ser
    130                 135                 140

Ser Ser Ser Gly Gly Gly Gly Ser Gly Gly Gly Gly Glu Val Gln Leu
145                 150                 155                 160

Leu Glu Ser Gly Pro Gly Leu Val Lys Pro Ser Glu Thr Leu Ser Leu
                165                 170                 175

Thr Cys Thr Val Ser Gly Gly Ser Ile Ser Ser Gly Asn Tyr Tyr Trp
            180                 185                 190

Ser Trp Ile Arg Gln Thr Pro Glu Lys Gly Leu Glu Trp Leu Gly Tyr
        195                 200                 205

Val His Tyr Thr Gly Ser Ser Lys Leu Asn Pro Ser Leu Lys Ser Arg
    210                 215                 220

Val Thr Ile Ser Val Asp Thr Tyr Thr Asn Gln Phe Ser Leu Ser Leu
225                 230                 235                 240

Ser Ser Met Thr Ala Ala Asp Thr Ala Val Tyr Tyr Cys Ala Arg Gly
                245                 250                 255

Lys Asn Cys Ala Asn Asp Ile Cys Tyr Ile Gly Ser Trp Phe Asp Pro
            260                 265                 270

Trp Gly Gln Gly Thr Leu Val Thr Val Ser Ser Ala Ser Thr Lys Gly
        275                 280                 285

Pro Ser Val Thr Ser Gly Gln Ala Gly Arg Asn Ala Val Gly Gln Asp
    290                 295                 300

Thr Gln Glu Val Ile Val Val Pro His Ser Leu Pro Phe Lys Val Val

305                 310                 315                 320

Val Ile Ser Ala Ile Leu Ala Leu Val Val Leu Thr Ile Ile Ser Leu
                325                 330                 335

Ile Ile Leu Ile Met Leu Trp Gln Lys Lys Pro Arg
            340                 345

<210>  9
<211>  344
<212>  PRT
<213>  artificial sequence

<220>
<223>  chemically synthesized

<400>  9

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Met Thr
            20                  25                  30

Gln Ser Pro Ser Ser Leu Ser Ala Ser Val Gly Asp Arg Val Thr Ile
        35                  40                  45

Thr Cys Arg Ala Ser Gln Gly Val Ser Arg Ala Leu Ala Trp Tyr Gln
    50                  55                  60

Gln Lys Pro Gly Asn Pro Pro Lys Leu Leu Ile Tyr Asp Ala Ser Asn
65                  70                  75                  80

Leu Gln Ser Gly Val Pro Ser Arg Phe Ser Gly Gly Gly Ser Gly Thr
                85                  90                  95

Glu Phe Ile Leu Thr Ile Ser Ser Leu Gln Pro Glu Asp Phe Ala Thr
            100                 105                 110

Tyr Tyr Cys Gln Gln Tyr Asn Ala Tyr Pro Trp Thr Phe Gly Gln Gly
        115                 120                 125

Thr Lys Leu Glu Ile Lys Gly Gly Ser Ser Arg Ser Ser Ser Ser Gly
    130                 135                 140

Gly Gly Gly Ser Gly Gly Gly Gly Gln Val Gln Leu Gln Glu Ser Gly
145                 150                 155                 160

Pro Gly Leu Val Lys Pro Ser Glu Thr Leu Ser Leu Thr Cys Thr Val
                165                 170                 175

Ser Gly Gly Ser Ile Ser Ser Gly Asn Tyr Tyr Trp Ser Trp Ile Arg
            180                 185                 190

Gln Thr Pro Glu Lys Gly Leu Glu Trp Leu Gly Tyr Val His Tyr Thr
        195                 200                 205

Gly Ser Ser Lys Leu Asn Pro Ser Leu Lys Ser Arg Val Thr Ile Ser
    210                 215                 220

Val Asp Thr Tyr Thr Asn Gln Phe Ser Leu Ser Leu Ser Ser Met Thr
225                 230                 235                 240

Ala Ala Asp Thr Ala Val Tyr Tyr Cys Ala Arg Gly Lys Asn Cys Ala
                245                 250                 255

Asn Asp Ile Cys Tyr Ile Gly Ser Trp Phe Asp Pro Trp Gly Gln Gly
            260                 265                 270

Thr Leu Val Thr Val Ser Ser Ala Ser Thr Lys Gly Pro Ser Val Thr
        275                 280                 285

Ser Gly Gln Ala Gly Arg Asn Ala Val Gly Gln Asp Thr Gln Glu Val
    290                 295                 300

Ile Val Val Pro His Ser Leu Pro Phe Lys Val Val Val Ile Ser Ala
305                 310                 315                 320

Ile Leu Ala Leu Val Val Leu Thr Ile Ile Ser Leu Ile Ile Leu Ile
                325                 330                 335

Met Leu Trp Gln Lys Lys Pro Arg
            340

<210>  10
<211>  349
<212>  PRT
<213>  artificial sequence

<220>
<223>  chemically synthesized

<400>  10

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Leu Thr
            20                  25                  30

Gln Pro Pro Ser Val Ser Gly Ala Pro Gly Gln Arg Val Thr Ile Ser
        35                  40                  45

Cys Thr Gly Thr Ser Ser Asn Ile Gly Ala Gly Tyr Ala Val His Trp
    50                  55                  60

Tyr Gln Gln Val Pro Gly Thr Ala Pro Lys Leu Leu Ile Phe Gly Lys
65                  70                  75                  80

Thr Asn Arg Pro Ser Gly Val Pro Gly Arg Phe Ser Gly Ser Lys Ala
                85                  90                  95

Gly Thr Ser Ala Ser Leu Ala Ile Thr Gly Leu Gln Pro Glu Asp Glu
            100                 105                 110

Ala His Tyr Tyr Cys Gln Ser Tyr Asp Ser Asn Leu Ser Glu Val Val
        115                 120                 125

Phe Gly Gly Gly Thr Gln Leu Thr Val Leu Gly Gly Gly Ser Ser Arg
    130                 135                 140

Ser Ser Ser Ser Gly Gly Gly Gly Ser Gly Gly Gly Gly Glu Val Gln
145                 150                 155                 160

Leu Val Gln Ser Gly Pro Gly Leu Val Lys Pro Ser Glu Thr Leu Ser
                165                 170                 175

Leu Thr Cys Thr Val Ser Gly Gly Ser Ile Ser Ser Gly Asn Tyr Tyr
            180                 185                 190

Trp Ser Trp Ile Arg Gln Thr Pro Glu Lys Gly Leu Glu Trp Leu Gly
        195                 200                 205

Tyr Val His Tyr Thr Gly Ser Ser Lys Leu Asn Pro Ser Leu Lys Ser
    210                 215                 220

Arg Val Thr Ile Ser Val Asp Thr Tyr Thr Asn Gln Phe Ser Leu Ser
225                 230                 235                 240

Leu Ser Ser Met Thr Ala Ala Asp Thr Ala Val Tyr Tyr Cys Ala Arg
                245                 250                 255

Gly Lys Asn Cys Ala Asn Asp Ile Cys Tyr Ile Gly Ser Trp Phe Asp
            260                 265                 270

Pro Trp Gly Gln Gly Thr Leu Val Thr Val Ser Ser Ala Ser Thr Lys
        275                 280                 285

Gly Pro Ser Val Thr Ser Gly Gln Ala Gly Arg Asn Ala Val Gly Gln
    290                 295                 300

Asp Thr Gln Glu Val Ile Val Val Pro His Ser Leu Pro Phe Lys Val
305                 310                 315                 320

Val Val Ile Ser Ala Ile Leu Ala Leu Val Val Leu Thr Ile Ile Ser
                325                 330                 335

Leu Ile Ile Leu Ile Met Leu Trp Gln Lys Lys Pro Arg
            340                 345

<210>  11
<211>  347
<212>  PRT
<213>  artificial sequence

<220>
<223>  chemically synthesized

<400>  11

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Leu Thr
            20                  25                  30

Gln Pro Pro Ser Val Ser Gly Ala Pro Gly Gln Arg Val Ser Ile Ser
        35                  40                  45

Cys Thr Gly Ser Ser Ser Asn Ile Gly Ala Arg Tyr Asp Val His Trp
    50                  55                  60

Tyr Gln Gln Leu Pro Gly Thr Ala Pro Lys Leu Leu Ile Tyr Gly Asn
65                  70                  75                  80

Thr Asn Arg Pro Ser Gly Val Pro Asp Arg Phe Ser Gly Ser Lys Ser
                85                  90                  95

Gly Ser Ser Ala Ser Leu Ala Ile Thr Gly Leu Gln Ala Glu Asp Glu
            100                 105                 110

Ala Asp Tyr Tyr Cys Gln Ser Tyr Asp Arg Ser Leu Ser Gly Val Val
        115                 120                 125

Phe Gly Gly Gly Thr Lys Leu Thr Val Leu Gly Gly Gly Ser Ser Arg
    130                 135                 140

Ser Ser Ser Ser Gly Gly Gly Gly Ser Gly Gly Gly Gly Glu Val Gln
145                 150                 155                 160

Leu Val Gln Ser Gly Pro Gly Leu Val Lys Pro Ser Glu Thr Leu Ser
                165                 170                 175

Leu Thr Cys Thr Val Ser Gly Gly Ser Ile Ser Ser Thr Ser Tyr Ser
            180                 185                 190

Trp Gly Trp Ile Arg Gln Pro Pro Gly Lys Gly Leu Glu Trp Ile Ala
        195                 200                 205

Thr Val Ser Tyr Ser Gly Arg Ser Tyr Ser Asn Pro Ser Leu Lys Ser
    210                 215                 220

Arg Val Thr Thr Ser Val Asp Thr Ser Lys Asn Gln Phe Ser Leu Arg
225                 230                 235                 240

Leu Gly Ser Val Thr Ala Ala Asp Thr Ala Val Tyr Tyr Cys Ala Arg
                245                 250                 255

Leu Tyr Tyr Ile Trp Arg Ser Tyr His Ser Gly Arg Phe Asp Tyr Trp
            260                 265                 270

Gly Gln Gly Thr Leu Val Pro Val Ser Ser Ala Ser Thr Lys Gly Pro
        275                 280                 285

Ser Val Thr Ser Gly Gln Ala Gly Arg Asn Ala Val Gly Gln Asp Thr
    290                 295                 300

Gln Glu Val Ile Val Val Pro His Ser Leu Pro Phe Lys Val Val Val
305                 310                 315                 320

Ile Ser Ala Ile Leu Ala Leu Val Val Leu Thr Ile Ile Ser Leu Ile
                325                 330                 335

Ile Leu Ile Met Leu Trp Gln Lys Lys Pro Arg
            340                 345

<210>  12
<211>  348
<212>  PRT
<213>  artificial sequence

<220>
<223>  chemically synthesized

<400>  12

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Leu Thr
            20                  25                  30

Gln Pro Pro Ser Val Ser Gly Leu Pro Gly Gln Ser Val Thr Val Ser
        35                  40                  45

Cys Thr Gly Thr Ser Ser Asp Val Ser His Ser Asn Tyr Val Ser Trp
    50                  55                  60

Tyr Gln Gln Leu Pro Gly Lys Ala Pro Lys Leu Ile Ile Tyr Asp Val
65                  70                  75                  80

Thr Lys Arg Pro Ser Gly Val Pro Asn Arg Phe Ser Gly Ser Lys Ser
                85                  90                  95

Gly Asn Thr Ala Ser Leu Thr Ile Ser Gly Leu Gln Thr Glu Asp Glu
            100                 105                 110

Ala Asp Tyr His Cys Cys Ser Tyr Ala Gly Gly Tyr Thr Trp Val Phe
        115                 120                 125

Gly Gly Gly Thr Gln Leu Thr Val Leu Gly Gly Gly Ser Ser Arg Ser
    130                 135                 140

Ser Ser Ser Gly Gly Gly Gly Ser Gly Gly Gly Gly Glu Val Gln Leu
145                 150                 155                 160

Val Glu Ser Gly Pro Gly Leu Val Lys Pro Ser Glu Thr Leu Ser Leu
                165                 170                 175

Thr Cys Thr Val Ser Gly Gly Ser Ile Ser Ser Gly Asn Tyr Tyr Trp
            180                 185                 190

Ser Trp Ile Arg Gln Thr Pro Glu Lys Gly Leu Glu Trp Leu Gly Tyr
        195                 200                 205

Val His Tyr Thr Gly Ser Ser Lys Leu Asn Pro Ser Leu Lys Ser Arg
    210                 215                 220

Val Thr Ile Ser Val Asp Thr Tyr Thr Asn Gln Phe Ser Leu Ser Leu
225                 230                 235                 240

Ser Ser Met Thr Ala Ala Asp Thr Ala Val Tyr Tyr Cys Ala Arg Gly
                245                 250                 255

Lys Asn Cys Ala Asn Asp Ile Cys Tyr Ile Gly Ser Trp Phe Asp Pro
            260                 265                 270

Trp Gly Gln Gly Thr Leu Val Thr Val Ser Ser Ala Ser Thr Lys Gly
        275                 280                 285

Pro Ser Val Thr Ser Gly Gln Ala Gly Arg Asn Ala Val Gly Gln Asp
    290                 295                 300

Thr Gln Glu Val Ile Val Val Pro His Ser Leu Pro Phe Lys Val Val
305                 310                 315                 320

Val Ile Ser Ala Ile Leu Ala Leu Val Val Leu Thr Ile Ile Ser Leu
                325                 330                 335

Ile Ile Leu Ile Met Leu Trp Gln Lys Lys Pro Arg
            340                 345

<210>  13
<211>  349
<212>  PRT
<213>  artificial sequence

<220>
<223>  chemically synthesized

<400>  13

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Leu Thr
            20                  25                  30

Gln Pro Pro Ser Met Ser Gly Ala Pro Gly Gln Arg Val Ser Ile Ser
        35                  40                  45

Cys Thr Gly Ser Ser Ser Asn Ile Gly Ala Arg Tyr Asp Val His Trp
    50                  55                  60

Tyr Gln Gln Leu Pro Gly Thr Ala Pro Lys Leu Leu Ile Tyr Gly Asn
65                  70                  75                  80

Thr Asn Arg Pro Ser Gly Val Pro Asp Arg Phe Ser Gly Ser Lys Ser
                85                  90                  95

Gly Ser Ser Ala Ser Leu Ala Ile Thr Gly Leu Gln Ala Glu Asp Glu
            100                 105                 110

Ala Asp Tyr Tyr Cys Gln Ser Tyr Asp Arg Ser Leu Ser Gly Val Val
        115                 120                 125

Phe Gly Gly Gly Thr Lys Leu Thr Val Leu Gly Gly Gly Ser Ser Arg
    130                 135                 140

Ser Ser Ser Ser Gly Gly Gly Gly Ser Gly Gly Gly Gly Gln Ile Thr
145                 150                 155                 160

Leu Lys Glu Ser Gly Pro Gly Leu Val Lys Pro Ser Glu Thr Leu Ser
                165                 170                 175

Leu Thr Cys Thr Val Ser Gly Gly Phe Ile Ser Ser Ser Ser Tyr Tyr
            180                 185                 190

Trp Gly Trp Ile Arg Gln Pro Pro Gly Lys Gly Leu Glu Trp Ile Gly
        195                 200                 205

Ser Ser Tyr Tyr Gly Gly Ser Thr Asn Tyr Asn Pro Ser Leu Lys Ser
    210                 215                 220

Arg Val Thr Ile Leu Val Asp Arg Ser Lys Asn Gln Phe Ser Leu Lys
225                 230                 235                 240

Leu Ser Ser Val Thr Ala Ala Asp Thr Ala Val Tyr Tyr Cys Ala Arg
                245                 250                 255

Ser Thr Val Ala Val Val Ser Met Ala Gly Pro Ser Gly Trp Phe Asp
            260                 265                 270

Pro Trp Gly Gln Gly Ile Met Val Thr Val Ser Ser Ala Ser Thr Lys
        275                 280                 285

Gly Pro Ser Val Thr Ser Gly Gln Ala Gly Arg Asn Ala Val Gly Gln
    290                 295                 300

Asp Thr Gln Glu Val Ile Val Val Pro His Ser Leu Pro Phe Lys Val
305                 310                 315                 320

Val Val Ile Ser Ala Ile Leu Ala Leu Val Val Leu Thr Ile Ile Ser
                325                 330                 335

Leu Ile Ile Leu Ile Met Leu Trp Gln Lys Lys Pro Arg
            340                 345

<210>  14
<211>  346
<212>  PRT
<213>  artificial sequence

<220>
<223>  chemically synthesized

<400>  14

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Val Thr
            20                  25                  30

Gln Glu Pro Ser Val Ser Gly Ala Pro Gly Gln Ser Val Thr Ile Ser
        35                  40                  45

Cys Thr Gly Gly Ser Ser Asn Ile Gly Ala Ser Tyr Asp Val His Trp
    50                  55                  60

Tyr Lys Gln Leu Pro Gly Ala Ala Pro Ile Leu Leu Ile Tyr Ala Asn
65                  70                  75                  80

Tyr Ile Arg Pro Ser Gly Val Pro Asp Arg Phe Ser Ala Ser Lys Ser
                85                  90                  95

Gly Thr Ser Ala Ser Leu Ala Ile Thr Gly Leu Gln Ala Glu Asp Glu
            100                 105                 110

Ala Asp Tyr Tyr Cys Gln Ser Tyr Asp Ser Ser Leu Ser Gly Val Val
        115                 120                 125

Phe Gly Gly Gly Thr Lys Leu Thr Val Leu Gly Gly Gly Ser Ser Arg
    130                 135                 140

Ser Ser Ser Ser Gly Gly Gly Gly Ser Gly Gly Gly Gly Gln Val Gln
145                 150                 155                 160

Leu Gln Glu Ser Gly Gly Gly Trp Val Gln Ser Gly Gly Ser Leu Arg
                165                 170                 175

Leu Ser Cys Ala Ala Ser Gly Phe Ser Phe Ser Ser Tyr Ala Met Ser
            180                 185                 190

Trp Val Arg Gln Ala Pro Gly Lys Gly Leu Glu Trp Val Ser Ala Met
        195                 200                 205

Ser Pro Ile Gly Gly Ser Thr Phe Tyr Ala Asp Ser Val Lys Gly Arg
    210                 215                 220

Phe Thr Ile Ser Arg Asp Asn Ser Lys Asn Thr Leu Phe Leu Gln Met
225                 230                 235                 240

Asn Ser Leu Arg Ala Glu Asp Thr Ala Val Tyr Tyr Cys Ala Lys Asp
                245                 250                 255

Ala Val Val Thr Ala Val Gly Leu Gly Arg Tyr Phe Asp Leu Trp Gly
            260                 265                 270

Arg Gly Thr Leu Val Ser Val Ser Ser Ala Ser Thr Lys Gly Pro Ser
        275                 280                 285

Val Thr Ser Gly Gln Ala Gly Arg Asn Ala Val Gly Gln Asp Thr Gln
    290                 295                 300

Glu Val Ile Val Val Pro His Ser Leu Pro Phe Lys Val Val Val Ile
305                 310                 315                 320

Ser Ala Ile Leu Ala Leu Val Val Leu Thr Ile Ile Ser Leu Ile Ile
                325                 330                 335

Leu Ile Met Leu Trp Gln Lys Lys Pro Arg
            340                 345

<210>  15
<211>  342
<212>  PRT
<213>  artificial sequence

<220>
<223>  chemically synthesized

<400>  15

Met Glu Thr Asp Thr Leu Leu Leu Trp Val Leu Leu Leu Trp Val Pro

1               5                   10                  15

Gly Ser Thr Gly Asp Tyr Glu Ala Gln Ala Ala Glu Leu Thr Leu Thr
            20                  25                  30

Gln Ser Pro Ser Ser Leu Ser Ala Ser Val Gly Asp Arg Val Ile Leu
        35                  40                  45

Thr Cys Arg Ala Gly Gln Ser Ile Ser Asn Tyr Val Asn Trp Tyr Gln
    50                  55                  60

Gln Arg Pro Gly Lys Ala Pro Asn Leu Leu Ile Tyr Gly Ala Ser Ser
65                  70                  75                  80

Leu Gln Pro Gly Val Pro Ser Arg Phe Ser Gly Ser Gly Ser Gly Thr
                85                  90                  95

Asp Phe Thr Leu Thr Ile Ser Gly Leu Gln Pro Glu Asp Phe Ala Val
            100                 105                 110

Tyr Tyr Cys Gln Gln Thr Tyr Ser Thr Pro Arg Thr Phe Gly Gln Gly
        115                 120                 125

Thr Arg Leu Glu Ile Lys Gly Gly Ser Ser Arg Ser Ser Ser Ser Gly
    130                 135                 140

Gly Gly Gly Ser Gly Gly Gly Gly Glu Val Gln Leu Val Gln Ser Gly
145                 150                 155                 160

Pro Gly Leu Val Lys Pro Ser Gly Thr Leu Ser Leu Thr Cys Ala Val
                165                 170                 175

Ser Gly Val Ser Ile Thr Ser Ser Asn Trp Trp Ser Trp Val Arg Gln
            180                 185                 190

Pro Pro Gly Lys Gly Pro Glu Trp Ile Gly Glu Val Phe His Ser Gly
        195                 200                 205

Ser Ile Asn Tyr Asn Pro Ser Leu Lys Ser Arg Val Thr Ile Ser Val
    210                 215                 220

Asp Lys Ser Lys Asn Gln Phe Ser Leu Arg Leu Asn Ser Val Thr Ala
225                 230                 235                 240

Ala Asp Thr Ala Val Tyr Tyr Cys Ala Arg Glu Phe Ala Gly Leu Ile
                245                 250                 255

Pro His Tyr Tyr Ser Tyr Gly Met Asp Val Trp Gly Gln Gly Thr Thr
            260                 265                 270

Val Thr Val Ser Ser Ala Ser Thr Lys Gly Pro Ser Val Thr Ser Gly
        275                 280                 285

Gln Ala Gly Arg Asn Ala Val Gly Gln Asp Thr Gln Glu Val Ile Val
    290                 295                 300

Val Pro His Ser Leu Pro Phe Lys Val Val Val Ile Ser Ala Ile Leu
305                 310                 315                 320

Ala Leu Val Val Leu Thr Ile Ile Ser Leu Ile Ile Leu Ile Met Leu
                325                 330                 335

Trp Gln Lys Lys Pro Arg
            340

<210>  16
<211>  347
<212>  PRT
<213>  artificial sequence

<220>
<223>  chemically synthesized

<400>  16

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Glu Leu Thr
            20                  25                  30

Gln Pro Pro Ser Val Ser Gly Ala Pro Gly Gln Arg Val Ser Ile Ser
        35                  40                  45

Cys Thr Gly Ser Ser Ser Asn Ile Gly Ala Arg Tyr Asp Val His Trp
    50                  55                  60

Tyr Gln Gln Leu Pro Gly Thr Ala Pro Lys Leu Leu Ile Tyr Gly Asn
65                  70                  75                  80

Thr Asn Arg Pro Ser Gly Val Pro Asp Arg Phe Ser Gly Ser Lys Ser
                85                  90                  95

Gly Ser Ser Ala Ser Leu Ala Ile Thr Gly Leu Gln Ala Glu Asp Glu

            100                 105                 110

Ala Asp Tyr Tyr Cys Gln Ser Tyr Asp Arg Ser Leu Ser Gly Val Val
        115                 120                 125

Phe Gly Gly Gly Thr Lys Val Thr Val Leu Gly Gly Gly Ser Ser Arg
    130                 135                 140

Ser Ser Ser Ser Gly Gly Gly Gly Ser Gly Gly Gly Gly Gln Ile Thr
145                 150                 155                 160

Leu Lys Glu Ser Gly Pro Gly Leu Val Arg Pro Ser Glu Thr Leu Ser
                165                 170                 175

Leu Thr Cys Ser Val Ser Gly Gly Ser Ile Asp Ser Thr Ser Tyr Ser
            180                 185                 190

Trp Gly Trp Ile Arg Gln Pro Pro Gly Lys Gly Leu Glu Trp Ile Ala
        195                 200                 205

Ser Ile His Tyr Lys Gly Arg Thr Gln Tyr Asn Pro Ser Leu Lys Ser
    210                 215                 220

Arg Leu Thr Ile Ser Val Asp Pro Ser Arg Ser Gln Phe Ser Leu Arg
225                 230                 235                 240

Leu Ser Ser Val Thr Ala Ala Asp Thr Ala Val Tyr Tyr Cys Ala Arg
                245                 250                 255

Leu Tyr Tyr Ile Trp Gly Ser Tyr Gln Ser Gly Arg Phe Asp Tyr Trp
            260                 265                 270

Gly Gln Gly Ser Leu Val Thr Val Ser Ser Ala Ser Thr Lys Gly Pro
        275                 280                 285

Ser Val Thr Ser Gly Gln Ala Gly Arg Asn Ala Val Gly Gln Asp Thr
    290                 295                 300

Gln Glu Val Ile Val Val Pro His Ser Leu Pro Phe Lys Val Val Val
305                 310                 315                 320

Ile Ser Ala Ile Leu Ala Leu Val Val Leu Thr Ile Ile Ser Leu Ile
                325                 330                 335

Ile Leu Ile Met Leu Trp Gln Lys Lys Pro Arg
            340                 345

<210>  17
<211>  350
<212>  PRT
<213>  artificial sequence

<220>
<223>  chemically synthesized

<400>  17

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Glu Leu Thr
            20                  25                  30

Gln Pro Pro Ser Val Ser Gly Ala Pro Gly Gln Arg Val Thr Ile Ser
        35                  40                  45

Cys Thr Gly Ser Asn Ser Asn Ile Gly Ala Gly Tyr Asp Val His Trp
    50                  55                  60

Tyr Gln Gln Leu Pro Gly Thr Ala Pro Lys Leu Leu Ile Tyr Asn Asn
65                  70                  75                  80

Asn Asn Arg Pro Ser Gly Val Pro Asp Arg Phe Ser Gly Ser Gln Ser
                85                  90                  95

Gly Thr Ser Ala Ser Leu Ala Ile Thr Gly Val Gln Ala Glu Asp Glu
            100                 105                 110

Ala Asp Tyr Tyr Cys Gln Ser Tyr Asp Ser Ser Leu Ser Gly Val Val
        115                 120                 125

Phe Gly Gly Gly Thr Gln Leu Thr Val Leu Gly Gly Gly Ser Ser Arg
    130                 135                 140

Ser Ser Ser Ser Gly Gly Gly Gly Ser Gly Gly Gly Gly Gly Val Gln
145                 150                 155                 160

Leu Val Glu Ser Gly Pro Arg Leu Val Lys Pro Ser Glu Thr Leu Ser
                165                 170                 175

Leu Thr Cys Phe Val Ser Gly Gly Ser Ile Ser Ser Ala Ser Tyr Gln
            180                 185                 190

Trp Ser Trp Leu Arg Gln Arg Pro Gly Gln Gly Leu Glu Trp Ile Gly

195                 200                 205

Tyr Ile Tyr Tyr Ser Gly Ser Ser Asn Tyr Asn Pro Ser Leu Lys Arg
    210                 215                 220

Arg Val Ser Phe Ser Ala Asp Ala Ser Lys Asn Gln Phe Ser Met Arg
225                 230                 235                 240

Leu Val Ser Leu Thr Ala Ala Asp Thr Ala Val Tyr Tyr Cys Ala Arg
                245                 250                 255

Gln Ser His Ile Ile Val Val Pro Thr Ala Gly Ala Leu Gly Thr Phe
            260                 265                 270

Asp Ile Trp Gly His Gly Thr Met Val Thr Val Ser Ser Ala Ser Thr
        275                 280                 285

Lys Gly Pro Ser Val Thr Ser Gly Gln Ala Gly Arg Asn Ala Val Gly
    290                 295                 300

Gln Asp Thr Gln Glu Val Ile Val Val Pro His Ser Leu Pro Phe Lys
305                 310                 315                 320

Val Val Val Ile Ser Ala Ile Leu Ala Leu Val Val Leu Thr Ile Ile
                325                 330                 335

Ser Leu Ile Ile Leu Ile Met Leu Trp Gln Lys Lys Pro Arg
            340                 345                 350

<210>  18
<211>  169
<212>  PRT
<213>  artificial sequence

<220>
<223>  chemically synthesized

<400>  18

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Met Thr
            20                  25                  30

Gln Ser Pro Ala Thr Leu Ser Val Ser Pro Gly Glu Thr Ala Thr Leu
        35                  40                  45

Ser Cys Arg Ala Ser Gln Ser Val Gly Ser Asn Leu Ala Trp Phe Gln
    50                  55                  60

Gln Lys Pro Gly Gln Ala Pro Arg Leu Leu Ile Tyr Gly Ala Ser Thr
65                  70                  75                  80

Arg Ala Thr Gly Ile Pro Ala Arg Phe Ser Gly Gly Gly Ser Gly Thr
                85                  90                  95

Glu Phe Thr Leu Thr Ile Ser Ser Leu Gln Ser Glu Asp Phe Val Val
            100                 105                 110

Tyr Tyr Cys His Gln Tyr Ala Asp Trp Pro Arg Thr Phe Gly Gln Gly
        115                 120                 125

Thr Lys Val Glu Ile Lys Gly Gly Ser Ser Arg Ser Ser Ser Ser Gly
    130                 135                 140

Gly Gly Gly Ser Gly Gly Gly Gly Gln Val Gln Leu Gln Gln Trp Gly
145                 150                 155                 160

Glu Ala Trp Ser Ser Arg Gly Gly Pro
                165

<210>  19
<211>  346
<212>  PRT
<213>  artificial sequence

<220>
<223>  chemically synthesized

<400>  19

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Val Leu Thr
            20                  25                  30

Gln Pro Pro Ser Val Ser Gly Ala Pro Gly Gln Arg Val Thr Ile Ser
        35                  40                  45

Cys Ser Gly Asn Ser Ser Asn Ile Gly Thr Arg Tyr Asp Val His Trp
    50                  55                  60

Tyr Gln Gln Phe Pro Gly Thr Ala Pro Lys Leu Leu Ile Tyr Gly Asn
65                  70                  75                  80

Thr Asn Arg Pro Ser Gly Val Pro Asp Arg Phe Ser Gly Ser Thr Ser
                85                  90                  95

Gly Ala Ser Ala Ser Leu Ala Ile Thr Gly Leu Gln Ala Asp Asp Glu
            100                 105                 110

Ala Asp Tyr Tyr Cys Gln Ser Tyr Asp Ser Ser Leu Arg Ala Thr Val
        115                 120                 125

Phe Gly Gly Gly Thr Gln Leu Thr Val Leu Gly Gly Gly Ser Ser Arg
    130                 135                 140

Ser Ser Ser Ser Gly Gly Gly Gly Ser Gly Gly Gly Gly Gln Val Gln
145                 150                 155                 160

Leu Val Gln Ser Gly Gly Gly Trp Val Gln Ser Gly Gly Ser Leu Arg
                165                 170                 175

Leu Ser Cys Ala Ala Ser Gly Phe Ser Phe Ser Ser Tyr Ala Met Ser
            180                 185                 190

Trp Val Arg Gln Ala Pro Gly Lys Gly Leu Glu Trp Val Ser Ala Met
        195                 200                 205

Ser Pro Ile Gly Gly Ser Thr Phe Tyr Ala Asp Ser Val Lys Gly Arg
    210                 215                 220

Phe Thr Ile Ser Arg Asp Asn Ser Lys Asn Thr Leu Phe Leu Gln Met
225                 230                 235                 240

Asn Ser Leu Arg Ala Glu Asp Thr Ala Val Tyr Tyr Cys Ala Lys Asp
                245                 250                 255

Ala Val Val Thr Ala Val Gly Leu Gly Trp Tyr Phe Asp Leu Trp Gly
            260                 265                 270

Arg Gly Thr Leu Val Ser Val Ser Ser Ala Ser Thr Lys Gly Pro Ser
        275                 280                 285

Ala Thr Ser Gly Gln Ala Gly Arg Asn Ala Val Gly Gln Asp Thr Gln
    290                 295                 300

Glu Val Ile Val Val Pro His Ser Leu Pro Phe Lys Val Val Val Ile
305                 310                 315                 320

Ser Ala Ile Leu Ala Leu Val Val Leu Thr Ile Ile Ser Leu Ile Ile
                325                 330                 335

Leu Ile Met Leu Trp Gln Lys Lys Pro Arg
            340                 345

<210>  20
<211>  350
<212>  PRT
<213>  artificial sequence

<220>
<223>  chemically synthesized

<400>  20

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Gly Gln Thr
            20                  25                  30

Gln Gln Leu Ser Val Ser Gly Ala Pro Gly Gln Ser Val Thr Ile Ser
        35                  40                  45

Cys Thr Gly Gly Ser Ser Asn Ile Gly Ala Ser Tyr Asp Val His Trp
    50                  55                  60

Tyr Lys Gln Leu Pro Gly Ala Ala Pro Ile Leu Leu Ile Tyr Ala Asn
65                  70                  75                  80

Tyr Ile Arg Pro Ser Gly Val Pro Asp Arg Phe Ser Ala Ser Lys Ser
                85                  90                  95

Gly Thr Ser Ala Ser Leu Ala Ile Thr Gly Leu Gln Ala Glu Asp Glu
            100                 105                 110

Ala Asp Tyr Tyr Cys Gln Ser Tyr Asp Ser Ser Leu Ser Gly Val Val
        115                 120                 125

Phe Gly Gly Gly Thr Lys Leu Thr Val Leu Gly Gly Gly Ser Ser Arg
    130                 135                 140

Ser Ser Ser Ser Gly Gly Gly Gly Ser Gly Gly Gly Gly Glu Val Gln
145                 150                 155                 160

Leu Val Gln Ser Gly Pro Gly Leu Val Lys Pro Ser Gln Thr Leu Ser
                165                 170                 175

Ile Thr Cys Thr Val Ser Gly Gly Ser Val Ser Asp Thr Ser Tyr Tyr
            180                 185                 190

Trp Ala Trp Val Arg Gln Pro Pro Gly Lys Gly Leu Glu Trp Ile Ala
        195                 200                 205

His Ala Phe Tyr Ser Gly Ser Ala Asn Tyr Asn Pro Ser Leu Lys Ser
    210                 215                 220

Arg Ala Thr Ile Ser Val Asp Thr Ser Arg Asn Gln Phe Ser Leu Arg
225                 230                 235                 240

Leu Asp Ser Val Thr Ala Ala Asp Thr Ala Val Tyr Tyr Cys Ala Arg
                245                 250                 255

Glu Thr His Leu Val Val Val Pro Gly Ala Gly Ala Leu Gly Ala Phe
            260                 265                 270

Asp Ile Trp Gly Gln Gly Thr Met Val Thr Val Ser Pro Ala Ser Thr
        275                 280                 285

Lys Gly Pro Ser Val Thr Ser Gly Gln Ala Gly Arg Asn Ala Val Gly
    290                 295                 300

Gln Asp Thr Gln Glu Val Ile Val Val Pro His Ser Leu Pro Phe Lys
305                 310                 315                 320

Val Val Val Ile Ser Ala Ile Leu Ala Leu Val Val Leu Thr Ile Ile
                325                 330                 335

Ser Leu Ile Ile Leu Ile Met Leu Trp Gln Lys Lys Pro Arg
            340                 345                 350

<210>  21
<211>  343
<212>  PRT
<213>  artificial sequence

<220>
<223>  chemically synthesized

<400>  21

Met Glu Thr Asp Thr Leu Leu Leu Trp Val Leu Leu Leu Trp Val Pro
1               5                   10                  15

Gly Ser Thr Gly Asp Tyr Glu Ala Gln Ala Ala Glu Leu Glu Leu Thr
            20                  25                  30

Gln Pro Pro Ser Val Ser Gly Ser Pro Gly Gln Ser Val Thr Ile Ser
        35                  40                  45

Cys Thr Gly Thr Ser Ser Asn Val Gly Gly Tyr Asn Tyr Val Ser Trp
    50                  55                  60

Tyr Gln Gln Tyr Pro Gly Lys Ala Pro Lys Leu Met Ile Tyr Asp Val
65                  70                  75                  80

Thr Lys Arg Pro Ser Gly Val Pro Asp Arg Phe Ser Gly Ser Lys Ser
                85                  90                  95

Gly Ser Thr Ala Ser Leu Thr Ile Ser Gly Leu Gln Ser Asp Asp Asp
            100                 105                 110

Ala Asp Tyr Tyr Cys Cys Ser Tyr Ala Gly Ser Tyr Ile Trp Val Phe
        115                 120                 125

Gly Gly Gly Thr Lys Leu Thr Val Leu Gly Gly Gly Ser Ser Arg Ser
    130                 135                 140

Ser Ser Ser Gly Gly Gly Gly Ser Gly Gly Gly Gly Glu Val Gln Leu
145                 150                 155                 160

Val Gln Ser Gly Pro Gly Leu Val Lys Pro Ser Glu Thr Leu Ser Leu
                165                 170                 175

Thr Cys Thr Val Ser Gly Val Ser Val Ser Ser Gly Ser Tyr His Trp
            180                 185                 190

Ser Trp Ile Arg Gln Thr Pro Gly Lys Gly Leu Glu Trp Ile Gly Tyr
        195                 200                 205

Ile Tyr Tyr Ile Gly Ser Thr Lys Tyr Asn Pro Ser Leu Lys Ser Arg
    210                 215                 220

Ala Thr Ile Ser Ile Asn Thr Ser Thr Asn Gln Phe Ser Leu Lys Leu
225                 230                 235                 240

Ser Ser Val Thr Ala Ala Asp Thr Ala Val Tyr Tyr Cys Ala Arg Glu
                245                 250                 255

Ser Thr Ser Tyr Gly Glu Arg Arg Phe Asp Tyr Trp Gly Gln Gly Thr
            260                 265                 270

Arg Val Thr Val Ser Ser Ala Ser Thr Lys Gly Pro Ser Val Thr Ser
        275                 280                 285

Gly Gln Ala Gly Arg Asn Ala Val Gly Gln Asp Thr Gln Glu Val Ile
    290                 295                 300

Val Val Pro His Ser Leu Pro Phe Lys Val Val Val Ile Ser Ala Ile
305                 310                 315                 320

Leu Ala Leu Val Val Leu Thr Ile Ile Ser Leu Ile Ile Leu Ile Met
                325                 330                 335

Leu Trp Gln Lys Lys Pro Arg
            340

<210>  22
<211>  526
<212>  PRT
<213>  artificial sequence

<220>
<223>  chemically synthesized

<400>  22

<210>  23
<211>  522
<212>  PRT
<213>  artificial sequence

<220>
<223>  chemically synthesized

<400>  23

Met Glu Thr Asp Thr Leu Leu Leu Trp Val Leu Leu Leu Trp Val Pro
1               5                   10                  15

Gly Ser Thr Gly Asp Ala Asp Pro Ala Gln Ala Ala Glu Leu Val Met
            20                  25                  30

Thr Gln Ser Pro Ser Ser Leu Ser Ala Ser Val Gly Asp Arg Val Thr
        35                  40                  45

Ile Thr Cys Arg Ala Ser Gln Gly Val Ser Arg Ala Leu Ala Trp Tyr
    50                  55                  60

Gln Gln Lys Pro Gly Asn Pro Pro Lys Leu Leu Ile Tyr Asp Ala Ser
65                  70                  75                  80

Asn Leu Gln Ser Gly Val Pro Ser Arg Phe Ser Gly Gly Gly Ser Gly
                85                  90                  95

Thr Glu Phe Ile Leu Thr Ile Ser Ser Leu Gln Pro Glu Asp Phe Ala
            100                 105                 110

Thr Tyr Tyr Cys Gln Gln Tyr Asn Ala Tyr Pro Trp Thr Phe Gly Gln
        115                 120                 125

Gly Thr Lys Leu Glu Ile Lys Gly Gly Ser Ser Arg Ser Ser Ser Ser
    130                 135                 140

Gly Gly Gly Gly Ser Gly Gly Gly Gly Gln Val Gln Leu Gln Glu Ser
145                 150                 155                 160

Gly Pro Gly Leu Val Lys Pro Ser Glu Thr Leu Ser Leu Thr Cys Thr
                165                 170                 175

Val Ser Gly Gly Ser Ile Ser Ser Gly Asn Tyr Tyr Trp Ser Trp Ile
            180                 185                 190

Arg Gln Thr Pro Glu Lys Gly Leu Glu Trp Leu Gly Tyr Val His Tyr
        195                 200                 205

Thr Gly Ser Ser Lys Leu Asn Pro Ser Leu Lys Ser Arg Val Thr Ile
    210                 215                 220

Ser Val Asp Thr Tyr Thr Asn Gln Phe Ser Leu Ser Leu Ser Ser Met
225                 230                 235                 240

Thr Ala Ala Asp Thr Ala Val Tyr Tyr Cys Ala Arg Gly Lys Asn Cys
                245                 250                 255

Ala Asn Asp Ile Cys Tyr Ile Gly Ser Trp Phe Asp Pro Trp Gly Gln
            260                 265                 270

Gly Thr Leu Val Thr Val Ser Ser Ala Ser Thr Lys Gly Pro Ser Val
        275                 280                 285

Thr Ser Gly Gln Ala Gly Arg Lys Leu Thr His Thr Cys Pro Pro Cys
    290                 295                 300

Pro Ala Pro Glu Ala Glu Gly Ala Pro Ser Val Phe Leu Phe Pro Pro
305                 310                 315                 320

Lys Pro Lys Asp Thr Leu Met Ile Ser Arg Thr Pro Glu Val Thr Cys
                325                 330                 335

Val Val Val Asp Val Ser His Glu Asp Pro Glu Val Lys Phe Asn Trp
            340                 345                 350

Tyr Val Asp Gly Val Glu Val His Asn Ala Lys Thr Lys Pro Arg Glu
        355                 360                 365

Glu Gln Tyr Asn Ser Thr Tyr Arg Val Val Ser Val Leu Thr Val Leu
    370                 375                 380

His Gln Asp Trp Leu Asn Gly Lys Glu Tyr Lys Cys Lys Val Ser Asn
385                 390                 395                 400

Lys Ala Leu Pro Ala Ser Ile Glu Lys Thr Ile Ser Lys Ala Lys Gly
                405                 410                 415

Gln Pro Arg Glu Pro Gln Val Tyr Thr Leu Pro Pro Ser Arg Asp Glu
            420                 425                 430

Leu Thr Lys Asn Gln Val Ser Leu Thr Cys Leu Val Lys Gly Phe Tyr
        435                 440                 445

Pro Ser Asp Ile Ala Val Glu Trp Glu Ser Asn Gly Gln Pro Glu Asn
    450                 455                 460

Asn Tyr Lys Thr Thr Pro Pro Val Leu Asp Ser Asp Gly Ser Phe Phe
465                 470                 475                 480

Leu Tyr Ser Lys Leu Thr Val Asp Lys Ser Arg Trp Gln Gln Gly Asn
                485                 490                 495

Val Phe Ser Cys Ser Val Met His Glu Ala Leu His Asn His Tyr Thr
            500                 505                 510

Gln Lys Ser Leu Ser Leu Ser Pro Gly Lys
        515                 520

<210>  24
<211>  525
<212>  PRT
<213>  artificial sequence

<220>
<223>  chemically synthesized

<400>  24

<210>  25
<211>  524
<212>  PRT
<213>  artificial sequence

<220>
<223>  chemically synthesized

<400>  25

<210>  26
<211>  69
<212>  DNA
<213>  artificial sequence

<220>
<223>  chemically synthesized

<400>  26
cctgctatgg gtactgctgc tctgggttcc aggttccact ggtgactatg aggcccaggc      60

ggccggtac                                                              69

<210>  27
<211>  70
<212>  DNA
<213>  artificial sequence

<220>
<223>  chemically synthesized

<400>  27
cctcctgcgt gtcctggccc acagcattgc ggccggcctg gccgctagcg gtaccggccg      60

cctgggcctc                                                              70


<210>  28
<211>  69
<212>  DNA
<213>  artificial sequence

<220>
<223>  chemically synthesized

<400>  28
ggccaggaca cgcaggaggt catcgtggtg ccacactcct tgccctttaa ggtggtggtg      60

atctcagcc                                                               69


<210>  29
<211>  70
<212>  DNA
<213>  artificial sequence

<220>
<223>  chemically synthesized

<400>  29
catgatgagg atgataaggg agatgatggt gagcaccacc agggccagga tggctgagat      60

caccaccacc                                                              70


<210>  30
<211>  54
<212>  DNA
<213>  artificial sequence

<220>
<223>  chemically synthesized

<400>  30
gagtctagag ccaccatgga gacagacaca ctcctgctat gggtactgct gctc            54


<210>  31
<211>  55
<212>  DNA
<213>  artificial sequence

<220>
<223>  chemically synthesized

<400>  31
ctcgggcccc taacgtggct tcttctgcca aagcatgatg aggatgataa gggag           55


<210>  32
<211>  57

<212>  DNA
<213>  artificial sequence

<220>
<223>  chemically synthesized


<220>
<221>  misc_feature
<222>  (57)..(57)
<223>  n is a, c, g, or t

<400>  32
aagcagtggt aacaacgcag agtacttttt tttttttttt tttttttttt tttttvn        57


<210>  33
<211>  30
<212>  DNA
<213>  artificial sequence

<220>
<223>  chemically synthesized


<220>
<221>  misc_RNA
<222>  (28)..(30)
<223>  nucleotides 28 to 30 are ribonucleotides

<400>  33
aagcagtggt aacaacgcag agtacgcggg                                      30


<210>  34
<211>  23
<212>  DNA
<213>  artificial sequence

<220>
<223>  chemically synthesized

<400>  34
aagcagtggt atcaacgcag agt                                             23


<210>  35
<211>  22
<212>  DNA
<213>  artificial sequence

<220>
<223>  chemically synthesized

<400>  35
acaaattgga ctaatcgatg gc                                              22


<210>  36
<211>  20
<212>  DNA

<213>  artificial sequence

<220>
<223>  chemically synthesized

<400>  36
gagcaaaaga gcattccaag                                                    20


<210>  37
<211>  10309
<212>  DNA
<213>  artificial sequence

<220>
<223>  chemically synthesized

<400>  37
ggtaccatgg agacagacac actcctgcta tgggtactgc tgctctgggt tccaggttcc      60

actggtgacg cggatccggc ccaggcggcc ttaattaaag gtttaaacgg ccaggccggc     120

cgcaagctta ctcacacatg cccaccgtgc ccagcacctg aagccgaggg ggcaccgtca     180

gtcttcctct tccccccaaa acccaaggac accctcatga tctcccggac ccctgaggtc     240

acatgcgtgg tggtggacgt gagccacgaa gaccctgagg tcaagttcaa ctggtacgtg     300

gacggcgtgg aggtgcataa tgccaagaca aagccgcggg aggagcagta caacagcacg     360

taccgtgtgg tcagcgtcct caccgtcctg caccaggact ggctgaatgg caaggagtac     420

aagtgcaagg tctccaacaa agccctccca gcctccatcg agaaaaccat ctccaaagcc     480

aaagggcagc cccgagaacc acaggtgtac accctgcccc catcccggga tgagctgacc     540

aagaaccagg tcagcctgac ctgcctggtc aaaggcttct atcccagcga catcgccgtg     600

gagtgggaga gcaatgggca gccggagaac aactacaaga ccacgcctcc cgtgttggac     660

tccgacggct ccttcttcct ctacagcaag ctcaccgtgg acaagagcag gtggcagcag     720

gggaacgtct tctcatgctc cgtgatgcat gaggctctgc acaaccacta cacgcagaag     780

agcctctccc tgtctccggg taaatgactc gaggcccgaa caaaaactca tctcagaaga     840

ggatctgaat agcgccgtcg accatcatca tcatcatcat tgagtttaac gatccagaca     900

tgataagata cattgatgag tttggacaaa ccacaactag aatgcagtga aaaaaatgct     960

ttatttgtga aatttgtgat gctattgctt tatttgtaac cattataagc tgcaataaac    1020

aagttaacaa caacaattgc attcatttta tgtttcaggt tcagggggag gtggggaggt    1080

tttttaaagc aagtaaaacc tctacaaatg tggtatggct gattatgatc cggctgcctc    1140

gcgcgtttcg gtgatgacgg tgaaaacctc tgacacatgc agctcccgga gacggtcaca    1200

gcttgtctgt aagcggatgc cgggagcaga caagcccgtc agggcgcgtc agcgggtgtt    1260

ggcgggtgtc ggggcgcagc catgaggtcg actctagagg atcgatcccc gccgccggac    1320

gaactaaacc tgactacggc atctctgccc cttcttcgcg gggcagtgca tgtaatccct 1380

tcagttggtt ggtacaactt gccaactggg ccctgttcca catgtgacac ggggggggac 1440

caaacacaaa ggggttctct gactgtagtt gacatcctta taaatggatg tgcacatttg 1500

ccaacactga gtggctttca tcctggagca gactttgcag tctgtggact gcaacacaac 1560

attgccttta tgtgtaactc ttggctgaag ctcttacacc aatgctgggg gacatgtacc 1620

tcccaggggc ccaggaagac tacgggaggc tacaccaacg tcaatcagag gggcctgtgt 1680

agctaccgat aagcggaccc tcaagagggc attagcaata gtgtttataa ggcccccttg 1740

ttaaccctaa acgggtagca tatgcttccc gggtagtagt atatactatc cagactaacc 1800

ctaattcaat agcatatgtt acccaacggg aagcatatgc tatcgaatta gggttagtaa 1860

aagggtccta aggaacagcg atatctccca ccccatgagc tgtcacggtt ttatttacat 1920

ggggtcagga ttccacgagg gtagtgaacc attttagtca caagggcagt ggctgaagat 1980

caaggagcgg gcagtgaact ctcctgaatc ttcgcctgct tcttcattct ccttcgttta 2040

gctaatagaa taactgctga gttgtgaaca gtaaggtgta tgtgaggtgc tcgaaaacaa 2100

ggtttcaggt gacgccccca gaataaaatt tggacggggg gttcagtggt ggcattgtgc 2160

tatgacacca atataaccct cacaaacccc ttgggcaata aatactagtg taggaatgaa 2220

acattctgaa tatctttaac aatagaaatc catggggtgg ggacaagccg taaagactgg 2280

atgtccatct cacacgaatt tatggctatg ggcaacacat aatcctagtg caatatgata 2340

ctggggttat taagatgtgt cccaggcagg gaccaagaca ggtgaaccat gttgttacac 2400

tctatttgta acaaggggaa agagagtgga cgccgacagc agcggactcc actggttgtc 2460

tctaacaccc ccgaaaatta aacggggctc cacgccaatg gggcccataa acaaagacaa 2520

gtggccactc ttttttttga aattgtggag tgggggcacg cgtcagcccc cacacgccgc 2580

cctgcggttt tggactgtaa aataagggtg taataacttg gctgattgta accccgctaa 2640

ccactgcggt caaaccactt gcccacaaaa ccactaatgg caccccgggg aatacctgca 2700

taagtaggtg ggcgggccaa gataggggcg cgattgctgc gatctggagg acaaattaca 2760

cacacttgcg cctgagcgcc aagcacaggg ttgttggtcc tcatattcac gaggtcgctg 2820

agagcacggt gggctaatgt tgccatgggt agcatatact acccaaatat ctggatagca 2880

tatgctatcc taatctatat ctgggtagca taggctatcc taatctatat ctgggtagca 2940

tatgctatcc taatctatat ctgggtagta tatgctatcc taatttatat ctgggtagca 3000

taggctatcc taatctatat ctgggtagca tatgctatcc taatctatat ctgggtagta 3060

tatgctatcc taatctgtat ccgggtagca tatgctatcc taatagagat tagggtagta 3120

tatgctatcc taatttatat ctgggtagca tatactaccc aaatatctgg atagcatatg 3180
ctatcctaat ctatatctgg gtagcatatg ctatcctaat ctatatctgg gtagcatagg 3240
ctatcctaat ctatatctgg gtagcatatg ctatcctaat ctatatctgg gtagtatatg 3300
ctatcctaat ttatatctgg gtagcatagg ctatcctaat ctatatctgg gtagcatatg 3360
ctatcctaat ctatatctgg gtagtatatg ctatcctaat ctgtatccgg gtagcatatg 3420
ctatcctcat gcatatacag tcagcatatg atacccagta gtagagtggg agtgctatcc 3480
tttgcatatg ccgccacctc ccaagggggc gtgaattttc gctgcttgtc cttttcctgc 3540
atgctggttg ctcccattct taggtgaatt taaggaggcc aggctaaagc cgtcgcatgt 3600
ctgattgctc accaggtaaa tgtcgctaat gttttccaac gcgagaaggt gttgagcgcg 3660
gagctgagtg acgtgacaac atgggtatgc ccaattgccc catgttggga ggacgaaaat 3720
ggtgacaaga cagatggcca gaaatacacc aacagcacgc atgatgtcta ctggggattt 3780
attctttagt gcgggggaat acacggcttt taatacgatt gagggcgtct cctaacaagt 3840
tacatcactc ctgcccttcc tcaccctcat ctccatcacc tccttcatct ccgtcatctc 3900
cgtcatcacc ctccgcggca gccccttcca ccataggtgg aaaccaggga ggcaaatcta 3960
ctccatcgtc aaagctgcac acagtcaccc tgatattgca ggtaggagcg ggctttgtca 4020
taacaaggtc cttaatcgca tccttcaaaa cctcagcaaa tatatgagtt tgtaaaaaga 4080
ccatgaaata acagacaatg gactccctta gcgggccagg ttgtgggccg ggtccagggg 4140
ccattccaaa ggggagacga ctcaatggtg taagacgaca ttgtggaata gcaagggcag 4200
ttcctcgcct taggttgtaa agggaggtct tactacctcc atatacgaac acaccggcga 4260
cccaagttcc ttcgtcggta gtcctttcta cgtgactcct agccaggaga gctcttaaac 4320
cttctgcaat gttctcaaat ttcgggttgg aacctccttg accacgatgc tttccaaacc 4380
accctccttt tttgcgcctg cctccatcac cctgaccccg gggtccagtg cttgggcctt 4440
ctcctgggtc atctgcgggg ccctgctcta tcgctcccgg gggcacgtca ggctcaccat 4500
ctgggccacc ttcttggtgg tattcaaaat aatcggcttc ccctacaggg tggaaaaatg 4560
gccttctacc tggagggggc ctgcgcggtg gagacccgga tgatgatgac tgactactgg 4620
gactcctggg cctcttttct ccacgtccac gacctctccc cctggctctt tcacgacttc 4680
cccccctggc tctttcacgt cctctacccc ggcggcctcc actacctcct cgaccccggc 4740
ctccactacc tcctcgaccc cggcctccac tgcctcctcg accccggcct ccacctcctg 4800
ctcctgcccc tcctgctcct gcccctcctc ctgctcctgc ccctcctgcc cctcctgctc 4860
ctgcccctcc tgcccctcct gctcctgccc ctcctgcccc tcctgctcct gcccctcctg 4920
cccctcctcc tgctcctgcc cctcctgccc ctcctcctgc tcctgcccct cctgcccctc 4980

ctgctcctgc ccctcctgcc cctcctgctc ctgcccctcc tgcccctcct gctcctgccc 5040

ctcctgctcc tgcccctcct gctcctgccc ctcctgctcc tgcccctcct gcccctcctg 5100

cccctcctcc tgctcctgcc cctcctgctc ctgcccctcc tgcccctcct gcccctcctg 5160

ctcctgcccc tcctcctgct cctgcccctc ctgcccctcc tgcccctcct cctgctcctg 5220

cccctcctgc ccctcctcct gctcctgccc ctcctcctgc tcctgcccct cctgcccctc 5280

ctgcccctcc tcctgctcct gcccctcctg cccctcctcc tgctcctgcc cctcctcctg 5340

ctcctgcccc tcctgcccct cctgcccctc ctcctgctcc tgcccctcct cctgctcctg 5400

cccctcctgc ccctcctgcc cctcctgccc ctcctcctgc tcctgcccct cctcctgctc 5460

ctgcccctcc tgctcctgcc cctcccgctc ctgctcctgc tcctgttcca ccgtgggtcc 5520

ctttgcagcc aatgcaactt ggacgttttt ggggtctccg gacaccatct ctatgtcttg 5580

gccctgatcc tgagccgccc ggggctcctg gtcttccgcc tcctcgtcct cgtcctcttc 5640

cccgtcctcg tccatggtta tcaccccctc ttctttgagg tccactgccg ccggagcctt 5700

ctggtccaga tgtgtctccc ttctctccta ggccatttcc aggtcctgta cctggcccct 5760

cgtcagacat gattcacact aaaagagatc aatagacatc tttattagac gacgctcagt 5820

gaatacaggg agtgcagact cctgccccct ccaacagccc ccccaccctc atccccttca 5880

tggtcgctgt cagacagatc caggtctgaa aattccccat cctccgaacc atcctcgtcc 5940

tcatcaccaa ttactcgcag cccggaaaac tcccgctgaa catcctcaag atttgcgtcc 6000

tgagcctcaa gccaggcctc aaattcctcg tccccctttt tgctggacgg tagggatggg 6060

gattctcggg acccctcctc ttcctcttca aggtcaccag acagagatgc tactggggca 6120

acggaagaaa agctgggtgc ggcctgtgag gatcagctta tcgatgataa gctgtcaaac 6180

atgagaattc ttgaagacga aagggcctcg tgatacgcct atttttatag gttaatgtca 6240

tgataataat ggtttcttag acgtcaggtg gcacttttcg gggaaatgtg cgcggaaccc 6300

ctatttgttt atttttctaa atacattcaa atatgtatcc gctcatgaga caataaccct 6360

gataaatgct tcaataatat tgaaaaagga agagtatgag tattcaacat ttccgtgtcg 6420

cccttattcc cttttttgcg gcattttgcc ttcctgtttt tgctcaccca gaaacgctgg 6480

tgaaagtaaa agatgctgaa gatcagttgg gtgcacgagt gggttacatc gaactggatc 6540

tcaacagcgg taagatcctt gagagttttc gccccgaaga acgttttcca atgatgagca 6600

cttttaaagt tctgctatgt ggcgcggtat tatcccgtgt tgacgccggg caagagcaac 6660

tcggtcgccg catacactat tctcagaatg acttggttga gtactcacca gtcacagaaa 6720

agcatcttac ggatggcatg acagtaagag aattatgcag tgctgccata accatgagtg 6780

ataacactgc ggccaactta cttctgacaa cgatcggagg accgaaggag ctaaccgctt 6840
ttttgcacaa catgggggat catgtaactc gccttgatcg ttgggaaccg gagctgaatg 6900
aagccatacc aaacgacgag cgtgacacca cgatgcctgc agcaatggca acaacgttgc 6960
gcaaactatt aactggcgaa ctacttactc tagcttcccg gcaacaatta atagactgga 7020
tggaggcgga taaagttgca ggaccacttc tgcgctcggc ccttccggct ggctggttta 7080
ttgctgataa atctggagcc ggtgagcgtg ggtctcgcgg tatcattgca gcactggggc 7140
cagatggtaa gccctcccgt atcgtagtta tctacacgac ggggagtcag gcaactatgg 7200
atgaacgaaa tagacagatc gctgagatag gtgcctcact gattaagcat tggtaactgt 7260
cagaccaagt ttactcatat atactttaga ttgatttaaa acttcatttt taatttaaaa 7320
ggatctaggt gaagatcctt tttgataatc tcatgaccaa aatcccttaa cgtgagtttt 7380
cgttccactg agcgtcagac cccgtagaaa agatcaaagg atcttcttga gatccttttt 7440
ttctgcgcgt aatctgctgc ttgcaaacaa aaaaaccacc gctaccagcg gtggtttgtt 7500
tgccggatca agagctacca actctttttc cgaaggtaac tggcttcagc agagcgcaga 7560
taccaaatac tgtccttcta gtgtagccgt agttaggcca ccacttcaag aactctgtag 7620
caccgcctac atacctcgct ctgctaatcc tgttaccagt ggctgctgcc agtggcgata 7680
agtcgtgtct taccgggttg gactcaagac gatagttacc ggataaggcg cagcggtcgg 7740
gctgaacggg gggttcgtgc acacagccca gcttggagcg aacgacctac accgaactga 7800
gatacctaca gcgtgagcta tgagaaagcg ccacgcttcc cgaagggaga aaggcggaca 7860
ggtatccggt aagcggcagg gtcggaacag gagagcgcac gagggagctt ccagggggaa 7920
acgcctggta tctttatagt cctgtcgggt ttcgccacct ctgacttgag cgtcgatttt 7980
tgtgatgctc gtcagggggg cggagcctat ggaaaaacgc cagcaacgcg gcctttttac 8040
ggttcctggc cttttgctgc gccgcgtgcg gctgctggag atggcggacg cgatggatat 8100
gttctgccaa gggttggttt gcgcattcac agttctccgc aagaattgat tggctccaat 8160
tcttggagtg gtgaatccgt tagcgaggcc atccagcctc gcgtcgaact agatgatccg 8220
ctgtggaatg tgtgtcagtt agggtgtgga aagtccccag gctccccagc aggcagaagt 8280
atgcaaagca tgcatctcaa ttagtcagca accaggtgtg gaaagtcccc aggctcccca 8340
gcaggcagaa gtatgcaaag catgcatctc aattagtcag caaccatagt cccgccccta 8400
actccgccca tcccgcccct aactccgccc agttccgccc attctccgcc ccatggctga 8460
ctaatttttt ttatttatgc agaggccgag gccgcggcct ctgagctatt ccagaagtag 8520
tgaggaggct tttttggagg gtgaccgcca cgaggtgccg ccaccatccc ctgacccacg 8580
cccctgaccc ctcacaagga gacgaccttc catgaccgag tacaagccca cggtgcgcct 8640

cgccacccgc gacgacgtcc cccgggccgt acgcaccctc gccgccgcgt tcgccgacta 8700

ccccgccacg cgccacaccg tcgaccccga ccgccacatc gaacgcgtca ccgagctgca 8760

agaactcttc ctcacgcgcg tcgggctcga catcggcaag gtgtgggtcg cggacgacgg 8820

cgccgcggtg gcggtctgga ccacgccgga gagcgtcgaa gcgggggcgg tgttcgccga 8880

gatcggcccg cgcatggccg agttgagcgg ttcccggctg gccgcgcagc aacagatgga 8940

aggcctcctg gcgccgcacc ggcccaagga gcccgcgtgg ttcctggcca ccgtcggcgt 9000

ctcgcccgac caccagggca agggtctggg cagcgccgtc gtgctccccg gagtggaggc 9060

ggccgagcgc gccggggtgc ccgccttcct ggagacctcc gcgccccgca acctcccctt 9120

ctacgagcgg ctcggcttca ccgtcaccgc cgacgtcgag tgcccgaagg accgcgcgac 9180

ctggtgcatg acccgcaagc ccggtgcctg acgcccgccc cacgacccgc agcgcccgac 9240

cgaaaggagc gcacgacccg gtccgacggc ggcccacggg tcccaggggg gtcgacctcg 9300

aaacttgttt attgcagctt ataatggtta caaataaagc aatagcatca caaatttcac 9360

aaataaagca tttttttcac tgcattctag ttgtggtttg tccaaactca tcaatgtatc 9420

ttatcatgtc tggatcgatc cgaacccctt cctcgaccaa ttctcatgtt tgacagctta 9480

tcatcgcaga tccgggcaac gttgttgcat tgctgcaggc gcagaactgg taggtatgga 9540

agatctatac attgaatcaa tattggcaat tagccatatt agtcattggt tatatagcat 9600

aaatcaatat tggctattgg ccattgcata cgttgtatct atatcataat atgtacattt 9660

atattggctc atgtccaata tgaccgccat gttgacattg attattgact agttattaat 9720

agtaatcaat tacggggtca ttagttcata gcccatatat ggagttccgc gttacataac 9780

ttacggtaaa tggcccgcct ggctgaccgc ccaacgaccc ccgcccattg acgtcaataa 9840

tgacgtatgt tcccatagta acgccaatag ggactttcca ttgacgtcaa tgggtggagt 9900

atttacggta aactgcccac ttggcagtac atcaagtgta tcatatgcca agtccgcccc 9960

ctattgacgt caatgacggt aaatggcccg cctggcatta tgcccagtac atgaccttac 10020

gggactttcc tacttggcag tacatctacg tattagtcat cgctattacc atggtgatgc 10080

ggttttggca gtacaccaat gggcgtggat agcggtttga ctcacgggga tttccaagtc 10140

tccaccccat tgacgtcaat gggagtttgt tttggcacca aaatcaacgg gactttccaa 10200

aatgtcgtaa taaccccgcc ccgttgacgc aaatgggcgg taggcgtgta cggtgggagg 10260

tctatataag cagagctcgt ttagtgaacc gtcagatctc tagaagctg 10309

<210> 38
<211> 10215
<212> DNA

<213>  artificial sequence

<220>
<223>  chemically synthesized

<400>  38
gagctcgtat ggacatattg tcgttagaac gcggctacaa ttaatacata accttatgta      60

tcatacacat acgatttagg ggacactata gattgacggc gtagtacaca ctattgaatc     120

aaacagccga ccaattgcac taccatcaca atggagaagc cagtagtaaa cgtagacgta     180

gacccccaga gtccgtttgt cgtgcaactg caaaaaagct tcccgcaatt tgaggtagta     240

gcacagcagg tcactccaaa tgaccatgct aatgccagag cattttcgca tctggccagt     300

aaactaatcg agctggaggt tcctaccaca gcgacgatct tggacatagg cagcgcaccg     360

gctcgtagaa tgttttccga gcaccagtat cattgtgtct gccccatgcg tagtccagaa     420

gacccggacc gcatgatgaa atacgccagt aaactggcgg aaaaagcgtg caagattaca     480

aacaagaact tgcatgagaa gattaaggat ctccggaccg tacttgatac gccggatgct     540

gaaacaccat cgctctgctt tcacaacgat gttacctgca acatgcgtgc cgaatattcc     600

gtcatgcagg acgtgtatat caacgctccc ggaactatct atcatcaggc tatgaaaggc     660

gtgcggaccc tgtactggat tggcttcgac accacccagt tcatgttctc ggctatggca     720

ggttcgtacc ctgcgtacaa caccaactgg gccgacgaga aagtccttga agcgcgtaac     780

atcggacttt gcagcacaaa gctgagtgaa ggtaggacag gaaaattgtc gataatgagg     840

aagaaggagt tgaagcccgg gtcgcgggtt tatttctccg taggatcgac actttatcca     900

gaacacagag ccagcttgca gagctggcat cttccatcgg tgttccactt gaatggaaag     960

cagtcgtaca cttgccgctg tgatacagtg gtgagttgcg aaggctacgt agtgaagaaa    1020

atcaccatca gtcccgggat cacgggagaa accgtgggat acgcggttac acacaatagc    1080

gagggcttct tgctatgcaa agttactgac acagtaaaag gagaacgggt atcgttccct    1140

gtgtgcacgt acatcccggc caccatatgc gatcagatga ctggtataat ggccacggat    1200

atatcacctg acgatgcaca aaaacttctg gttgggctca accagcgaat tgtcattaac    1260

ggtaggacta acaggaacac caacaccatg caaaattacc ttctgccgat catagcacaa    1320

gggttcagca aatgggctaa ggagcgcaag gatgatcttg ataacgagaa aatgctgggt    1380

actagagaac gcaagcttac gtatggctgc ttgtgggcgt ttcgcactaa gaaagtacat    1440

tcgttttatc gcccacctgg aacgcagacc tgcgtaaaag tcccagcctc ttttagcgct    1500

tttcccatgt cgtccgtatg gacgacctct ttgcccatgt cgctgaggca gaaattgaaa    1560

ctggcattgc aaccaaagaa ggaggaaaaa ctgctgcagg tctcggagga attagtcatg    1620

gaggccaagg ctgcttttga ggatgctcag gaggaagcca gagcggagaa gctccgagaa    1680

gcacttccac cattagtggc agacaaaggc atcgaggcag ccgcagaagt tgtctgcgaa 1740

gtggaggggc tccaggcgga catcggagca gcattagttg aaaccccgcg cggtcacgta 1800

aggataatac ctcaagcaaa tgaccgtatg atcggacagt atatcgttgt ctcgccaaac 1860

tctgtgctga agaatgccaa actcgcacca gcgcacccgc tagcagatca ggttaagatc 1920

ataacacact ccggaagatc aggaaggtac gcggtcgaac catacgacgc taaagtactg 1980

atgccagcag gaggtgccgt accatggcca gaattcctag cactgagtga gagcgccacg 2040

ttagtgtaca acgaaagaga gtttgtgaac cgcaaactat accacattgc catgcatggc 2100

cccgccaaga atacagaaga ggagcagtac aaggttacaa aggcagagct tgcagaaaca 2160

gagtacgtgt ttgacgtgga caagaagcgt tgcgttaaga aggaagaagc ctcaggtctg 2220

gtcctctcgg gagaactgac caaccctccc tatcatgagc tagctctgga gggactgaag 2280

acccgacctg cggtcccgta caaggtcgaa acaataggag tgataggcac accggggtcg 2340

ggcaagtcag ctattatcaa gtcaactgtc acggcacgag atcttgttac cagcggaaag 2400

aaagaaaatt gtcgcgaaat tgaggccgac gtgctaagac tgaggggtat gcagattacg 2460

tcgaagacag tagattcggt tatgctcaac ggatgccaca aagccgtaga agtgctgtac 2520

gttgacgaag cgttcgcgtg ccacgcagga gcactacttg ccttgattgc tatcgtcagg 2580

ccccgcaaga aggtagtact atgcggagac cccatgcaat gcggattctt caacatgatg 2640

caactaaagg tacatttcaa tcaccctgaa aaagacatat gcaccaagac attctacaag 2700

tatatctccc ggcgttgcac acagccagtt acagctattg tatcgacact gcattacgat 2760

ggaaagatga aaaccacgaa cccgtgcaag aagaacattg aaatcgatat tacaggggcc 2820

acaaagccga agccagggga tatcatcctg acatgtttcc gcgggtgggt taagcaattg 2880

caaatcgact atcccggaca tgaagtaatg acagccgcgg cctcacaagg gctaaccaga 2940

aaaggagtgt atgccgtccg gcaaaaagtc aatgaaaacc cactgtacgc gatcacatca 3000

gagcatgtga acgtgttgct cacccgcact gaggacaggc tagtgtggaa aaccttgcag 3060

ggcgacccat ggattaagca gcccactaac atacctaaag gaaactttca ggctactata 3120

gaggactggg aagctgaaca caagggaata attgctgcaa taaacagccc cactccccgt 3180

gccaatccgt tcagctgcaa gaccaacgtt tgctgggcga aagcattgga accgatacta 3240

gccacggccg gtatcgtact taccggttgc cagtggagcg aactgttccc acagtttgcg 3300

gatgacaaac cacattcggc catttacgcc ttagacgtaa tttgcattaa gtttttcggc 3360

atggacttga caagcggact gttttctaaa cagagcatcc cactaacgta ccatcccgcc 3420

gattcagcga ggccggtagc tcattgggac aacagcccag gaacccgcaa gtatgggtac 3480

gatcacgcca ttgccgccga actctcccgt agatttccgg tgttccagct agctgggaag 3540

ggcacacaac ttgatttgca gacggggaga accagagtta tctctgcaca gcataacctg 3600

gtcccggtga accgcaatct tcctcacgcc ttagtccccg agtacaagga gaagcaaccc 3660

ggcccggtca aaaaattctt gaaccagttc aaacaccact cagtacttgt ggtatcagag 3720

gaaaaaattg aagctccccg taagagaatc gaatggatcg ccccgattgg catagccggt 3780

gcagataaga actacaacct ggctttcggg tttccgccgc aggcacggta cgacctggtg 3840

ttcatcaaca ttggaactaa atacagaaac caccactttc agcagtgcga agaccatgcg 3900

gcgaccttaa aaaccctttc gcgttcggcc ctgaattgcc ttaacccagg aggcaccctc 3960

gtggtgaagt cctatggcta cgccgaccgc aacagtgagg acgtagtcac cgctcttgcc 4020

agaaagtttg tcagggtgtc tgcagcgaga ccagattgtg tctcaagcaa tacagaaatg 4080

tacctgattt tccgacaact agacaacagc cgtacacggc aattcacccc gcaccatctg 4140

aattgcgtga tttcgtccgt gtatgagggt acaagagatg gagttggagc cgcgccgtca 4200

taccgcacca aaagggagaa tattgctgac tgtcaagagg aagcagttgt caacgcagcc 4260

aatccgctgg gtagaccagg cgaaggagtc tgccgtgcca tctataaacg ttggccgacc 4320

agttttaccg attcagccac ggagacaggc accgcaagaa tgactgtgtg cctaggaaag 4380

aaagtgatcc acgcggtcgg ccctgatttc cggaagcacc cagaagcaga agccttgaaa 4440

ttgctacaaa acgcctacca tgcagtggca gacttagtaa atgaacataa catcaagtct 4500

gtcgccattc cactgctatc tacaggcatt tacgcagccg gaaaagaccg ccttgaagta 4560

tcacttaact gcttgacaac cgcgctagac agaactgacg cggacgtaac catctattgc 4620

ctggataaga agtggaagga aagaatcgac gcggcactcc aacttaagga gtctgtaaca 4680

gagctgaagg atgaagatat ggagatcgac gatgagttag tatggattca tccagacagt 4740

tgcttgaagg gaagaaaggg attcagtact acaaaaggaa aattgtattc gtacttcgaa 4800

ggcaccaaat tccatcaagc agcaaaagac atggcggaga taaaggtcct gttccctaat 4860

gaccaggaaa gtaatgaaca actgtgtgcc tacatattgg gtgagaccat ggaagcaatc 4920

cgcgaaaagt gcccggtcga ccataacccg tcgtctagcc cgcccaaaac gttgccgtgc 4980

ctttgcatgt atgccatgac gccagaaagg gtccacagac ttagaagcaa taacgtcaaa 5040

gaagttacag tatgctcctc cacccccctt cctaagcaca aaattaagaa tgttcagaag 5100

gttcagtgca cgaaagtagt cctgtttaat ccgcacactc ccgcattcgt tcccgcccgt 5160

aagtacatag aagtgccaga acagcctacc gctcctcctg cacaggctga ggaagccccc 5220

gaagttgtag cgacaccgtc accatctaca gctgataaca cctcgcttga tgtcacagac 5280

atctcactgg atatggatga cagtagcgaa ggctcacttt tttcgagctt tagcggatcg 5340

gacaactcta ttactagtat ggacagttgg tcgtcaggac ctagttcact agagatagta 5400

gaccgaaggc aggtggtggt ggctgacgtt catgccgtcc aagagcctgc ccctattcca 5460

ccgccaaggc taaagaagat ggcccgcctg gcagcggcaa gaaaagagcc cactccaccg 5520

gcaagcaata gctctgagtc cctccacctc tcttttggtg gggtatccat gtccctcgga 5580

tcaattttcg acggagagac ggcccgccag gcagcggtac aacccctggc aacaggcccc 5640

acggatgtgc ctatgtcttt cggatcgttt tccgacggag agattgatga gctgagccgc 5700

agagtaactg agtccgaacc cgtcctgttt ggatcatttg aaccgggcga agtgaactca 5760

attatatcgt cccgatcagc cgtatctttt ccactacgca agcagagacg tagacgcagg 5820

agcaggagga ctgaatactg actaaccggg gtaggtgggt acatattttc gacggacaca 5880

ggccctgggc acttgcaaaa gaagtccgtt ctgcagaacc agcttacaga accgaccttg 5940

gagcgcaatg tcctggaaag aattcatgcc ccggtgctcg acacgtcgaa agaggaacaa 6000

ctcaaactca ggtaccagat gatgcccacc gaagccaaca aaagtaggta ccagtctcgt 6060

aaagtagaaa atcagaaagc cataaccact gagcgactac tgtcaggact acgactgtat 6120

aactctgcca cagatcagcc agaatgctat aagatcacct atccgaaacc attgtactcc 6180

agtagcgtac cggcgaacta ctccgatcca cagttcgctg tagctgtctg taacaactat 6240

ctgcatgaga actatccgac agtagcatct tatcagatta ctgacgagta cgatgcttac 6300

ttggatatgg tagacgggac agtcgcctgc ctggatactg caaccttctg ccccgctaag 6360

cttagaagtt acccgaaaaa acatgagtat agagccccga atatccgcag tgcggttcca 6420

tcagcgatgc agaacacgct acaaaatgtg ctcattgccg caactaaaag aaattgcaac 6480

gtcacgcaga tgcgtgaact gccaacactg gactcagcga cattcaatgt cgaatgcttt 6540

cgaaaatatg catgtaatga cgagtattgg gaggagttcg ctcggaagcc aattaggatt 6600

accactgagt ttgtcaccgc atatgtagct agactgaaag gccctaaggc cgccgcacta 6660

tttgcaaaga cgtataattt ggtcccattg caagaagtgc ctatggatag attcgtcatg 6720

gacatgaaaa gagacgtgaa agttacacca ggcacgaaac acacagaaga aagaccgaaa 6780

gtacaagtga tacaagccgc agaacccctg gcgactgctt acttatgcgg gattcaccgg 6840

gaattagtgc gtaggcttac ggccgtcttg cttccaaaca ttcacacgct ttttgacatg 6900

tcggcggagg attttgatgc aatcatagca gaacacttca agcaaggcga cccggtactg 6960

gagacggata tcgcatcatt cgacaaaagc caagacgacg ctatggcgtt aaccggtctg 7020

atgatcttgg aggacctggg tgtggatcaa ccactactcg acttgatcga gtgcgccttt 7080

ggagaaatat catccaccca tctacctacg ggtactcgtt ttaaattcgg ggcgatgatg 7140

aaatccggaa tgttcctcac actttttgtc aacacagttt tgaatgtcgt tatcgccagc 7200

agagtactag aagagcggct taaaacgtcc agatgtgcag cgttcattgg cgacgacaac 7260

atcatacatg gagtagtatc tgacaaagaa atggctgaga ggtgcgccac ctggctcaac 7320

atggaggtta agatcatcga cgcagtcatc ggtgagagac caccttactt ctgcggcgga 7380

tttatcttgc aagattcggt tacttccaca gcgtgccgcg tggcggatcc cctgaaaagg 7440

ctgtttaagt tgggtaaacc gctcccagcc gacgacgagc aagacgaaga cagaagacgc 7500

gctctgctag atgaaacaaa ggcgtggttt agagtaggta taacaggcac tttagcagtg 7560

gccgtgacga cccggtatga ggtagacaat attacacctg tcctactggc attgagaact 7620

tttgcccaga gcaaaagagc attccaagcc atcagagggg aaataaagca tctctacggt 7680

ggtcctaaat agtcagcata gtacatttca tctgactaat actacaacac caccacctct 7740

agagccacca tggagacaga cacactcctg ctatgggtac tgctgctctg ggttccaggt 7800

tccactggtg actatgaggc ccaggcggcc ggtaccgcta gcggccaggc cggccgcaat 7860

gctgtgggcc aggacacgca ggaggtcatc gtggtgccac actccttgcc ctttaaggtg 7920

gtggtgatct cagccatcct ggccctggtg gtgctcacca tcatctccct tatcatcctc 7980

atcatgcttt ggcagaagaa gccacgttag ggcccgccca tcgattagtc caatttgttg 8040

gcccaatgat ccgaccagca aaactcgatg tacttccgag gaactgatgt gcataatgca 8100

tcaggctggt acattagatc cccgcttacc gcgggcaata tagcaacact aaaaactcga 8160

tgtacttccg aggaagcgca gtgcataatg ctgcgcagtg ttgccacata accactatat 8220

taaccattta tctagcggac gccaaaaact caatgtattt ctgaggaagc gtggtgcata 8280

atgccacgca gcgtctgcat aacttttatt atttctttta ttaatcaaca aaattttgtt 8340

tttaacattt caaaaaaaaa aaaaaaaaaa aaaaaaaaaa aaaaaaaagg gaattcctcg 8400

attaattaag cggccgctcg aggggaatta attcttgaag acgaaagggc caggtggcac 8460

ttttcgggga aatgtgcgcg gaacccctat ttgtttattt ttctaaatac attcaaatat 8520

gtatccgctc atgagacaat aaccctgata aatgcttcaa taatattgaa aaaggaagag 8580

tatgagtatt caacatttcc gtgtcgccct tattcccttt tttgcggcat tttgccttcc 8640

tgtttttgct cacccagaaa cgctggtgaa agtaaaagat gctgaagatc agttgggtgc 8700

acgagtgggt tacatcgaac tggatctcaa cagcggtaag atccttgaga gttttcgccc 8760

cgaagaacgt tttccaatga tgagcacttt taaagttctg ctatgtggcg cggtattatc 8820

ccgtgttgac gccgggcaag agcaactcgg tcgccgcata cactattctc agaatgactt 8880

ggttgagtac tcaccagtca cagaaaagca tcttacggat ggcatgacag taagagaatt 8940

atgcagtgct gccataacca tgagtgataa cactgcggcc aacttacttc tgacaacgat 9000

cggaggaccg aaggagctaa ccgctttttt gcacaacatg ggggatcatg taactcgcct 9060

tgatcgttgg gaaccggagc tgaatgaagc cataccaaac gacgagcgtg acaccacgat 9120

gcctgtagca atggcaacaa cgttgcgcaa actattaact ggcgaactac ttactctagc 9180

ttcccggcaa caattaatag actggatgga ggcggataaa gttgcaggac cacttctgcg 9240

ctcggccctt ccggctggct ggtttattgc tgataaatct ggagccggtg agcgtgggtc 9300

tcgcggtatc attgcagcac tggggccaga tggtaagccc tcccgtatcg tagttatcta 9360

cacgacgggg agtcaggcaa ctatggatga acgaaataga cagatcgctg agataggtgc 9420

ctcactgatt aagcattggt aactgtcaga ccaagtttac tcatatatac tttagattga 9480

tttaaaactt catttttaat ttaaaaggat ctaggtgaag atcctttttg ataatctcat 9540

gaccaaaatc ccttaacgtg agttttcgtt ccactgagcg tcagaccccg tagaaaagat 9600

caaaggatct tcttgagatc ctttttttct gcgcgtaatc tgctgcttgc aaacaaaaaa 9660

accaccgcta ccagcggtgg tttgtttgcc ggatcaagag ctaccaactc tttttccgaa 9720

ggtaactggc ttcagcagag cgcagatacc aaatactgtc cttctagtgt agccgtagtt 9780

aggccaccac ttcaagaact ctgtagcacc gcctacatac ctcgctctgc taatcctgtt 9840

accagtggct gctgccagtg gcgataagtc gtgtcttacc gggttggact caagacgata 9900

gttaccggat aaggcgcagc ggtcgggctg aacggggggt tcgtgcacac agcccagctt 9960

ggagcgaacg acctacaccg aactgagata cctacagcgt gagcattgag aaagcgccac 10020

gcttcccgaa gggagaaagg cggacaggta tccggtaagc ggcagggtcg gaacaggaga 10080

gcgcacgagg gagcttccag ggggaaacgc ctggtatctt tatagtcctg tcgggtttcg 10140

ccacctctga cttgagcgtc gatttttgtg atgctcgtca ggggggcgga gcctatggaa 10200

aaacgccagc aacgc 10215

<210> 39
<211> 10245
<212> DNA
<213> artificial sequence

<220>
<223> chemically synthesized

<400> 39
gagctcgtat ggacatattg tcgttagaac gcggctacaa ttaatacata accttatgta 60

tcatacacat acgatttagg ggacactata gattgacggc gtagtacaca ctattgaatc 120

aaacagccga ccaattgcac taccatcaca atggagaagc cagtagtaaa cgtagacgta 180

gacccccaga gtccgtttgt cgtgcaactg caaaaaagct tcccgcaatt tgaggtagta 240

gcacagcagg tcactccaaa tgaccatgct aatgccagag cattttcgca tctggccagt 300

aaactaatcg agctggaggt tcctaccaca gcgacgatct tggacatagg cagcgcaccg 360

gctcgtagaa tgttttccga gcaccagtat cattgtgtct gccccatgcg tagtccagaa 420

gacccggacc gcatgatgaa atacgccagt aaactggcgg aaaaagcgtg caagattaca 480

aacaagaact tgcatgagaa gattaaggat ctccggaccg tacttgatac gccggatgct 540

gaaacaccat cgctctgctt tcacaacgat gttacctgca acatgcgtgc cgaatattcc 600

gtcatgcagg acgtgtatat caacgctccc ggaactatct atcatcaggc tatgaaaggc 660

gtgcggaccc tgtactggat tggcttcgac accacccagt tcatgttctc ggctatggca 720

ggttcgtacc ctgcgtacaa caccaactgg gccgacgaga aagtccttga agcgcgtaac 780

atcggacttt gcagcacaaa gctgagtgaa ggtaggacag gaaaattgtc gataatgagg 840

aagaaggagt tgaagcccgg gtcgcgggtt tatttctccg taggatcgac actttatcca 900

gaacacagag ccagcttgca gagctggcat cttccatcgg tgttccactt gaatggaaag 960

cagtcgtaca cttgccgctg tgatacagtg gtgagttgcg aaggctacgt agtgaagaaa 1020

atcaccatca gtcccgggat cacgggagaa accgtgggat acgcggttac acacaatagc 1080

gagggcttct tgctatgcaa agttactgac acagtaaaag gagaacgggt atcgttccct 1140

gtgtgcacgt acatcccggc caccatatgc gatcagatga ctggtataat ggccacggat 1200

atatcacctg acgatgcaca aaaacttctg gttgggctca accagcgaat tgtcattaac 1260

ggtaggacta acaggaacac caacaccatg caaaattacc ttctgccgat catagcacaa 1320

gggttcagca aatgggctaa ggagcgcaag gatgatcttg ataacgagaa aatgctgggt 1380

actagagaac gcaagcttac gtatggctgc ttgtgggcgt ttcgcactaa gaaagtacat 1440

tcgttttatc gcccacctgg aacgcagacc tgcgtaaaag tcccagcctc ttttagcgct 1500

tttcccatgt cgtccgtatg gacgacctct ttgcccatgt cgctgaggca gaaattgaaa 1560

ctggcattgc aaccaaagaa ggaggaaaaa ctgctgcagg tctcggagga attagtcatg 1620

gaggccaagg ctgcttttga ggatgctcag gaggaagcca gagcggagaa gctccgagaa 1680

gcacttccac cattagtggc agacaaaggc atcgaggcag ccgcagaagt tgtctgcgaa 1740

gtggaggggc tccaggcgga catcggagca gcattagttg aaaccccgcg cggtcacgta 1800

aggataatac ctcaagcaaa tgaccgtatg atcggacagt atatcgttgt ctcgccaaac 1860

tctgtgctga agaatgccaa actcgcacca gcgcacccgc tagcagatca ggttaagatc 1920

ataacacact ccggaagatc aggaaggtac gcggtcgaac catacgacgc taaagtactg 1980

atgccagcag gaggtgccgt accatggcca gaattcctag cactgagtga gagcgccacg 2040

ttagtgtaca acgaaagaga gtttgtgaac cgcaaactat accacattgc catgcatggc 2100

cccgccaaga atacagaaga ggagcagtac aaggttacaa aggcagagct tgcagaaaca 2160

gagtacgtgt ttgacgtgga caagaagcgt tgcgttaaga aggaagaagc ctcaggtctg 2220

gtcctctcgg gagaactgac caaccctccc tatcatgagc tagctctgga gggactgaag 2280

acccgacctg cggtcccgta caaggtcgaa acaataggag tgataggcac accggggtcg 2340

ggcaagtcag ctattatcaa gtcaactgtc acggcacgag atcttgttac cagcggaaag 2400

aaagaaaatt gtcgcgaaat tgaggccgac gtgctaagac tgaggggtat gcagattacg 2460

tcgaagacag tagattcggt tatgctcaac ggatgccaca aagccgtaga agtgctgtac 2520

gttgacgaag cgttcgcgtg ccacgcagga gcactacttg ccttgattgc tatcgtcagg 2580

ccccgcaaga aggtagtact atgcggagac cccatgcaat gcggattctt caacatgatg 2640

caactaaagg tacatttcaa tcaccctgaa aaagacatat gcaccaagac attctacaag 2700

tatatctccc ggcgttgcac acagccagtt acagctattg tatcgacact gcattacgat 2760

ggaaagatga aaaccacgaa cccgtgcaag aagaacattg aaatcgatat tacaggggcc 2820

acaaagccga agccagggga tatcatcctg acatgtttcc gcgggtgggt taagcaattg 2880

caaatcgact atcccggaca tgaagtaatg acagccgcgg cctcacaagg gctaaccaga 2940

aaaggagtgt atgccgtccg gcaaaaagtc aatgaaaacc cactgtacgc gatcacatca 3000

gagcatgtga acgtgttgct cacccgcact gaggacaggc tagtgtggaa aaccttgcag 3060

ggcgacccat ggattaagca gcccactaac atacctaaag gaaactttca ggctactata 3120

gaggactggg aagctgaaca caagggaata attgctgcaa taaacagccc cactccccgt 3180

gccaatccgt tcagctgcaa gaccaacgtt tgctgggcga aagcattgga accgatacta 3240

gccacggccg gtatcgtact taccggttgc cagtggagcg aactgttccc acagtttgcg 3300

gatgacaaac cacattcggc catttacgcc ttagacgtaa tttgcattaa gtttttcggc 3360

atggacttga caagcggact gttttctaaa cagagcatcc cactaacgta ccatcccgcc 3420

gattcagcga ggccggtagc tcattgggac aacagcccag gaacccgcaa gtatgggtac 3480

gatcacgcca ttgccgccga actctcccgt agatttccgg tgttccagct agctgggaag 3540

ggcacacaac ttgatttgca gacggggaga accagagtta tctctgcaca gcataacctg 3600

gtcccggtga accgcaatct tcctcacgcc ttagtccccg agtacaagga gaagcaaccc 3660

ggcccggtca aaaaattctt gaaccagttc aaacaccact cagtacttgt ggtatcagag 3720

gaaaaaattg aagctccccg taagagaatc gaatggatcg ccccgattgg catagccggt 3780

gcagataaga actacaacct ggctttcggg tttccgccgc aggcacggta cgacctggtg 3840

ttcatcaaca ttggaactaa atacagaaac caccactttc agcagtgcga agaccatgcg 3900

gcgaccttaa aaaccctttc gcgttcggcc ctgaattgcc ttaacccagg aggcaccctc 3960

gtggtgaagt cctatggcta cgccgaccgc aacagtgagg acgtagtcac cgctcttgcc 4020

agaaagtttg tcagggtgtc tgcagcgaga ccagattgtg tctcaagcaa tacagaaatg 4080

tacctgattt tccgacaact agacaacagc cgtacacggc aattcacccc gcaccatctg 4140

aattgcgtga tttcgtccgt gtatgagggt acaagagatg gagttggagc cgcgccgtca 4200

taccgcacca aaagggagaa tattgctgac tgtcaagagg aagcagttgt caacgcagcc 4260

aatccgctgg gtagaccagg cgaaggagtc tgccgtgcca tctataaacg ttggccgacc 4320

agttttaccg attcagccac ggagacaggc accgcaagaa tgactgtgtg cctaggaaag 4380

aaagtgatcc acgcggtcgg ccctgatttc cggaagcacc cagaagcaga agccttgaaa 4440

ttgctacaaa acgcctacca tgcagtggca gacttagtaa atgaacataa catcaagtct 4500

gtcgccattc cactgctatc tacaggcatt tacgcagccg gaaaagaccg ccttgaagta 4560

tcacttaact gcttgacaac cgcgctagac agaactgacg cggacgtaac catctattgc 4620

ctggataaga agtggaagga aagaatcgac gcggcactcc aacttaagga gtctgtaaca 4680

gagctgaagg atgaagatat ggagatcgac gatgagttag tatggattca tccagacagt 4740

tgcttgaagg gaagaaaggg attcagtact acaaaaggaa aattgtattc gtacttcgaa 4800

ggcaccaaat tccatcaagc agcaaaagac atggcggaga taaaggtcct gttccctaat 4860

gaccaggaaa gtaatgaaca actgtgtgcc tacatattgg gtgagaccat ggaagcaatc 4920

cgcgaaaagt gcccggtcga ccataacccg tcgtctagcc cgcccaaaac gttgccgtgc 4980

ctttgcatgt atgccatgac gccagaaagg gtccacagac ttagaagcaa taacgtcaaa 5040

gaagttacag tatgctcctc cacccccctt cctaagcaca aaattaagaa tgttcagaag 5100

gttcagtgca cgaaagtagt cctgtttaat ccgcacactc ccgcattcgt tcccgcccgt 5160

aagtacatag aagtgccaga acagcctacc gctcctcctg cacaggctga ggaagccccc 5220

gaagttgtag cgacaccgtc accatctaca gctgataaca cctcgcttga tgtcacagac 5280

atctcactgg atatggatga cagtagcgaa ggctcacttt tttcgagctt tagcggatcg 5340

gacaactcta ttactagtat ggacagttgg tcgtcaggac ctagttcact agagatagta 5400

gaccgaaggc aggtggtggt ggctgacgtt catgccgtcc aagagcctgc ccctattcca 5460

ccgccaaggc taaagaagat ggcccgcctg gcagcggcaa gaaaagagcc cactccaccg 5520

gcaagcaata gctctgagtc cctccacctc tcttttggtg gggtatccat gtccctcgga 5580

tcaattttcg acggagagac ggcccgccag gcagcggtac aacccctggc aacaggcccc 5640

acggatgtgc ctatgtcttt cggatcgttt tccgacggag agattgatga gctgagccgc 5700

agagtaactg agtccgaacc cgtcctgttt ggatcatttg aaccgggcga agtgaactca 5760

attatatcgt cccgatcagc cgtatctttt ccactacgca agcagagacg tagacgcagg 5820

agcaggagga ctgaatactg actaaccggg gtaggtgggt acatattttc gacggacaca 5880

ggccctgggc acttgcaaaa gaagtccgtt ctgcagaacc agcttacaga accgaccttg 5940

gagcgcaatg tcctggaaag aattcatgcc ccggtgctcg acacgtcgaa agaggaacaa 6000

ctcaaactca ggtaccagat gatgcccacc gaagccaaca aaagtaggta ccagtctcgt 6060

aaagtagaaa atcagaaagc cataaccact gagcgactac tgtcaggact acgactgtat 6120

aactctgcca cagatcagcc agaatgctat aagatcacct atccgaaacc attgtactcc 6180

agtagcgtac cggcgaacta ctccgatcca cagttcgctg tagctgtctg taacaactat 6240

ctgcatgaga actatccgac agtagcatct tatcagatta ctgacgagta cgatgcttac 6300

ttggatatgg tagacgggac agtcgcctgc ctggatactg caaccttctg ccccgctaag 6360

cttagaagtt acccgaaaaa acatgagtat agagccccga atatccgcag tgcggttcca 6420

tcagcgatgc agaacacgct acaaaatgtg ctcattgccg caactaaaag aaattgcaac 6480

gtcacgcaga tgcgtgaact gccaacactg gactcagcga cattcaatgt cgaatgcttt 6540

cgaaaatatg catgtaatga cgagtattgg gaggagttcg ctcggaagcc aattaggatt 6600

accactgagt ttgtcaccgc atatgtagct agactgaaag gccctaaggc cgccgcacta 6660

tttgcaaaga cgtataattt ggtcccattg caagaagtgc ctatggatag attcgtcatg 6720

gacatgaaaa gagacgtgaa agttacacca ggcacgaaac acacagaaga aagaccgaaa 6780

gtacaagtga tacaagccgc agaacccctg gcgactgctt acttatgcgg gattcaccgg 6840

gaattagtgc gtaggcttac ggccgtcttg cttccaaaca ttcacacgct ttttgacatg 6900

tcggcggagg attttgatgc aatcatagca gaacacttca agcaaggcga cccggtactg 6960

gagacggata tcgcatcatt cgacaaaagc caagacgacg ctatggcgtt aaccggtctg 7020

atgatcttgg aggacctggg tgtggatcaa ccactactcg acttgatcga gtgcgccttt 7080

ggagaaatat catccaccca tctacctacg ggtactcgtt ttaaattcgg ggcgatgatg 7140

aaatccggaa tgttcctcac actttttgtc aacacagttt tgaatgtcgt tatcgccagc 7200

agagtactag aagagcggct taaaacgtcc agatgtgcag cgttcattgg cgacgacaac 7260

atcatacatg gagtagtatc tgacaaagaa atggctgaga ggtgcgccac ctggctcaac 7320

atggaggtta agatcatcga cgcagtcatc ggtgagagac caccttactt ctgcggcgga 7380

tttatcttgc aagattcggt tacttccaca gcgtgccgcg tggcggatcc cctgaaaagg 7440

ctgtttaagt tgggtaaacc gctcccagcc gacgacgagc aagacgaaga cagaagacgc 7500

gctctgctag atgaaacaaa ggcgtggttt agagtaggta taacaggcac tttagcagtg 7560

gccgtgacga cccggtatga ggtagacaat attacacctg tcctactggc attgagaact 7620

tttgcccaga gcaaaagagc attccaagcc atcagagggg aaataaagca tctctacggt 7680

ggtcctaaat agtcagcata gtacatttca tctgactaat actacaacac caccacctct 7740

agagccacca tggagacaga cacactcctg ctatgggtac tgctgctctg ggttccaggt 7800

tccactggtg actatgaggc ccaggcggcc ggtaccgcta gcggccaggc cggccgctat 7860

ccttacgacg tgccagatta tgcctctaat gctgtgggcc aggacacgca ggaggtcatc 7920

gtggtgccac actccttgcc ctttaaggtg gtggtgatct cagccatcct ggccctggtg 7980

gtgctcacca tcatctccct tatcatcctc atcatgcttt ggcagaagaa gccacgttag 8040

gggcccgcca tcgattagtc caatttgttg gcccaatgat ccgaccagca aaactcgatg 8100

tacttccgag gaactgatgt gcataatgca tcaggctggt acattagatc cccgcttacc 8160

gcgggcaata tagcaacact aaaaactcga tgtacttccg aggaagcgca gtgcataatg 8220

ctgcgcagtg ttgccacata accactatat taaccattta tctagcggac gccaaaaact 8280

caatgtattt ctgaggaagc gtggtgcata atgccacgca gcgtctgcat aacttttatt 8340

atttctttta ttaatcaaca aaattttgtt tttaacattt caaaaaaaaa aaaaaaaaaa 8400

aaaaaaaaaa aaaaaaaagg gaattcctcg attaattaag cggccgctcg aggggaatta 8460

attcttgaag acgaaagggc caggtggcac ttttcgggga aatgtgcgcg gaacccctat 8520

ttgtttattt ttctaaatac attcaaatat gtatccgctc atgagacaat aaccctgata 8580

aatgcttcaa taatattgaa aaaggaagag tatgagtatt caacatttcc gtgtcgccct 8640

tattcccttt tttgcggcat tttgccttcc tgtttttgct cacccagaaa cgctggtgaa 8700

agtaaaagat gctgaagatc agttgggtgc acgagtgggt tacatcgaac tggatctcaa 8760

cagcggtaag atccttgaga gttttcgccc cgaagaacgt tttccaatga tgagcacttt 8820

taaagttctg ctatgtggcg cggtattatc ccgtgttgac gccgggcaag agcaactcgg 8880

tcgccgcata cactattctc agaatgactt ggttgagtac tcaccagtca cagaaaagca 8940

tcttacggat ggcatgacag taagagaatt atgcagtgct gccataacca tgagtgataa 9000

cactgcggcc aacttacttc tgacaacgat cggaggaccg aaggagctaa ccgctttttt 9060

gcacaacatg ggggatcatg taactcgcct tgatcgttgg gaaccggagc tgaatgaagc 9120

cataccaaac gacgagcgtg acaccacgat gcctgtagca atggcaacaa cgttgcgcaa 9180

actattaact ggcgaactac ttactctagc ttcccggcaa caattaatag actggatgga 9240

ggcggataaa gttgcaggac cacttctgcg ctcggccctt ccggctggct ggtttattgc 9300

tgataaatct ggagccggtg agcgtgggtc tcgcggtatc attgcagcac tggggccaga 9360

tggtaagccc tcccgtatcg tagttatcta cacgacgggg agtcaggcaa ctatggatga 9420

acgaaataga cagatcgctg agataggtgc ctcactgatt aagcattggt aactgtcaga 9480

ccaagtttac tcatatatac tttagattga tttaaaactt catttttaat ttaaaaggat 9540

ctaggtgaag atcctttttg ataatctcat gaccaaaatc ccttaacgtg agttttcgtt 9600

ccactgagcg tcagaccccg tagaaaagat caaaggatct tcttgagatc ctttttttct 9660

gcgcgtaatc tgctgcttgc aaacaaaaaa accaccgcta ccagcggtgg tttgtttgcc 9720

ggatcaagag ctaccaactc tttttccgaa ggtaactggc ttcagcagag cgcagatacc 9780

aaatactgtc cttctagtgt agccgtagtt aggccaccac ttcaagaact ctgtagcacc 9840

gcctacatac ctcgctctgc taatcctgtt accagtggct gctgccagtg gcgataagtc 9900

gtgtcttacc gggttggact caagacgata gttaccggat aaggcgcagc ggtcgggctg 9960

aacggggggt tcgtgcacac agcccagctt ggagcgaacg acctacaccg aactgagata 10020

cctacagcgt gagcattgag aaagcgccac gcttcccgaa gggagaaagg cggacaggta 10080

tccggtaagc ggcagggtcg gaacaggaga gcgcacgagg gagcttccag ggggaaacgc 10140

ctggtatctt tatagtcctg tcgggtttcg ccacctctga cttgagcgtc gatttttgtg 10200

atgctcgtca ggggggcgga gcctatggaa aaacgccagc aacgc 10245

<210> 40
<211> 315
<212> DNA
<213> artificial sequence

<220>
<223> chemically synthesized

<400> 40
gagtctagag ccaccatgga gacagacaca ctcctgctat gggtactgct gctctgggtt 60

ccaggttcca ctggtgacta tgaggcccag gcggccggta ccgctagcgg ccaggccggc 120

cgctatcctt acgacgtgcc agattatgcc tctaatgctg tgggccagga cacgcaggag 180

gtcatcgtgg tgccacactc cttgcccttt aaggtggtgg tgatctcagc catcctggcc 240

ctggtggtgc tcaccatcat ctcccttatc atcctcatca tgctttggca gaagaagcca 300

cgttaggggc ccgag 315

<210> 41
<211> 100
<212> DNA
<213> artificial sequence

<220>
<223> chemically synthesized

<400> 41

cctcctgcgt gtcctggccc acagcattag aggcataatc tggcacgtcg taaggatagc     60

ggccggcctg gccgctagcg gtaccggccg cctgggcctc                          100


<210>  42
<211>  77
<212>  DNA
<213>  artificial sequence

<220>
<223>  chemically synthesized

<400>  42
ggtggttcct ctagatcttc ctcctctggt ggcggtggct cgggcggtgg tgggcaggtg     60

cagctggtgc agtctgg                                                    77


<210>  43
<211>  77
<212>  DNA
<213>  artificial sequence

<220>
<223>  chemically synthesized

<400>  43
ggtggttcct ctagatcttc ctcctctggt ggcggtggct cgggcggtgg tgggcagatc     60

accttgaagg agtctgg                                                    77


<210>  44
<211>  76
<212>  DNA
<213>  artificial sequence

<220>
<223>  chemically synthesized

<400>  44
ggtggttcct ctagatcttc ctcctctggt ggcggtggct cgggcggtgg tggggaggtg     60

cagctgktgg agtctg                                                     76


<210>  45
<211>  77
<212>  DNA
<213>  artificial sequence

<220>
<223>  chemically synthesized

<400>  45
ggtggttcct ctagatcttc ctcctctggt ggcggtggct cgggcggtgg tgggcaggtg     60

cagctacagc agtgggg                                                    77

<210>  46
<211>  77
<212>  DNA
<213>  artificial sequence

<220>
<223>  chemically synthesized

<400>  46
ggtggttcct ctagatcttc ctcctctggt ggcggtggct cgggcggtgg tgggcaggtg     60

cagctgcagg agtcggg                                                    77


<210>  47
<211>  77
<212>  DNA
<213>  artificial sequence

<220>
<223>  chemically synthesized

<400>  47
ggtggttcct ctagatcttc ctcctctggt ggcggtggct cgggcggtgg tggggaggtg     60

cagctggtgs agtctgg                                                    77


<210>  48
<211>  46
<212>  DNA
<213>  artificial sequence

<220>
<223>  chemically synthesized

<400>  48
cctggccggc ctggccacta gtgaccgatg ggcccttggt ggargc                    46


<210>  49
<211>  37
<212>  DNA
<213>  artificial sequence

<220>
<223>  chemically synthesized

<400>  49
gggcccaggc ggccgagctc cagatgaccc agtctcc                              37


<210>  50
<211>  37
<212>  DNA
<213>  artificial sequence

<220>
<223>  chemically synthesized

<400>  50

gggcccaggc ggccgagctc gtgatgacyc agtctcc                                37

<210>  51
<211>  37
<212>  DNA
<213>  artificial sequence

<220>
<223>  chemically synthesized

<400>  51
gggcccaggc ggccgagctc gtgwtgacrc agtctcc                                37

<210>  52
<211>  37
<212>  DNA
<213>  artificial sequence

<220>
<223>  chemically synthesized

<400>  52
gggcccaggc ggccgagctc acactcacgc agtctcc                                37

<210>  53
<211>  42
<212>  DNA
<213>  artificial sequence

<220>
<223>  chemically synthesized

<400>  53
ggaagatcta gaggaaccac ctttgatytc caccttggtc cc                          42

<210>  54
<211>  42
<212>  DNA
<213>  artificial sequence

<220>
<223>  chemically synthesized

<400>  54
ggaagatcta gaggaaccac ctttgatctc cagcttggtc cc                          42

<210>  55
<211>  42
<212>  DNA
<213>  artificial sequence

<220>
<223>  chemically synthesized

<400>  55
ggaagatcta gaggaaccac ctttgatatc cactttggtc cc                          42

<210>  56
<211>  42
<212>  DNA
<213>  artificial sequence

<220>
<223>  chemically synthesized

<400>  56
ggaagatcta gaggaaccac ctttaatctc cagtcgtgtc cc                          42


<210>  57
<211>  40
<212>  DNA
<213>  artificial sequence

<220>
<223>  chemically synthesized

<400>  57
gggcccaggc ggccgagctc gtgbtgacgc agccgccctc                             40


<210>  58
<211>  40
<212>  DNA
<213>  artificial sequence

<220>
<223>  chemically synthesized

<400>  58
gggcccaggc ggccgagctc gtgctgactc agccaccctc                             40


<210>  59
<211>  43
<212>  DNA
<213>  artificial sequence

<220>
<223>  chemically synthesized

<400>  59
gggcccaggc ggccgagctc gccctgactc agcctccctc cgt                         43


<210>  60
<211>  46
<212>  DNA
<213>  artificial sequence

<220>
<223>  chemically synthesized

<400>  60
gggcccaggc ggccgagctc gagctgactc agccaccctc agtgtc                      46

<210>  61
<211>  40
<212>  DNA
<213>  artificial sequence

<220>
<223>  chemically synthesized

<400>  61
gggcccaggc ggccgagctc gtgctgactc aatcgccctc                              40


<210>  62
<211>  40
<212>  DNA
<213>  artificial sequence

<220>
<223>  chemically synthesized

<400>  62
gggcccaggc ggccgagctc atgctgactc agccccactc                              40


<210>  63
<211>  40
<212>  DNA
<213>  artificial sequence

<220>
<223>  chemically synthesized

<400>  63
gggcccaggc ggccgagctc gtggtgacyc aggagccmtc                              40


<210>  64
<211>  40
<212>  DNA
<213>  artificial sequence

<220>
<223>  chemically synthesized

<400>  64
gggcccaggc ggccgagctc gtgctgactc agccaccttc                              40


<210>  65
<211>  40
<212>  DNA
<213>  artificial sequence

<220>
<223>  chemically synthesized

<400>  65
gggcccaggc ggccgagctc gggcagactc agcagctctc                              40

<210>  66
<211>  45
<212>  DNA
<213>  artificial sequence

<220>
<223>  chemically synthesized

<400>  66
ggaagatcta gaggaaccac cgcctaggac ggtcascttg gtscc                45


<210>  67
<211>  45
<212>  DNA
<213>  artificial sequence

<220>
<223>  chemically synthesized

<400>  67
ggaagatcta gaggaaccac cgcctaaaat gatcagctgg gttcc                45


<210>  68
<211>  45
<212>  DNA
<213>  artificial sequence

<220>
<223>  chemically synthesized

<400>  68
ggaagatcta gaggaaccac cgccgaggac ggtcagctsg gtscc                45


<210>  69
<211>  41
<212>  DNA
<213>  artificial sequence

<220>
<223>  chemically synthesized

<400>  69
gaggaggagg aggaggaggc ggggcccagg cggccgagct c                    41


<210>  70
<211>  41
<212>  DNA
<213>  artificial sequence

<220>
<223>  chemically synthesized

<400>  70
gaggaggagg aggaggagcc tggccggcct ggccactagt g                    41


<210>  71

<211>  4625
<212>  DNA
<213>  artificial sequence

<220>
<223>  chemically synthesized

<400>  71
atgcattagt tattaatagt aatcaattac ggggtcatta gttcatagcc catatatgga      60

gttccgcgtt acataactta cggtaaatgg cccgcctggc tgaccgccca acgacccccg     120

cccattgacg tcaataatga cgtatgttcc catagtaacg ccaataggga ctttccattg     180

acgtcaatgg gtggagtatt tacggtaaac tgcccacttg gcagtacatc aagtgtatca     240

tatgccaagt acgcccccta ttgacgtcaa tgacggtaaa tggcccgcct ggcattatgc     300

ccagtacatg accttatggg actttcctac ttggcagtac atctacgtat tagtcatcgc     360

tattaccatg gtgatgcggt tttggcagta catcaatggg cgtggatagc ggtttgactc     420

acggggattt ccaagtctcc accccattga cgtcaatggg agtttgtttt ggcaccaaaa     480

tcaacgggac tttccaaaat gtcgtaacaa ctccgcccca ttgacgcaaa tgggcggtag     540

gcgtgtacgg tgggaggtct atataagcag agctggttta gtgaaccgtc agatccgcta     600

gcgattacgc caagctcgaa attaaccctc actaaaggga acaaaagctg gagctggcta     660

gcgccaccat ggacatgagg gtccccgctc agctcctggg gctcctgcta ctctggctcc     720

gaggtgccag atgtgacatc gagctcctgc aggaattcga tatcaaacga actgtggctg     780

caccatctgt cttcatcttc ccgccatctg atgagcagtt gaaatctgga actgcctctg     840

ttgtgtgcct gctgaataac ttctatccca gagaggccaa agtacagtgg aaggtggata     900

acgccctcca atcgggtaac tcccaggaga gtgtcacaga gcaggacagc aaggacagca     960

cctacagcct cagcagcacc ctgacgctga gcaaagcaga ctacgagaaa cacaaagtct    1020

acgcctgcga agtcacccat cagggcctga gttcgcccgt cacaaagagc ttcaacaggg    1080

gagagtgtta ggtttaaacg gtaccaggta agtgtaccca attcgcccta tagtgagtcg    1140

tattacaatt cactcgatcg cccttcccaa cagttgcgca gcctgaatgg cgaatggaga    1200

tccaattttt aagtgtataa tgtgttaaac tactgattct aattgtttgt gtattttaga    1260

ttcacagtcc caaggctcat ttcaggcccc tcagtcctca cagtctgttc atgatcataa    1320

tcagccatac cacatttgta gaggttttac ttgctttaaa aaacctccca cacctccccc    1380

tgaacctgaa acataaaatg aatgcaattg ttgttgttaa cttgtttatt gcagcttata    1440

atggttacaa ataaagcaat agcatcacaa atttcacaaa taaagcattt ttttcactgc    1500

attctagttg tggtttgtcc aaactcatca atgtatctta acgcgtaaat tgtaagcgtt    1560

aatattttgt taaaattcgc gttaaatttt tgttaaatca gctcattttt taaccaatag    1620

gccgaaatcg gcaaaatccc ttataaatca aaagaataga ccgagatagg gttgagtgtt 1680

gttccagttt ggaacaagag tccactatta aagaacgtgg actccaacgt caaagggcga 1740

aaaaccgtct atcagggcga tggcccacta cgtgaaccat caccctaatc aagttttttg 1800

gggtcgaggt gccgtaaagc actaaatcgg aaccctaaag ggagcccccg atttagagct 1860

tgacggggaa agccggcgaa cgtggcgaga aaggaaggga agaaagcgaa aggagcgggc 1920

gctagggcgc tggcaagtgt agcggtcacg ctgcgcgtaa ccaccacacc cgccgcgctt 1980

aatgcgccgc tacagggcgc gtcaggtggc acttttcggg gaaatgtgcg cggaacccct 2040

atttgtttat ttttctaaat acattcaaat atgtatccgc tcatgagaca ataaccctga 2100

taaatgcttc aataatattg aaaaaggaag aatcctgagg cggaaagaac cagctgtgga 2160

atgtgtgtca gttagggtgt ggaaagtccc caggctcccc agcaggcaga agtatgcaaa 2220

gcatgcatct caattagtca gcaaccaggt gtggaaagtc cccaggctcc ccagcaggca 2280

gaagtatgca aagcatgcat ctcaattagt cagcaaccat agtcccgccc ctaactccgc 2340

ccatcccgcc cctaactccg cccagttccg cccattctcc gccccatggc tgactaattt 2400

tttttattta tgcagaggcc gaggccgcct cggcctctga gctattccag aagtagtgag 2460

gaggcttttt tggaggccta ggcttttgca aagatcgatc aagagacagg atgaggatcg 2520

tttcgcatga ttgaacaaga tggattgcac gcaggttctc cggccgcttg ggtggagagg 2580

ctattcggct atgactgggc acaacagaca atcggctgct ctgatgccgc cgtgttccgg 2640

ctgtcagcgc aggggcgccc ggttcttttt gtcaagaccg acctgtccgg tgccctgaat 2700

gaactgcaag acgaggcagc gcggctatcg tggctggcca cgacgggcgt tccttgcgca 2760

gctgtgctcg acgttgtcac tgaagcggga agggactggc tgctattggg cgaagtgccg 2820

gggcaggatc tcctgtcatc tcaccttgct cctgccgaga aagtatccat catggctgat 2880

gcaatgcggc ggctgcatac gcttgatccg gctacctgcc cattcgacca ccaagcgaaa 2940

catcgcatcg agcgagcacg tactcggatg gaagccggtc ttgtcgatca ggatgatctg 3000

gacgaagaac atcaggggct cgcgccagcc gaactgttcg ccaggctcaa ggcgagcatg 3060

cccgacggcg aggatctcgt cgtgacccat ggcgatgcct gcttgccgaa tatcatggtg 3120

gaaaatggcc gcttttctgg attcatcgac tgtggccggc tgggtgtggc ggaccgctat 3180

caggacatag cgttggctac ccgtgatatt gctgaagaac ttggcggcga atgggctgac 3240

cgcttcctcg tgctttacgg tatcgccgct cccgattcgc agcgcatcgc cttctatcgc 3300

cttcttgacg agttcttctg agcgggactc tggggttcga aatgaccgac caagcgacgc 3360

ccaacctgcc atcacgagat ttcgattcca ccgccgcctt ctatgaaagg ttgggcttcg 3420

gaatcgtttt ccgggacgcc ggctggatga tcctccagcg cggggatctc atgctggagt 3480

tcttcgccca ccctaggggg aggctaactg aaacacggaa ggagacaata ccggaaggaa 3540

cccgcgctat gacggcaata aaaagacaga ataaaacgca cggtgttggg tcgtttgttc 3600

ataaacgcgg ggttcggtcc cagggctggc actctgtcga taccccaccg agaccccatt 3660

ggggccaata cgcccgcgtt tcttcctttt ccccacccca ccccccaagt tcgggtgaag 3720

gcccagggct cgcagccaac gtcggggcgg caggccctgc catagcctca ggttactcat 3780

atatacttta gattgattta aaacttcatt tttaatttaa aaggatctag gtgaagatcc 3840

tttttgataa tctcatgacc aaaatccctt aacgtgagtt ttcgttccac tgagcgtcag 3900

accccgtaga aaagatcaaa ggatcttctt gagatccttt ttttctgcgc gtaatctgct 3960

gcttgcaaac aaaaaaacca ccgctaccag cggtggtttg tttgccggat caagagctac 4020

caactctttt tccgaaggta actggcttca gcagagcgca gataccaaat actgtccttc 4080

tagtgtagcc gtagttaggc caccacttca agaactctgt agcaccgcct acatacctcg 4140

ctctgctaat cctgttacca gtggctgctg ccagtggcga taagtcgtgt cttaccgggt 4200

tggactcaag acgatagtta ccggataagg cgcagcggtc gggctgaacg gggggttcgt 4260

gcacacagcc cagcttggag cgaacgacct acaccgaact gagataccta cagcgtgagc 4320

tatgagaaag cgccacgctt cccgaaggga gaaaggcgga caggtatccg gtaagcggca 4380

gggtcggaac aggagagcgc acgagggagc ttccaggggg aaacgcctgg tatctttata 4440

gtcctgtcgg gtttcgccac ctctgacttg agcgtcgatt tttgtgatgc tcgtcagggg 4500

ggcggagcct atggaaaaac gccagcaacg cggccttttt acggttcctg gccttttgct 4560

ggccttttgc tcacatgttc tttcctgcgt tatcccctga ttctgtggat aaccgtatta 4620

ccgcc 4625

<210> 72
<211> 49
<212> DNA
<213> artificial sequence

<220>
<223> chemically synthesized

<400> 72
ggctagcgcc accatggaca tgagggtccc cgctcagctc ctggggctc 49

<210> 73
<211> 47
<212> DNA
<213> artificial sequence

<220>

<223>  chemically synthesized

<400>  73
caggagctga gcggggaccc tcatgtccat ggtggcgcta gccagct                    47


<210>  74
<211>  47
<212>  DNA
<213>  artificial sequence

<220>
<223>  chemically synthesized

<400>  74
ctgctactct ggctccgagg tgccagatgt gacatcgagc tcctgca                    47


<210>  75
<211>  49
<212>  DNA
<213>  artificial sequence

<220>
<223>  chemically synthesized

<400>  75
ggagctcgat gtcacatctg gcacctcgga gccagagtag caggagccc                  49


<210>  76
<211>  35
<212>  DNA
<213>  artificial sequence

<220>
<223>  chemically synthesized

<400>  76
gaggaggata tcaaacgaac tgtggctgca ccatc                                 35


<210>  77
<211>  68
<212>  DNA
<213>  artificial sequence

<220>
<223>  chemically synthesized

<400>  77
gaggagggta ccgtttaaac ctaacactct cccctgttga agctctttgt gacgggcgaa      60

ctcaggcc                                                               68


<210>  78
<211>  5257
<212>  DNA
<213>  artificial sequence

<220>
<223>  chemically synthesized

<400>  78
atgcattagt tattaatagt aatcaattac ggggtcatta gttcatagcc catatatgga      60

gttccgcgtt acataactta cggtaaatgg cccgcctggc tgaccgccca acgacccccg     120

cccattgacg tcaataatga cgtatgttcc catagtaacg ccaataggga ctttccattg     180

acgtcaatgg gtggagtatt tacggtaaac tgcccacttg gcagtacatc aagtgtatca     240

tatgccaagt acgcccccta ttgacgtcaa tgacggtaaa tggcccgcct ggcattatgc     300

ccagtacatg accttatggg actttcctac ttggcagtac atctacgtat tagtcatcgc     360

tattaccatg gtgatgcggt tttggcagta catcaatggg cgtggatagc ggtttgactc     420

acggggattt ccaagtctcc accccattga cgtcaatggg agtttgtttt ggcaccaaaa     480

tcaacgggac tttccaaaat gtcgtaacaa ctccgcccca ttgacgcaaa tgggcggtag     540

gcgtgtacgg tgggaggtct atataagcag agctggttta gtgaaccgtc agatccgcta     600

gcgattacgc caagctcgaa attaaccctc actaaaggga acaaaagctg gagctcggcg     660

cgccaccatg gactggacct ggaggatcct cttcttggtg gcagcagcca caggagccca     720

ctcccagatg caactgctcg aggcctccac caagggccca tcggtcttcc ccctggcgcc     780

ctgctccagg agcacctccg agagcacagc ggccctgggc tgcctggtca aggactactt     840

ccccgaaccg gtgacggtgt cgtggaactc aggcgctctg accagcggcg tgcacacctt     900

cccagctgtc ctacagtcct caggactcta ctccctcagc agcgtggtga ccgtgccctc     960

cagcaacttc ggcacccaga cctacacctg caacgtagat cacaagccca gcaacaccaa    1020

ggtggacaag acagttgagc gcaaatgttg tgtcgagtgc ccaccgtgcc cagcaccacc    1080

tgtggcagga ccgtcagtct tcctcttccc cccaaaaccc aaggacaccc tcatgatctc    1140

ccggacccct gaggtcacgt gcgtggtggt ggacgtgagc cacgaagacc ccgaggtcca    1200

gttcaactgg tacgtggacg gcgtggaggt gcataatgcc aagacaaagc cacgggagga    1260

gcagttcaac agcacgttcc gtgtggtcag cgtcctcacc gttgtgcacc aggactggct    1320

gaacggcaag gagtacaagt gcaaggtctc caacaaaggc ctcccagccc ccatcgagaa    1380

aaccatctcc aaaaccaaag ggcagccccg agaaccacag gtgtacaccc tgcccccatc    1440

ccgggaggag atgaccaaga accaggtcag cctgacctgc ctggtcaaag gcttctaccc    1500

cagcgacatc gccgtggagt gggagagcaa tgggcagccg gagaacaact acaagaccac    1560

acctcccatg ctggactccg acggctcctt cttcctctac agcaagctca ccgtggacaa    1620

gagcaggtgg cagcagggga acgtcttctc atgctccgtg atgcatgagg ctctgcacaa    1680

ccactacacg cagaagagcc tgtccctgtc tccgggtaaa tgattaatta aggtaccagg    1740

taagtgtacc caattcgccc tatagtgagt cgtattacaa ttcactcgat cgcccttccc 1800

aacagttgcg cagcctgaat ggcgaatgga gatccaattt ttaagtgtat aatgtgttaa 1860

actactgatt ctaattgttt gtgtatttta gattcacagt cccaaggctc atttcaggcc 1920

cctcagtcct cacagtctgt tcatgatcat aatcagccat accacatttg tagaggtttt 1980

acttgcttta aaaaacctcc cacacctccc cctgaacctg aaacataaaa tgaatgcaat 2040

tgttgttgtt aacttgttta ttgcagctta taatggttac aaataaagca atagcatcac 2100

aaatttcaca aataaagcat ttttttcact gcattctagt tgtggtttgt ccaaactcat 2160

caatgtatct taacgcgtaa attgtaagcg ttaatatttt gttaaaattc gcgttaaatt 2220

tttgttaaat cagctcattt tttaaccaat aggccgaaat cggcaaaatc ccttataaat 2280

caaaagaata gaccgagata gggttgagtg ttgttccagt ttggaacaag agtccactat 2340

taaagaacgt ggactccaac gtcaaagggc gaaaaaccgt ctatcagggc gatggcccac 2400

tacgtgaacc atcaccctaa tcaagttttt tggggtcgag gtgccgtaaa gcactaaatc 2460

ggaaccctaa agggagcccc cgatttagag cttgacgggg aaagccggcg aacgtggcga 2520

gaaaggaagg gaagaaagcg aaaggagcgg gcgctagggc gctggcaagt gtagcggtca 2580

cgctgcgcgt aaccaccaca cccgccgcgc ttaatgcgcc gctacagggc gcgtcaggtg 2640

gcacttttcg gggaaatgtg cgcggaaccc ctatttgttt atttttctaa atacattcaa 2700

atatgtatcc gctcatgaga caataaccct gataaatgct tcaataatat tgaaaaagga 2760

agaatcctga ggcggaaaga accagctgtg gaatgtgtgt cagttagggt gtggaaagtc 2820

cccaggctcc ccagcaggca gaagtatgca aagcatgcat ctcaattagt cagcaaccag 2880

gtgtggaaag tccccaggct ccccagcagg cagaagtatg caaagcatgc atctcaatta 2940

gtcagcaacc atagtcccgc ccctaactcc gcccatcccg cccctaactc cgcccagttc 3000

cgcccattct ccgccccatg gctgactaat tttttttatt tatgcagagg ccgaggccgc 3060

ctcggcctct gagctattcc agaagtagtg aggaggcttt tttggaggcc taggcttttg 3120

caaagatcga tcaagagaca ggatgaggat cgtttcgcat gattgaacaa gatggattgc 3180

acgcaggttc tccggccgct tgggtggaga ggctattcgg ctatgactgg gcacaacaga 3240

caatcggctg ctctgatgcc gccgtgttcc ggctgtcagc gcaggggcgc ccggttcttt 3300

ttgtcaagac cgacctgtcc ggtgccctga atgaactgca agacgaggca gcgcggctat 3360

cgtggctggc cacgacgggc gttccttgcg cagctgtgct cgacgttgtc actgaagcgg 3420

gaagggactg gctgctattg ggcgaagtgc cggggcagga tctcctgtca tctcaccttg 3480

ctcctgccga gaaagtatcc atcatggctg atgcaatgcg gcggctgcat acgcttgatc 3540

cggctacctg cccattcgac caccaagcga aacatcgcat cgagcgagca cgtactcgga 3600

tggaagccgg tcttgtcgat caggatgatc tggacgaaga acatcagggg ctcgcgccag 3660

ccgaactgtt cgccaggctc aaggcgagca tgcccgacgg cgaggatctc gtcgtgaccc 3720

atggcgatgc ctgcttgccg aatatcatgg tggaaaatgg ccgcttttct ggattcatcg 3780

actgtggccg gctgggtgtg gcggaccgct atcaggacat agcgttggct acccgtgata 3840

ttgctgaaga acttggcggc gaatgggctg accgcttcct cgtgctttac ggtatcgccg 3900

ctcccgattc gcagcgcatc gccttctatc gccttcttga cgagttcttc tgagcgggac 3960

tctggggttc gaaatgaccg accaagcgac gcccaacctg ccatcacgag atttcgattc 4020

caccgccgcc ttctatgaaa ggttgggctt cggaatcgtt ttccgggacg ccggctggat 4080

gatcctccag cgcggggatc tcatgctgga gttcttcgcc caccctaggg ggaggctaac 4140

tgaaacacgg aaggagacaa taccggaagg aacccgcgct atgacggcaa taaaaagaca 4200

gaataaaacg cacggtgttg ggtcgtttgt tcataaacgc ggggttcggt cccagggctg 4260

gcactctgtc gataccccac cgagacccca ttggggccaa tacgcccgcg tttcttcctt 4320

ttccccaccc caccccccaa gttcgggtga aggcccaggg ctcgcagcca acgtcggggc 4380

ggcaggccct gccatagcct caggttactc atatatactt tagattgatt taaaacttca 4440

tttttaattt aaaaggatct aggtgaagat cctttttgat aatctcatga ccaaaatccc 4500

ttaacgtgag ttttcgttcc actgagcgtc agaccccgta gaaaagatca aaggatcttc 4560

ttgagatcct ttttttctgc gcgtaatctg ctgcttgcaa acaaaaaaac caccgctacc 4620

agcggtggtt tgtttgccgg atcaagagct accaactctt tttccgaagg taactggctt 4680

cagcagagcg cagataccaa atactgtcct tctagtgtag ccgtagttag gccaccactt 4740

caagaactct gtagcaccgc ctacatacct cgctctgcta atcctgttac cagtggctgc 4800

tgccagtggc gataagtcgt gtcttaccgg gttggactca agacgatagt taccggataa 4860

ggcgcagcgg tcgggctgaa cggggggttc gtgcacacag cccagcttgg agcgaacgac 4920

ctacaccgaa ctgagatacc tacagcgtga gctatgagaa agcgccacgc ttcccgaagg 4980

gagaaaggcg gacaggtatc cggtaagcgg cagggtcgga acaggagagc gcacgaggga 5040

gcttccaggg ggaaacgcct ggtatcttta tagtcctgtc gggtttcgcc acctctgact 5100

tgagcgtcga tttttgtgat gctcgtcagg ggggcggagc ctatggaaaa acgccagcaa 5160

cgcggccttt ttacggttcc tggccttttg ctggcctttt gctcacatgt tctttcctgc 5220

gttatcccct gattctgtgg ataaccgtat taccgcc 5257

<210> 79
<211> 38

<212>  DNA
<213>  artificial sequence

<220>
<223>  chemically synthesized

<400>  79
cggcgcgcca ccatggactg gacctggagg atcctctt                              38


<210>  80
<211>  48
<212>  DNA
<213>  artificial sequence

<220>
<223>  chemically synthesized

<400>  80
accaagaaga ggatcctcca ggtccagtcc atggtggcgc gccgagct                   48


<210>  81
<211>  44
<212>  DNA
<213>  artificial sequence

<220>
<223>  chemically synthesized

<400>  81
cttggtggca gcagccacag gagcccactc ccagatgcaa ctgc                       44


<210>  82
<211>  42
<212>  DNA
<213>  artificial sequence

<220>
<223>  chemically synthesized

<400>  82
tcgagcagtt gcatctggga gtgggctcct gtggctgctg cc                         42


<210>  83
<211>  69
<212>  DNA
<213>  artificial sequence

<220>
<223>  chemically synthesized

<400>  83
gaggagctcg aggcctccac caagggccca tcggtcttcc ccctggcgcc ctgctccagg      60

agcacctcc                                                              69


<210>  84

<211>  42
<212>  DNA
<213>  artificial sequence

<220>
<223>  chemically synthesized

<400>  84
gaggagggta ccttaattaa tcatttaccc ggagacaggg ag                          42


<210>  85
<211>  4582
<212>  DNA
<213>  artificial sequence

<220>
<223>  chemically synthesized

<400>  85
   atgcattagt tattaatagt aatcaattac ggggtcatta gttcatagcc catatatgga      60
   gttccgcgtt acataactta cggtaaatgg cccgcctggc tgaccgccca acgacccccg     120
   cccattgacg tcaataatga cgtatgttcc catagtaacg ccaataggga ctttccattg     180
   acgtcaatgg gtggagtatt tacggtaaac tgcccacttg gcagtacatc aagtgtatca     240
   tatgccaagt acgcccccta ttgacgtcaa tgacggtaaa tggcccgcct ggcattatgc     300
   ccagtacatg accttatggg actttcctac ttggcagtac atctacgtat tagtcatcgc     360
   tattaccatg gtgatgcggt tttggcagta catcaatggg cgtggatagc ggtttgactc     420
   acggggattt ccaagtctcc accccattga cgtcaatggg agtttgtttt ggcaccaaaa     480
   tcaacgggac tttccaaaat gtcgtaacaa ctccgcccca ttgacgcaaa tgggcggtag     540
   gcgtgtacgg tgggaggtct atataagcag agctggttta gtgaaccgtc agatccgcta     600
   gcgattacgc caagctcgaa attaaccctc actaaaggga acaaaagctg gagctcggcg     660
   cgccaccatg gactggacct ggaggatcct cttcttggtg gcagcagcca caggagccca     720
   ctcccagatg caactgctcg aggcctccac caagggccca tcggtcttcc ccctggcgcc     780
   ctgctccagg agcacctccg agagcacagc ggccctgggc tgcctggtca aggactactt     840
   ccccgaaccg gtgacggtgt cgtggaactc aggcgctctg accagcggcg tgcacacctt     900
   cccagctgtc ctacagtcct caggactcta ctccctcagc agcgtggtga ccgtgccctc     960
   cagcaacttc ggcacccaga cctacacctg caacgtagat cacaagccca gcaacaccaa    1020
   ggtggacaag acagttgagc gcaaatgatt aattaaggta ccaggtaagt gtacccaatt    1080
   cgccctatag tgagtcgtat tacaattcac tcgatcgccc ttcccaacag ttgcgcagcc    1140
   tgaatggcga atggagatcc aatttttaag tgtataatgt gttaaactac tgattctaat    1200
   tgtttgtgta ttttagattc acagtcccaa ggctcatttc aggcccctca gtcctcacag    1260
   tctgttcatg atcataatca gccataccac atttgtagag gttttacttg ctttaaaaaa 1320
   cctcccacac ctccccctga acctgaaaca taaaatgaat gcaattgttg ttgttaactt 1380
   gtttattgca gcttataatg gttacaaata aagcaatagc atcacaaatt tcacaaataa 1440
   agcatttttt tcactgcatt ctagttgtgg tttgtccaaa ctcatcaatg tatcttaacg 1500
   cgtaaattgt aagcgttaat attttgttaa aattcgcgtt aaatttttgt taaatcagct 1560
   cattttttaa ccaataggcc gaaatcggca aaatccctta taaatcaaaa gaatagaccg 1620
   agatagggtt gagtgttgtt ccagtttgga acaagagtcc actattaaag aacgtggact 1680
   ccaacgtcaa agggcgaaaa accgtctatc agggcgatgg cccactacgt gaaccatcac 1740
   cctaatcaag ttttttgggg tcgaggtgcc gtaaagcact aaatcggaac cctaaaggga 1800
   gcccccgatt tagagcttga cggggaaagc cggcgaacgt ggcgagaaag gaagggaaga 1860
   aagcgaaagg agcgggcgct agggcgctgg caagtgtagc ggtcacgctg cgcgtaacca 1920
   ccacacccgc cgcgcttaat gcgccgctac agggcgcgtc aggtggcact tttcggggaa 1980
   atgtgcgcgg aacccctatt tgtttatttt tctaaataca ttcaaatatg tatccgctca 2040
   tgagacaata accctgataa atgcttcaat aatattgaaa aaggaagaat cctgaggcgg 2100
   aaagaaccag ctgtggaatg tgtgtcagtt agggtgtgga aagtccccag gctccccagc 2160
   aggcagaagt atgcaaagca tgcatctcaa ttagtcagca accaggtgtg gaaagtcccc 2220
   aggctcccca gcaggcagaa gtatgcaaag catgcatctc aattagtcag caaccatagt 2280
   cccgccccta actccgccca tcccgcccct aactccgccc agttccgccc attctccgcc 2340
   ccatggctga ctaatttttt ttatttatgc agaggccgag gccgcctcgg cctctgagct 2400
   attccagaag tagtgaggag gcttttttgg aggcctaggc ttttgcaaag atcgatcaag 2460
   agacaggatg aggatcgttt cgcatgattg aacaagatgg attgcacgca ggttctccgg 2520
   ccgcttgggt ggagaggcta ttcggctatg actgggcaca acagacaatc ggctgctctg 2580
   atgccgccgt gttccggctg tcagcgcagg ggcgcccggt tctttttgtc aagaccgacc 2640
   tgtccggtgc cctgaatgaa ctgcaagacg aggcagcgcg gctatcgtgg ctggccacga 2700
   cgggcgttcc ttgcgcagct gtgctcgacg ttgtcactga agcgggaagg gactggctgc 2760
   tattgggcga agtgccgggg caggatctcc tgtcatctca ccttgctcct gccgagaaag 2820
   tatccatcat ggctgatgca atgcggcggc tgcatacgct tgatccggct acctgcccat 2880
   tcgaccacca agcgaaacat cgcatcgagc gagcacgtac tcggatggaa gccggtcttg 2940
   tcgatcagga tgatctggac gaagaacatc aggggctcgc gccagccgaa ctgttcgcca 3000
   ggctcaaggc gagcatgccc gacggcgagg atctcgtcgt gacccatggc gatgcctgct 3060
   tgccgaatat catggtggaa aatggccgct tttctggatt catcgactgt ggccggctgg 3120
   gtgtggcgga ccgctatcag gacatagcgt tggctacccg tgatattgct gaagaacttg 3180
   gcggcgaatg ggctgaccgc ttcctcgtgc tttacggtat cgccgctccc gattcgcagc 3240
   gcatcgcctt ctatcgcctt cttgacgagt tcttctgagc gggactctgg ggttcgaaat 3300
   gaccgaccaa gcgacgccca acctgccatc acgagatttc gattccaccg ccgccttcta 3360
   tgaaaggttg ggcttcggaa tcgttttccg ggacgccggc tggatgatcc tccagcgcgg 3420
   ggatctcatg ctggagttct tcgcccaccc taggggaggc taactgaaac acggaaggag 3480
   acaataccgg aaggaacccg cgctatgacg gcaataaaaa gacagaataa aacgcacggt 3540
   gttgggtcgt ttgttcataa acgcggggtt cggtcccagg gctggcactc tgtcgatacc 3600
   ccaccgagac cccattgggg ccaatacgcc cgcgtttctt ccttttcccc accccacccc 3660
   ccaagttcgg gtgaaggccc agggctcgca gccaacgtcg gggcggcagg ccctgccata 3720
   gcctcaggtt actcatatat actttagatt gatttaaaac ttcattttta atttaaaagg 3780
   atctaggtga agatcctttt tgataatctc atgaccaaaa tcccttaacg tgagttttcg 3840
   ttccactgag cgtcagaccc cgtagaaaag atcaaaggat cttcttgaga tccttttttt 3900
   ctgcgcgtaa tctgctgctt gcaaacaaaa aaaccaccgc taccagcggt ggtttgtttg 3960
   ccggatcaag agctaccaac tctttttccg aaggtaactg gcttcagcag agcgcagata 4020
   ccaaatactg tccttctagt gtagccgtag ttaggccacc acttcaagaa ctctgtagca 4080
   ccgcctacat acctcgctct gctaatcctg ttaccagtgg ctgctgccag tggcgataag 4140
   tcgtgtctta ccgggttgga ctcaagacga tagttaccgg ataaggcgca gcggtcgggc 4200
   tgaacggggg gttcgtgcac acagcccagc ttggagcgaa cgacctacac cgaactgaga 4260
   tacctacagc gtgagctatg agaaagcgcc acgcttcccg aagggagaaa ggcggacagg 4320
   tatccggtaa gcggcagggt cggaacagga gagcgcacga gggagcttcc agggggaaac 4380
   gcctggtatc tttatagtcc tgtcgggttt cgccacctct gacttgagcg tcgatttttg 4440
   tgatgctcgt caggggggcg gagcctatgg aaaaacgcca gcaacgcggc ctttttacgg 4500
   ttcctggcct tttgctggcc ttttgctcac atgttctttc ctgcgttatc ccctgattct 4560
   gtggataacc gtattaccgc cc 4582

<210> 86
<211> 33
<212> DNA
<213> artificial sequence

<220>
<223> chemically synthesized

<400>  86
gaggagctcg aggcctccac caagggccca tcg                                    33


<210>  87
<211>  44
<212>  DNA
<213>  artificial sequence

<220>
<223>  chemically synthesized

<400>  87
gaggagggta ccttaattaa tcatttgcgc tcaactgtct tgtc                        44


<210>  88
<211>  5269
<212>  DNA
<213>  artificial sequence

<220>
<223>  chemically synthesized

<400>  88
atgcattagt tattaatagt aatcaattac ggggtcatta gttcatagcc catatatgga      60

gttccgcgtt acataactta cggtaaatgg cccgcctggc tgaccgccca acgacccccg     120

cccattgacg tcaataatga cgtatgttcc catagtaacg ccaataggga ctttccattg     180

acgtcaatgg gtggagtatt tacggtaaac tgcccacttg gcagtacatc aagtgtatca     240

tatgccaagt acgcccccta ttgacgtcaa tgacggtaaa tggcccgcct ggcattatgc     300

ccagtacatg accttatggg actttcctac ttggcagtac atctacgtat tagtcatcgc     360

tattaccatg gtgatgcggt tttggcagta catcaatggg cgtggatagc ggtttgactc     420

acggggattt ccaagtctcc accccattga cgtcaatggg agtttgtttt ggcaccaaaa     480

tcaacgggac tttccaaaat gtcgtaacaa ctccgcccca ttgacgcaaa tgggcggtag     540

gcgtgtacgg tgggaggtct atataagcag agctggttta gtgaaccgtc agatccgcta     600

gcgattacgc caagctcgaa attaaccctc actaaaggga acaaaagctg gagctcggcg     660

cgccaccatg gactggacct ggaggatcct cttcttggtg gcagcagcca caggagccca     720

ctcccagatg caactgctcg aggcctccac caagggccca tcggtcttcc ccctggcacc     780

ctcctccaag agcacctctg ggggcacagc ggccctgggc tgcctggtca aggactactt     840

ccccgaaccg gtgacggtgt cgtggaactc aggcgccctg accagcggcg tgcacacctt     900

cccggctgtc ctacagtcct caggactcta ctccctcagc agcgtggtga ccgtgccctc     960

cagcagcttg ggcacccaga cctacatctg caacgtgaat cacaagccca gcaacaccaa    1020

ggtggacaag aaagttgagc ccaaatcttg tgacaaaact cacacatgcc caccgtgccc    1080

agcacctgaa ctcctggggg gaccgtcagt cttcctcttc cccccaaaac ccaaggacac 1140

cctcatgatc tcccggaccc ctgaggtcac atgcgtggtg gtggacgtga gccacgaaga 1200

ccctgaggtc aagttcaact ggtacgtgga cggcgtggag gtgcataatg ccaagacaaa 1260

gccgcgggag gagcagtaca acagcacgta ccgtgtggtc agcgtcctca ccgtcctgca 1320

ccaggactgg ctgaatggca aggagtacaa gtgcaaggtc tccaacaaag ccctcccagc 1380

ccccatcgag aaaaccatct ccaaagccaa agggcagccc cgagaaccac aggtgtacac 1440

cctgccccca tcccgggatg agctgaccaa gaaccaggtc agcctgacct gcctggtcaa 1500

aggcttctat cccagcgaca tcgccgtgga gtgggagagc aatgggcagc cggagaacaa 1560

ctacaagacc acgcctcccg tgctggactc cgacggctcc ttcttcctct acagcaagct 1620

caccgtggac aagagcaggt ggcagcaggg gaacgtcttc tcatgctccg tgatgcatga 1680

ggctctgcac aaccactaca cgcagaagag cctctccctg tctccgggta aatgattaat 1740

taaggtacca ggtaagtgta cccaattcgc cctatagtga gtcgtattac aattcactcg 1800

atcgcccttc ccaacagttg cgcagcctga atggcgaatg gagatccaat ttttaagtgt 1860

ataatgtgtt aaactactga ttctaattgt ttgtgtattt tagattcaca gtcccaaggc 1920

tcatttcagg cccctcagtc ctcacagtct gttcatgatc ataatcagcc ataccacatt 1980

tgtagaggtt ttacttgctt taaaaaacct cccacacctc cccctgaacc tgaaacataa 2040

aatgaatgca attgttgttg ttaacttgtt tattgcagct tataatggtt acaaataaag 2100

caatagcatc acaaatttca caaataaagc atttttttca ctgcattcta gttgtggttt 2160

gtccaaactc atcaatgtat cttaacgcgt aaattgtaag cgttaatatt ttgttaaaat 2220

tcgcgttaaa tttttgttaa atcagctcat tttttaacca ataggccgaa atcggcaaaa 2280

tcccttataa atcaaaagaa tagaccgaga tagggttgag tgttgttcca gtttggaaca 2340

agagtccact attaaagaac gtggactcca acgtcaaagg gcgaaaaacc gtctatcagg 2400

gcgatggccc actacgtgaa ccatcaccct aatcaagttt tttggggtcg aggtgccgta 2460

aagcactaaa tcggaaccct aaagggagcc cccgatttag agcttgacgg ggaaagccgg 2520

cgaacgtggc gagaaaggaa gggaagaaag cgaaaggagc gggcgctagg gcgctggcaa 2580

gtgtagcggt cacgctgcgc gtaaccacca cacccgccgc gcttaatgcg ccgctacagg 2640

gcgcgtcagg tggcactttt cggggaaatg tgcgcggaac ccctatttgt ttatttttct 2700

aaatacattc aaatatgtat ccgctcatga gacaataacc ctgataaatg cttcaataat 2760

attgaaaaag gaagaatcct gaggcggaaa gaaccagctg tggaatgtgt gtcagttagg 2820

gtgtggaaag tccccaggct ccccagcagg cagaagtatg caaagcatgc atctcaatta 2880

gtcagcaacc aggtgtggaa agtccccagg ctccccagca ggcagaagta tgcaaagcat 2940

gcatctcaat tagtcagcaa ccatagtccc gcccctaact ccgcccatcc cgcccctaac 3000

tccgcccagt tccgcccatt ctccgcccca tggctgacta atttttttta tttatgcaga 3060

ggccgaggcc gcctcggcct ctgagctatt ccagaagtag tgaggaggct tttttggagg 3120

cctaggcttt tgcaaagatc gatcaagaga caggatgagg atcgtttcgc atgattgaac 3180

aagatggatt gcacgcaggt tctccggccg cttgggtgga gaggctattc ggctatgact 3240

gggcacaaca gacaatcggc tgctctgatg ccgccgtgtt ccggctgtca gcgcaggggc 3300

gcccggttct ttttgtcaag accgacctgt ccggtgccct gaatgaactg caagacgagg 3360

cagcgcggct atcgtggctg gccacgacgg gcgttccttg cgcagctgtg ctcgacgttg 3420

tcactgaagc gggaagggac tggctgctat tgggcgaagt gccggggcag gatctcctgt 3480

catctcacct tgctcctgcc gagaaagtat ccatcatggc tgatgcaatg cggcggctgc 3540

atacgcttga tccggctacc tgcccattcg accaccaagc gaaacatcgc atcgagcgag 3600

cacgtactcg gatggaagcc ggtcttgtcg atcaggatga tctggacgaa gaacatcagg 3660

ggctcgcgcc agccgaactg ttcgccaggc tcaaggcgag catgcccgac ggcgaggatc 3720

tcgtcgtgac ccatggcgat gcctgcttgc cgaatatcat ggtggaaaat ggccgctttt 3780

ctggattcat cgactgtggc cggctgggtg tggcggaccg ctatcaggac atagcgttgg 3840

ctacccgtga tattgctgaa gaacttggcg gcgaatgggc tgaccgcttc ctcgtgcttt 3900

acggtatcgc cgctcccgat tcgcagcgca tcgccttcta tcgccttctt gacgagttct 3960

tctgagcggg actctggggt tcgaaatgac cgaccaagcg acgcccaacc tgccatcacg 4020

agatttcgat tccaccgccg ccttctatga aaggttgggc ttcggaatcg ttttccggga 4080

cgccggctgg atgatcctcc agcgcgggga tctcatgctg gagttcttcg cccaccctag 4140

ggggaggcta actgaaacac ggaaggagac aataccggaa ggaacccgcg ctatgacggc 4200

aataaaaaga cagaataaaa cgcacggtgt tgggtcgttt gttcataaac gcggggttcg 4260

gtcccagggc tggcactctg tcgatacccc accgagaccc cattggggcc aatacgcccg 4320

cgtttcttcc ttttccccac cccacccccc aagttcgggt gaaggcccag ggctcgcagc 4380

caacgtcggg gcggcaggcc ctgccatagc ctcaggttac tcatatatac tttagattga 4440

tttaaaactt catttttaat ttaaaaggat ctaggtgaag atcctttttg ataatctcat 4500

gaccaaaatc ccttaacgtg agttttcgtt ccactgagcg tcagaccccg tagaaaagat 4560

caaaggatct tcttgagatc ctttttttct gcgcgtaatc tgctgcttgc aaacaaaaaa 4620

accaccgcta ccagcggtgg tttgtttgcc ggatcaagag ctaccaactc tttttccgaa 4680

ggtaactggc ttcagcagag cgcagatacc aaatactgtc cttctagtgt agccgtagtt 4740

aggccaccac ttcaagaact ctgtagcacc gcctacatac ctcgctctgc taatcctgtt 4800

accagtggct gctgccagtg gcgataagtc gtgtcttacc gggttggact caagacgata 4860

gttaccggat aaggcgcagc ggtcgggctg aacggggggt tcgtgcacac agcccagctt 4920

ggagcgaacg acctacaccg aactgagata cctacagcgt gagctatgag aaagcgccac 4980

gcttcccgaa gggagaaagg cggacaggta tccggtaagc ggcagggtcg gaacaggaga 5040

gcgcacgagg gagcttccag ggggaaacgc ctggtatctt tatagtcctg tcgggtttcg 5100

ccacctctga cttgagcgtc gatttttgtg atgctcgtca ggggggcgga gcctatggaa 5160

aaacgccagc aacgcggcct ttttacggtt cctggccttt tgctggcctt ttgctcacat 5220

gttctttcct gcgttatccc ctgattctgt ggataaccgt attaccgcc 5269

<210> 89
<211> 33
<212> DNA
<213> artificial sequence

<220>
<223> chemically synthesized

<400> 89
caagggccca tcggtcttcc ccctggcacc ctc 33

<210> 90
<211> 5273
<212> DNA
<213> artificial sequence

<220>
<223> chemically synthesized

<400> 90
atgcattagt tattaatagt aatcaattac ggggtcatta gttcatagcc catatatgga 60

gttccgcgtt acataactta cggtaaatgg cccgcctggc tgaccgccca acgacccccg 120

cccattgacg tcaataatga cgtatgttcc catagtaacg ccaataggga ctttccattg 180

acgtcaatgg gtggagtatt tacggtaaac tgcccacttg gcagtacatc aagtgtatca 240

tatgccaagt acgcccccta ttgacgtcaa tgacggtaaa tggcccgcct ggcattatgc 300

ccagtacatg accttatggg actttcctac ttggcagtac atctacgtat tagtcatcgc 360

tattaccatg gtgatgcggt tttggcagta catcaatggg cgtggatagc ggtttgactc 420

acggggattt ccaagtctcc accccattga cgtcaatggg agtttgtttt ggcaccaaaa 480

tcaacgggac tttccaaaat gtcgtaacaa ctccgcccca ttgacgcaaa tgggcggtag 540

gcgtgtacgg tgggaggtct atataagcag agctggttta gtgaaccgtc agatccgcta 600

gcgattacgc caagctcgaa attaaccctc actaaaggga acaaaagctg gagctcggcg 660

cgccaccatg gactggacct ggaggatcct cttcttggtg gcagcagcca caggagccca 720
ctcccagatg caactgctcg aggcctccac caagggccca tcggtcttcc ccctggcgcc 780
ctgctccagg agcacctccg agagcacagc ggccctgggc tgcctggtca aggactactt 840
ccccgaaccg gtgacggtgt cgtggaactc aggcgccctg accagcggcg tgcacacctt 900
cccggctgtc ctacagtcct caggactcta ctccctcagc agcgtggtga ccgtgccctc 960
cagcagcttg ggcacgaaga cctacacctg caatgtagat cacaagccca gcaacaccaa 1020
ggtggacaag agagttgagt ccaaatatgg tcccccatgc ccatcatgcc cagcacctga 1080
gttcctgggg ggaccatcag tcttcctgtt ccccccaaaa cccaaggaca ctctcatgat 1140
ctcccggacc cctgaggtca cgtgcgtggt ggtggacgtg agccaggaag accccgaggt 1200
ccagttcaac tggtacgtgg atggcgtgga ggtgcataat gccaagacaa agccgcggga 1260
ggagcagttc aacagcacgt accgtgtggt cagcgtcctc accgtcgtgc accaggactg 1320
gctgaacggc aaggagtaca agtgcaaggt ctccaacaaa ggcctcccgt cctccatcga 1380
gaaaaccatc tccaaagcca aagggcagcc ccgagagcca caggtgtaca ccctgccccc 1440
atcccaggag gagatgacca agaaccaggt cagcctgacc tgcctggtca aaggcttcta 1500
ccccagcgac atcgccgtgg agtgggagag caatgggcag ccggagaaca actacaagac 1560
cacgcctccc gtgctggact ccgacggctc cttcttcctc tacagcaggc taaccgtgga 1620
caagagcagg tggcaggagg ggaatgtctt ctcatgctcc gtgatgcatg aggctctgca 1680
caaccactac acgcagaaga gcctctccct gtctctgggt aaatgagtgc cagggccggt 1740
taattaaggt accaggtaag tgtacccaat tcgccctata gtgagtcgta ttacaattca 1800
ctcgatcgcc cttcccaaca gttgcgcagc ctgaatggcg aatggagatc caatttttaa 1860
gtgtataatg tgttaaacta ctgattctaa ttgtttgtgt attttagatt cacagtccca 1920
aggctcattt caggcccctc agtcctcaca gtctgttcat gatcataatc agccatacca 1980
catttgtaga ggttttactt gctttaaaaa acctcccaca cctccccctg aacctgaaac 2040
ataaaatgaa tgcaattgtt gttgttaact tgtttattgc agcttataat ggttacaaat 2100
aaagcaatag catcacaaat ttcacaaata aagcattttt ttcactgcat tctagttgtg 2160
gtttgtccaa actcatcaat gtatcttaac gcgtaaattg taagcgttaa tattttgtta 2220
aaattcgcgt taaatttttg ttaaatcagc tcatttttta accaataggc cgaaatcggc 2280
aaaatccctt ataaatcaaa agaatagacc gagatagggt tgagtgttgt tccagtttgg 2340
aacaagagtc cactattaaa gaacgtggac tccaacgtca aagggcgaaa aaccgtctat 2400
cagggcgatg gcccactacg tgaaccatca ccctaatcaa gtttttgggg gtcgaggtgc 2460

cgtaaagcac taaatcggaa ccctaaaggg agcccccgat ttagagcttg acggggaaag 2520

ccggcgaacg tggcgagaaa ggaagggaag aaagcgaaag gagcgggcgc tagggcgctg 2580

gcaagtgtag cggtcacgct gcgcgtaacc accacacccg ccgcgcttaa tgcgccgcta 2640

cagggcgcgt caggtggcac ttttcgggga aatgtgcgcg gaacccctat ttgtttattt 2700

ttctaaatac attcaaatat gtatccgctc atgagacaat aaccctgata aatgcttcaa 2760

taatattgaa aaaggaagaa tcctgaggcg gaaagaacca gctgtggaat gtgtgtcagt 2820

tagggtgtgg aaagtcccca ggctccccag caggcagaag tatgcaaagc atgcatctca 2880

attagtcagc aaccaggtgt ggaaagtccc caggctcccc agcaggcaga agtatgcaaa 2940

gcatgcatct caattagtca gcaaccatag tcccgcccct aactccgccc atcccgcccc 3000

taactccgcc cagttccgcc cattctccgc cccatggctg actaattttt tttatttatg 3060

cagaggccga ggccgcctcg gcctctgagc tattccagaa gtagtgagga ggcttttttg 3120

gaggcctagg cttttgcaaa gatcgatcaa gagacaggat gaggatcgtt tcgcatgatt 3180

gaacaagatg gattgcacgc aggttctccg gccgcttggg tggagaggct attcggctat 3240

gactgggcac aacagacaat cggctgctct gatgccgccg tgttccggct gtcagcgcag 3300

gggcgcccgg ttctttttgt caagaccgac ctgtccggtg ccctgaatga actgcaagac 3360

gaggcagcgc ggctatcgtg gctggccacg acgggcgttc cttgcgcagc tgtgctcgac 3420

gttgtcactg aagcgggaag ggactggctg ctattgggcg aagtgccggg gcaggatctc 3480

ctgtcatctc accttgctcc tgccgagaaa gtatccatca tggctgatgc aatgcggcgg 3540

ctgcatacgc ttgatccggc tacctgccca ttcgaccacc aagcgaaaca tcgcatcgag 3600

cgagcacgta ctcggatgga agccggtctt gtcgatcagg atgatctgga cgaagaacat 3660

caggggctcg cgccagccga actgttcgcc aggctcaagg cgagcatgcc cgacggcgag 3720

gatctcgtcg tgacccatgg cgatgcctgc ttgccgaata tcatggtgga aaatggccgc 3780

ttttctggat tcatcgactg tggccggctg ggtgtggcgg accgctatca ggacatagcg 3840

ttggctaccc gtgatattgc tgaagaactt ggcggcgaat gggctgaccg cttcctcgtg 3900

ctttacggta tcgccgctcc cgattcgcag cgcatcgcct tctatcgcct tcttgacgag 3960

ttcttctgag cgggactctg gggttcgaaa tgaccgacca agcgacgccc aacctgccat 4020

cacgagattt cgattccacc gccgccttct atgaaaggtt gggcttcgga atcgttttcc 4080

gggacgccgg ctggatgatc ctccagcgcg gggatctcat gctggagttc ttcgcccacc 4140

ctagggggag gctaactgaa acacggaagg agacaatacc ggaaggaacc cgcgctatga 4200

cggcaataaa aagacagaat aaaacgcacg gtgttgggtc gtttgttcat aaacgcgggg 4260

ttcggtccca gggctggcac tctgtcgata ccccaccgag accccattgg ggccaatacg 4320

cccgcgtttc ttccttttcc ccaccccacc ccccaagttc gggtgaaggc ccagggctcg 4380

cagccaacgt cggggcggca ggccctgcca tagcctcagg ttactcatat atactttaga 4440

ttgatttaaa acttcatttt taatttaaaa ggatctaggt gaagatcctt tttgataatc 4500

tcatgaccaa aatcccttaa cgtgagtttt cgttccactg agcgtcagac cccgtagaaa 4560

agatcaaagg atcttcttga gatccttttt ttctgcgcgt aatctgctgc ttgcaaacaa 4620

aaaaaccacc gctaccagcg gtggtttgtt tgccggatca agagctacca actctttttc 4680

cgaaggtaac tggcttcagc agagcgcaga taccaaatac tgtccttcta gtgtagccgt 4740

agttaggcca ccacttcaag aactctgtag caccgcctac atacctcgct ctgctaatcc 4800

tgttaccagt ggctgctgcc agtggcgata agtcgtgtct taccgggttg gactcaagac 4860

gatagttacc ggataaggcg cagcggtcgg gctgaacggg gggttcgtgc acacagccca 4920

gcttggagcg aacgacctac accgaactga gatacctaca gcgtgagcta tgagaaagcg 4980

ccacgcttcc cgaagggaga aaggcggaca ggtatccggt aagcggcagg gtcggaacag 5040

gagagcgcac gagggagctt ccagggggaa acgcctggta tctttatagt cctgtcgggt 5100

ttcgccacct ctgacttgag cgtcgatttt tgtgatgctc gtcagggggg cggagcctat 5160

ggaaaaacgc cagcaacgcg gcctttttac ggttcctggc cttttgctgg ccttttgctc 5220

acatgttctt tcctgcgtta tcccctgatt ctgtggataa ccgtattacc gcc 5273

<210> 91
<211> 44
<212> DNA
<213> artificial sequence

<220>
<223> chemically synthesized

<400> 91
gaggagggta ccttaattaa ccggccctgg cactcattta ccca 44

<210> 92
<211> 28
<212> DNA
<213> artificial sequence

<220>
<223> chemically synthesized

<220>
<221> misc_feature
<222> (21)..(21)
<223> n is a, c, g, or t

<400> 92

gcggccgaga tcgagctcac ncagwctc                                          28

<210>  93
<211>  23
<212>  DNA
<213>  artificial sequence

<220>
<223>  chemically synthesized

<400>  93
acctttgata tccagtcgtg tcc                                               23

<210>  94
<211>  23
<212>  DNA
<213>  artificial sequence

<220>
<223>  chemically synthesized

<400>  94
acctttgata tccasyttgg tcc                                               23

<210>  95
<211>  27
<212>  DNA
<213>  artificial sequence

<220>
<223>  chemically synthesized

<220>
<221>  misc_feature
<222>  (24)..(24)
<223>  n is a, c, g, or t

<400>  95
caggcggccg agatcgagct cabncar                                           27

<210>  96
<211>  27
<212>  DNA
<213>  artificial sequence

<220>
<223>  chemically synthesized

<400>  96
caggcggccg agatcgagct cabdcag                                           27

<210>  97
<211>  29
<212>  DNA
<213>  artificial sequence

<220>
<223>  chemically synthesized

<400>  97
caggcggccg agatcgagct caykcagcc                                         29


<210>  98
<211>  27
<212>  DNA
<213>  artificial sequence

<220>
<223>  chemically synthesized

<400>  98
caggcggccg agatcgagct cacycar                                           27


<210>  99
<211>  28
<212>  DNA
<213>  artificial sequence

<220>
<223>  chemically synthesized

<400>  99
caggcggccg agatcgagct cactcagc                                          28


<210>  100
<211>  24
<212>  DNA
<213>  artificial sequence

<220>
<223>  chemically synthesized

<400>  100
accgccgagg atatccagct gggt                                              24


<210>  101
<211>  24
<212>  DNA
<213>  artificial sequence

<220>
<223>  chemically synthesized

<400>  101
accgcctagg atatcsasct tggt                                              24


<210>  102
<211>  23
<212>  DNA
<213>  artificial sequence

<220>
<223>  chemically synthesized

<400>  102
saggtgcagc tgctcgagtc kgg                                               23


<210>  103
<211>  21
<212>  DNA
<213>  artificial sequence

<220>
<223>  chemically synthesized

<400>  103
gccactagtg accgatgggc c                                                 21


<210>  104
<211>  15920
<212>  DNA
<213>  artificial sequence

<220>
<223>  chemically synthesized

<400>  104
cgcgttttga gatttctgtc gccgactaaa ttcatgtcgc gcgatagtgg tgtttatcgc      60

cgatagagat ggcgatattg gaaaaatcga tatttgaaaa tatggcatat tgaaaatgtc     120

gccgatgtga gtttctgtgt aactgatatc gccatttttc caaaagtgat ttttgggcat     180

acgcgatatc tggcgatagc gcttatatcg tttacggggg atggcgatag acgactttgg     240

tgacttgggc gattctgtgt gtcgcaaata tcgcagtttc gatataggtg acagacgata     300

tgaggctata tcgccgatag aggcgacatc aagctggcac atggccaatg catatcgatc     360

tatacattga atcaatattg gccattagcc atattattca ttggttatat agcataaatc     420

aatattggct attggccatt gcatacgttg tatccatatc ataatatgta catttatatt     480

ggctcatgtc caacattacc gccatgttga cattgattat tgactagtta ttaatagtaa     540

tcaattacgg ggtcattagt tcatagccca tatatggagt tccgcgttac ataacttacg     600

gtaaatggcc cgcctggctg accgcccaac gacccccgcc cattgacgtc aataatgacg     660

tatgttccca tagtaacgcc aatagggact ttccattgac gtcaatgggt ggagtattta     720

cggtaaactg cccacttggc agtacatcaa gtgtatcata tgccaagtac gccccctatt     780

gacgtcaatg acggtaaatg gcccgcctgg cattatgccc agtacatgac cttatgggac     840

tttcctactt ggcagtacat ctacgtatta gtcatcgcta ttaccatggt gatgcggttt     900

tggcagtaca tcaatgggcg tggatagcgg tttgactcac ggggatttcc aagtctccac     960

cccattgacg tcaatgggag tttgttttgg caccaaaatc aacgggactt tccaaaatgt    1020

cgtaacaact ccgccccatt gacgcaaatg ggcggtaggc gtgtacggtg ggaggtctat 1080

ataagcagag ctcgtttagt gaaccgtcag atcgcctgga gacgccatcc acgctgtttt 1140

gacctccata gaagacaccg ggaccgatcc agcctccgcg gccgggaacg gtgcattgga 1200

acgcggattc cccgtgccaa gagtgacgta agtaccgcct atagagtcta taggcccacc 1260

cccttggctt cttatgcatg ctatactgtt tttggcttgg ggtctataca cccccgcttc 1320

ctcatgttat aggtgatggt atagcttagc ctataggtgt gggttattga ccattattga 1380

ccactcccct attggtgacg atactttcca ttactaatcc ataacatggc tctttgccac 1440

aactctcttt attggctata tgccaataca ctgtccttca gagactgaca cggactctgt 1500

atttttacag gatggggtct catttattat ttacaaattc acatatacaa caccaccgtc 1560

cccagtgccc gcagttttta ttaaacataa cgtgggatct ccacgcgaat ctcgggtacg 1620

tgttccggac atgggctctt ctccggtagc ggcggagctt ctacatccga gccctgctcc 1680

catgcctcca gcgactcatg gtcgctcggc agctccttgc tcctaacagt ggaggccaga 1740

cttaggcaca gcacgatgcc caccaccacc agtgtgccgc acaaggccgt ggcggtaggg 1800

tatgtgtctg aaaatgagct cggggagcgg gcttgcaccg ctgacgcatt tggaagactt 1860

aaggcagcgg cagaagaaga tgcaggcagc tgagttgttg tgttctgata agagtcagag 1920

gtaactcccg ttgcggtgct gttaacggtg gagggcagtg tagtctgagc agtactcgtt 1980

gctgccgcgc gcgccaccag acataatagc tgacagacta acagactgtt cctttccatg 2040

ggtcttttct gcagtcaccg tccttgacac gaagctgtcg cgagtcgcta gcaaggttta 2100

aacgaattca ttgatcataa tcagccatac cacatttgta gaggttttac ttgctttaaa 2160

aaacctccca cacctccccc tgaacctgaa acataaaatg aatgcaattg ttgttgttaa 2220

cttgtttatt gcagcttata atggttacaa ataaagcaat agcatcacaa atttcacaaa 2280

taaagcattt ttttcactgc attctagttg tggtttgtcc aaactcatca atgtatctta 2340

tcatgtctgg cggccgccga tatttgaaaa tatggcatat tgaaaatgtc gccgatgtga 2400

gtttctgtgt aactgatatc gccatttttc caaaagtgat ttttgggcat acgcgatatc 2460

tggcgatagc gcttatatcg tttacggggg atggcgatag acgactttgg tgacttgggc 2520

gattctgtgt gtcgcaaata tcgcagtttc gatataggtg acagacgata tgaggctata 2580

tcgccgatag aggcgacatc aagctggcac atggccaatg catatcgatc tatacattga 2640

atcaatattg gccattagcc atattattca ttggttatat agcataaatc aatattggct 2700

attggccatt gcatacgttg tatccatatc ataatatgta catttatatt ggctcatgtc 2760

caacattacc gccatgttga cattgattat tgactagtta ttaatagtaa tcaattacgg 2820

ggtcattagt tcatagccca tatatggagt tccgcgttac ataacttacg gtaaatggcc 2880

cgcctggctg accgcccaac gacccccgcc cattgacgtc aataatgacg tatgttccca 2940

tagtaacgcc aatagggact ttccattgac gtcaatgggt ggagtattta cggtaaactg 3000

cccacttggc agtacatcaa gtgtatcata tgccaagtac gccccctatt gacgtcaatg 3060

acggtaaatg gcccgcctgg cattatgccc agtacatgac cttatgggac tttcctactt 3120

ggcagtacat ctacgtatta gtcatcgcta ttaccatggt gatgcggttt tggcagtaca 3180

tcaatgggcg tggatagcgg tttgactcac ggggatttcc aagtctccac cccattgacg 3240

tcaatgggag tttgttttgg caccaaaatc aacgggactt tccaaaatgt cgtaacaact 3300

ccgccccatt gacgcaaatg ggcggtaggc gtgtacggtg ggaggtctat ataagcagag 3360

ctcgtttagt gaaccgtcag atcgcctgga gacgccatcc acgctgtttt gacctccata 3420

gaagacaccg ggaccgatcc agcctccgcg gccgggaacg gtgcattgga acgcggattc 3480

cccgtgccaa gagtgacgta agtaccgcct atagagtcta taggcccacc cccttggctt 3540

cttatgcatg ctatactgtt tttggcttgg ggtctataca cccccgcttc ctcatgttat 3600

aggtgatggt atagcttagc ctataggtgt gggttattga ccattattga ccactcccct 3660

attggtgacg atactttcca ttactaatcc ataacatggc tctttgccac aactctcttt 3720

attggctata tgccaataca ctgtccttca gagactgaca cggactctgt atttttacag 3780

gatggggtct catttattat ttacaaattc acatatacaa caccaccgtc cccagtgccc 3840

gcagttttta ttaaacataa cgtgggatct ccacgcgaat ctcgggtacg tgttccggac 3900

atgggctctt ctccggtagc ggcggagctt ctacatccga gccctgctcc catgcctcca 3960

gcgactcatg gtcgctcggc agctccttgc tcctaacagt ggaggccaga cttaggcaca 4020

gcacgatgcc caccaccacc agtgtgccgc acaaggccgt ggcggtaggg tatgtgtctg 4080

aaaatgagct cggggagcgg gcttgcaccg ctgacgcatt tggaagactt aaggcagcgg 4140

cagaagaaga tgcaggcagc tgagttgttg tgttctgata agagtcagag gtaactcccg 4200

ttgcggtgct gttaacggtg gagggcagtg tagtctgagc agtactcgtt gctgccgcgc 4260

gcgccaccag acataatagc tgacagacta acagactgtt cctttccatg ggtcttttct 4320

gcagtcaccg tccttgacac gaagcttggc gcgcccttta attaagactc gagcaattca 4380

ttgatcataa tcagccatac cacatttgta gaggttttac ttgctttaaa aaacctccca 4440

cacctccccc tgaacctgaa acataaaatg aatgcaattg ttgttgttaa cttgtttatt 4500

gcagcttata atggttacaa ataaagcaat agcatcacaa atttcacaaa taaagcattt 4560

ttttcactgc attctagttg tggtttgtcc aaactcatca atgtatctta tcatgtctgg 4620

atcctctaga attcagcaag gtcgccacgc acaagatcaa tattaacaat cagtcatctc 4680

tctttagcaa taaaaaggtg aaaaattaca ttttaaaaat gacaccatag acgatgtatg 4740

aaaataatct acttggaaat aaatctaggc aaagaagtgc aagactgtta cccagaaaac 4800

ttacaaattg taaatgagag gttagtgaag atttaaatga atgaagatct aaataaactt 4860

ataaattgtg agagaaatta atgaatgtct aagttaatgc agaaacggag agacatacta 4920

tattcatgaa ctaaaagact taatattgtg aaggtatact ttcttttcac ataaatttgt 4980

agtcaatatg ttcaccccaa aaaagctgtt tgttaacttg tcaacctcat ttcaaaatgt 5040

atatagaaag cccaaagaca ataacaaaaa tattcttgta gaacaaaatg ggaaagaatg 5100

ttccactaaa tatcaagatt tagagcaaag catgagatgt gtggggatag acagtgaggc 5160

tgataaaata gagtagagct cagaaacaga cccattgata tatgtaagtg acctatgaaa 5220

aaaatatggc attttacaat gggaaaatga tgatcttttt cttttttaga aaaacaggga 5280

aatatattta tatgtaaaaa ataaaaggga acccatatgt cataccatac acacaaaaaa 5340

attccagtga attataagtc taaatggaga aggcaaaact ttaaatcttt tagaaaataa 5400

tatagaagca tgccatcatg acttcagtgt agagaaaaat ttcttatgac tcaaagtcct 5460

aaccacaaag aaaagattgt taattagatt gcatgaatat taagacttat ttttaaaatt 5520

aaaaaaccat taagaaaagt caggccatag aatgacagaa aatatttgca acaccccagt 5580

aaagagaatt gtaatatgca gattataaaa agaagtctta caaatcagta aaaaataaaa 5640

ctagacaaaa atttgaacag atgaaagaga aactctaaat aatcattaca catgagaaac 5700

tcaatctcag aaatcagaga actatcattg catatacact aaattagaga aatattaaaa 5760

ggctaagtaa catctgtggc aatattgatg gtatataacc ttgatatgat gtgatgagaa 5820

cagtacttta ccccatgggc ttcctcccca aacccttacc ccagtataaa tcatgacaaa 5880

tatactttaa aaaccattac cctatatcta accagtactc ctcaaaactg tcaaggtcat 5940

caaaaataag aaaagtctga ggaactgtca aaactaagag gaacccaagg agacatgaga 6000

attatatgta atgtggcatt ctgaatgaga tcccagaaca gaaaaagaac agtagctaaa 6060

aaactaatga aatataaata aagtttgaac tttagttttt tttaaaaaag agtagcatta 6120

acacggcaaa gtcattttca tatttttctt gaacattaag tacaagtcta taattaaaaa 6180

ttttttaaat gtagtctgga acattgccag aaacagaagt acagcagcta tctgtgctgt 6240

cgcctaacta tccatagctg attggtctaa aatgagatac atcaacgctc ctccatgttt 6300

tttgttttct ttttaaatga aaaactttat tttttaagag gagtttcagg ttcatagcaa 6360

aattgagagg aaggtacatt caagctgagg aagttttcct ctattcctag tttactgaga 6420

gattgcatca tgaatgggtg ttaaattttg tcaaatgctt tttctgtgtc tatcaatatg 6480

accatgtgat tttcttcttt aacctgttga tgggacaaat tacgttaatt gattttcaaa 6540
cgttgaacca cccttacata tctggaataa attctacttg gttgtggtgt atattttttg 6600
atacattctt ggattctttt tgctaatatt ttgttgaaaa tgtttgtatc tttgttcatg 6660
agagatattg gtctgttgtt ttcttttctt gtaatgtcat tttctagttc cggtattaag 6720
gtaatgctgg cctagttgaa tgatttagga agtattccct ctgcttctgt cttctgaaag 6780
agattgtaga aagttgatac aatttttttt tctttaaata tcttgataga attctagagg 6840
atcgatcccc gccgccggac gaactaaacc tgactacggc atctctgccc cttcttcgcg 6900
gggcagtgca tgtaatccct tcagttggtt ggtacaactt gccaactggg ccctgttcca 6960
catgtgacac ggggggggac caaacacaaa ggggttctct gactgtagtt gacatcctta 7020
taaatggatg tgcacatttg ccaacactga gtggctttca tcctggagca gactttgcag 7080
tctgtggact gcaacacaac attgccttta tgtgtaactc ttggctgaag ctcttacacc 7140
aatgctgggg gacatgtacc tcccaggggc ccaggaagac tacgggaggc tacaccaacg 7200
tcaatcagag gggcctgtgt agctaccgat aagcggaccc tcaagagggc attagcaata 7260
gtgtttataa ggcccccttg ttaaccctaa acgggtagca tatgcttccc gggtagtagt 7320
atatactatc cagactaacc ctaattcaat agcatatgtt acccaacggg aagcatatgc 7380
tatcgaatta gggttagtaa aagggtccta aggaacagcg atatctccca ccccatgagc 7440
tgtcacggtt ttatttacat ggggtcagga ttccacgagg gtagtgaacc attttagtca 7500
caagggcagt ggctgaagat caaggagcgg gcagtgaact ctcctgaatc ttcgcctgct 7560
tcttcattct ccttcgttta gctaatagaa taactgctga gttgtgaaca gtaaggtgta 7620
tgtgaggtgc tcgaaaacaa ggtttcaggt gacgccccca gaataaaatt tggacggggg 7680
gttcagtggt ggcattgtgc tatgacacca atataaccct cacaaacccc ttgggcaata 7740
aatactagtg taggaatgaa acattctgaa tatctttaac aatagaaatc catggggtgg 7800
ggacaagccg taaagactgg atgtccatct cacacgaatt tatggctatg ggcaacacat 7860
aatcctagtg caatatgata ctggggttat taagatgtgt cccaggcagg gaccaagaca 7920
ggtgaaccat gttgttacac tctatttgta acaaggggaa agagagtgga cgccgacagc 7980
agcggactcc actggttgtc tctaacaccc ccgaaaatta aacggggctc cacgccaatg 8040
gggcccataa acaaagacaa gtggccactc ttttttttga aattgtggag tgggggcacg 8100
cgtcagcccc cacacgccgc cctgcggttt tggactgtaa aataagggtg taataacttg 8160
gctgattgta accccgctaa ccactgcggt caaaccactt gcccacaaaa ccactaatgg 8220
caccccgggg aatacctgca taagtaggtg ggcgggccaa gataggggcg cgattgctgc 8280
gatctggagg acaaattaca cacacttgcg cctgagcgcc aagcacaggg ttgttggtcc 8340

tcatattcac gaggtcgctg agagcacggt gggctaatgt tgccatgggt agcatatact 8400

acccaaatat ctggatagca tatgctatcc taatctatat ctgggtagca taggctatcc 8460

taatctatat ctgggtagca tatgctatcc taatctatat ctgggtagta tatgctatcc 8520

taatttatat ctgggtagca taggctatcc taatctatat ctgggtagca tatgctatcc 8580

taatctatat ctgggtagta tatgctatcc taatctgtat ccgggtagca tatgctatcc 8640

taatagagat tagggtagta tatgctatcc taatttatat ctgggtagca tatactaccc 8700

aaatatctgg atagcatatg ctatcctaat ctatatctgg gtagcatatg ctatcctaat 8760

ctatatctgg gtagcatagg ctatcctaat ctatatctgg gtagcatatg ctatcctaat 8820

ctatatctgg gtagtatatg ctatcctaat ttatatctgg gtagcatagg ctatcctaat 8880

ctatatctgg gtagcatatg ctatcctaat ctatatctgg gtagtatatg ctatcctaat 8940

ctgtatccgg gtagcatatg ctatcctcat gcatatacag tcagcatatg atacccagta 9000

gtagagtggg agtgctatcc tttgcatatg ccgccacctc ccaagggggc gtgaattttc 9060

gctgcttgtc cttttcctgc atgctggttg ctcccattct taggtgaatt taaggaggcc 9120

aggctaaagc cgtcgcatgt ctgattgctc accaggtaaa tgtcgctaat gttttccaac 9180

gcgagaaggt gttgagcgcg gagctgagtg acgtgacaac atgggtatgc ccaattgccc 9240

catgttggga ggacgaaaat ggtgacaaga cagatggcca gaaatacacc aacagcacgc 9300

atgatgtcta ctggggattt attctttagt gcgggggaat acacggcttt taatacgatt 9360

gagggcgtct cctaacaagt tacatcactc ctgcccttcc tcaccctcat ctccatcacc 9420

tccttcatct ccgtcatctc cgtcatcacc ctccgcggca gccccttcca ccataggtgg 9480

aaaccaggga ggcaaatcta ctccatcgtc aaagctgcac acagtcaccc tgatattgca 9540

ggtaggagcg ggctttgtca taacaaggtc cttaatcgca tccttcaaaa cctcagcaaa 9600

tatatgagtt tgtaaaaaga ccatgaaata acagacaatg gactccctta gcgggccagg 9660

ttgtgggccg ggtccagggg ccattccaaa ggggagacga ctcaatggtg taagacgaca 9720

ttgtggaata gcaagggcag ttcctcgcct taggttgtaa agggaggtct tactacctcc 9780

atatacgaac acaccggcga cccaagttcc ttcgtcggta gtcctttcta cgtgactcct 9840

agccaggaga gctcttaaac cttctgcaat gttctcaaat ttcgggttgg aacctccttg 9900

accacgatgc tttccaaacc accctccttt tttgcgcctg cctccatcac cctgaccccg 9960

gggtccagtg cttgggcctt ctcctgggtc atctgcgggg ccctgctcta tcgctcccgg 10020

gggcacgtca ggctcaccat ctgggccacc ttcttggtgg tattcaaaat aatcggcttc 10080

ccctacaggg tggaaaaatg gccttctacc tggagggggc ctgcgcggtg gagacccgga 10140

tgatgatgac tgactactgg gactcctggg cctcttttct ccacgtccac gacctctccc 10200
cctggctctt tcacgacttc cccccctggc tctttcacgt cctctacccc ggcggcctcc 10260
actacctcct cgaccccggc ctccactacc tcctcgaccc cggcctccac tgcctcctcg 10320
accccggcct ccacctcctg ctcctgcccc tcctgctcct gcccctcctc ctgctcctgc 10380
ccctcctgcc cctcctgctc ctgcccctcc tgcccctcct gctcctgccc ctcctgcccc 10440
tcctgctcct gcccctcctg cccctcctcc tgctcctgcc cctcctgccc ctcctcctgc 10500
tcctgcccct cctgcccctc ctgctcctgc ccctcctgcc cctcctgctc ctgcccctcc 10560
tgcccctcct gctcctgccc ctcctgctcc tgcccctcct gctcctgccc ctcctgctcc 10620
tgcccctcct gcccctcctg cccctcctcc tgctcctgcc cctcctgctc ctgcccctcc 10680
tgcccctcct gcccctcctg ctcctgcccc tcctcctgct cctgcccctc ctgcccctcc 10740
tgcccctcct cctgctcctg cccctcctgc ccctcctcct gctcctgccc ctcctcctgc 10800
tcctgcccct cctgcccctc ctgcccctcc tcctgctcct gcccctcctg cccctcctcc 10860
tgctcctgcc cctcctcctg ctcctgcccc tcctgcccct cctgcccctc ctcctgctcc 10920
tgcccctcct cctgctcctg cccctcctgc ccctcctgcc cctcctgccc ctcctcctgc 10980
tcctgcccct cctcctgctc ctgcccctcc tgctcctgcc cctcccgctc ctgctcctgc 11040
tcctgttcca ccgtgggtcc ctttgcagcc aatgcaactt ggacgttttt ggggtctccg 11100
gacaccatct ctatgtcttg gccctgatcc tgagccgccc ggggctcctg gtcttccgcc 11160
tcctcgtcct cgtcctcttc cccgtcctcg tccatggtta tcaccccctc ttctttgagg 11220
tccactgccg ccggagcctt ctggtccaga tgtgtctccc ttctctccta ggccatttcc 11280
aggtcctgta cctggcccct cgtcagacat ggtaaatcga tggccatggt ggccacgtgt 11340
tcacgacacc tgaaatggaa gaaaaaaact ttgaaccact gtctgaggct tgagaatgaa 11400
ccaagatcca aactcaaaaa ggccaaattc caaggagaat tacatcaagt gccaagctgg 11460
cctaacttca gtctccaccc actcagtgtg gggaaactcc atcgcataaa acccctcccc 11520
ccaacctaaa gacgacgtac tccaaaagct ccagaactaa tcgaggtgcc tggacggcgc 11580
ccggtactcc gtggagtcac atgaagcgac ggctgaggac ggaaaggccc ttttcctttg 11640
tgtgggtgac tcacccgccc gctctcccga gcgccgcgtc ctccattttg agccccctgg 11700
agcagggccg ggaagcggcc atctttccgc tcacgcaact ggtgccgacc gggccagcct 11760
tgccgcccag ggcggggcga tacacggcgg cgcgaggcca ggcaccagag caggccggcc 11820
agcttgagac tacccccgtc cgattctcgg tggccgcgct cgcaggcccc gcctcgccga 11880
acatgtgcgc tgggacgcac gggccccgtc gccggccgcg ggcccaaaaa ccgaaatacc 11940
agtgtgcaga tcctggcccg catttacaag actatcttgc cagaaaaaaa gcgtcgcagc 12000

aggtcatcaa aaattttaaa tggctagaga cttatcgaaa gcagcgagac aggcgcgaag 12060

gtgccaccag attcgcacgc ggcggcccca gcgcccaggc caggcctcaa ctcaagcacg 12120

aggcgaaggg gctcctaaag cgcaaggccc gcccctggct ccagctcggg atcaagaatc 12180

acgtactgga gccaggtgga agtaattcaa ggcacgcaag ggccataacc cgtaaagagg 12240

ccaggcccgc gggaaccaca cacggcactt acctgtgttc tggcggcaaa cccgttgcga 12300

aaaagaacgt tcacggcgac tactgcactt atatacggtt ctcccccacc ctcgggaaaa 12360

aggcggagcc agtacacgac atcactttcc cagtttaccc cgcgccacct tctctaggca 12420

ccggttcaat tgccgacccc tccccccaac ttctcgggga ctgtgggcga tgtgcgctct 12480

gcccactgac gggcaccgga gcctcacgaa gcttgaattc ggtaccatcg atgataagct 12540

gtcaaacatg agaattcttg aagacgaaag ggcctcgtga tacgcctatt tttataggtt 12600

aatgtcatga taataatggt ttcttagacg tcaggtggca cttttcgggg aaatgtgcgc 12660

ggaaccccta tttgtttatt tttctaaata cattcaaata tgtatccgct catgagacaa 12720

taaccctgat aaatgcttca ataatattga aaaaggaaga gtatgagtat tcaacatttc 12780

cgtgtcgccc ttattccctt ttttgcggca ttttgccttc ctgtttttgc tcacccagaa 12840

acgctggtga aagtaaaaga tgctgaagat cagttgggtg cacgagtggg ttacatcgaa 12900

ctggatctca acagcggtaa gatccttgag agttttcgcc ccgaagaacg ttttccaatg 12960

atgagcactt ttaaagttct gctatgtggc gcggtattat cccgtgttga cgccgggcaa 13020

gagcaactcg gtcgccgcat acactattct cagaatgact tggttgagta ctcaccagtc 13080

acagaaaagc atcttacgga tggcatgaca gtaagagaat tatgcagtgc tgccataacc 13140

atgagtgata acactgcggc caacttactt ctgacaacga tcggaggacc gaaggagcta 13200

accgcttttt tgcacaacat gggggatcat gtaactcgcc ttgatcgttg ggaaccggag 13260

ctgaatgaag ccataccaaa cgacgagcgt gacaccacga tgcctgcagc aatggcaaca 13320

acgttgcgca aactattaac tggcgaacta cttactctag cttcccggca acaattaata 13380

gactggatgg aggcggataa agttgcagga ccacttctgc gctcggccct tccggctggc 13440

tggtttattg ctgataaatc tggagccggt gagcgtgggt ctcgcggtat cattgcagca 13500

ctggggccag atggtaagcc ctcccgtatc gtagttatct acacgacggg gagtcaggca 13560

actatggatg aacgaaatag acagatcgct gagataggtg cctcactgat taagcattgg 13620

taactgtcag accaagttta ctcatatata ctttagattg atttaaaact tcatttttaa 13680

tttaaaagga tctaggtgaa gatccttttt gataatctca tgaccaaaat cccttaacgt 13740

gagttttcgt tccactgagc gtcagacccc gtagaaaaga tcaaaggatc ttcttgagat 13800

cctttttttc tgcgcgtaat ctgctgcttg caaacaaaaa aaccaccgct accagcggtg 13860

gtttgtttgc cggatcaaga gctaccaact ctttttccga aggtaactgg cttcagcaga 13920

gcgcagatac caaatactgt ccttctagtg tagccgtagt taggccacca cttcaagaac 13980

tctgtagcac cgcctacata cctcgctctg ctaatcctgt taccagtggc tgctgccagt 14040

ggcgataagt cgtgtcttac cgggttggac tcaagacgat agttaccgga taaggcgcag 14100

cggtcgggct gaacgggggg ttcgtgcaca cagcccagct tggagcgaac gacctacacc 14160

gaactgagat acctacagcg tgagctatga gaaagcgcca cgcttcccga agggagaaag 14220

gcggacaggt atccggtaag cggcagggtc ggaacaggag agcgcacgag ggagcttcca 14280

gggggaaacg cctggtatct ttatagtcct gtcgggtttc gccacctctg acttgagcgt 14340

cgatttttgt gatgctcgtc aggggggcgg agcctatgga aaaacgccag caacgcggcc 14400

tttttacggt tcctggcctt ttgctgcgcc gcgtgcggct gctggagatg gcggacgcga 14460

tggatatgtt ctgccaaggg ttggtttgcg cattcacagt tctccgcaag aattgattgg 14520

ctccaattct tggagtggtg aatccgttag cgaggccatc cagcctcgcg tcgaactaga 14580

tgatccgctg tggaatgtgt gtcagttagg gtgtggaaag tccccaggct ccccagcagg 14640

cagaagtatg caaagcatgc atctcaatta gtcagcaacc aggtgtggaa agtccccagg 14700

ctccccagca ggcagaagta tgcaaagcat gcatctcaat tagtcagcaa ccatagtccc 14760

gcccctaact ccgcccatcc cgcccctaac tccgcccagt tccgcccatt ctccgcccca 14820

tggctgacta atttttttta tttatgcaga ggccgaggcc gcctcggcct ctgagctatt 14880

ccagaagtag tgaggaggct tttttggagg gtgaccgcca cgaggtgccg ccaccatccc 14940

ctgacccacg cccctgaccc ctcacaagga gacgaccttc catgaccgag tacaagccca 15000

cggtgcgcct cgccacccgc gacgacgtcc cccgggccgt acgcaccctc gccgccgcgt 15060

tcgccgacta ccccgccacg cgccacaccg tcgaccccga ccgccacatc gaacgcgtca 15120

ccgagctgca agaactcttc ctcacgcgcg tcgggctcga catcggcaag gtgtgggtcg 15180

cggacgacgg cgccgcggtg gcggtctgga ccacgccgga gagcgtcgaa gcgggggcgg 15240

tgttcgccga gatcggcccg cgcatggccg agttgagcgg ttcccggctg gccgcgcagc 15300

aacagatgga aggcctcctg gcgccgcacc ggcccaagga gcccgcgtgg ttcctggcca 15360

ccgtcggcgt ctcgcccgac caccagggca agggtctggg cagcgccgtc gtgctccccg 15420

gagtggaggc ggccgagcgc gccggggtgc ccgccttcct ggagacctcc gcgccccgca 15480

acctcccctt ctacgagcgg ctcggcttca ccgtcaccgc cgacgtcgag tgcccgaagg 15540

accgcgcgac ctggtgcatg acccgcaagc ccggtgcctg acgcccgccc cacgacccgc 15600

agcgcccgac cgaaaggagc gcacgacccg gtccgacggc ggcccacggg tcccaggggg 15660

gtcgacctcg aaacttgttt attgcagctt ataatggtta caaataaagc aatagcatca 15720

caaatttcac aaataaagca tttttttcac tgcattctag ttgtggtttg tccaaactca 15780

tcaatgtatc ttatcatgtc tggatcgatc cgaacccctt cctcgaccaa ttctcatgtt 15840

tgacagctta tcatcgcaga tccgggcaac gttgttgcat tgctgcaggc gcagaactgg 15900

taggtatgga agatctgggg 15920

<210> 105
<211> 21
<212> PRT
<213> Mus musculus

<400> 105

Met Glu Thr Asp Thr Leu Leu Leu Trp Val Leu Leu Leu Trp Val Pro
1               5                   10                  15

Gly Ser Thr Gly Asp
            20

<210> 106
<211> 51
<212> PRT
<213> Homo sapiens

<400> 106

Arg Asn Ala Val Gly Gln Asp Thr Gln Glu Val Ile Val Val Pro His
1               5                   10                  15

Ser Leu Pro Phe Lys Val Val Val Ile Ser Ala Ile Leu Ala Leu Val
            20                  25                  30

Val Leu Thr Ile Ile Ser Leu Ile Ile Leu Ile Met Leu Trp Gln Lys
        35                  40                  45

Lys Pro Arg
    50

<210> 107
<211> 18
<212> PRT
<213> artificial sequence

<220>
<223> chemically synthesized

<400> 107

Gly Gly Ser Ser Arg Ser Ser Ser Ser Gly Gly Gly Gly Ser Gly Gly

1               5                   10                  15

Gly Gly

<210>  108
<211>  10
<212>  PRT
<213>  artificial sequence

<220>
<223>  chemically synthesized

<400>  108

Tyr Pro Tyr Asp Val Pro Asp Tyr Ala Ser
1               5                   10

<210>  109
<211>  225
<212>  PRT
<213>  artificial sequence

<220>
<223>  chemically synthesized

<400>  109

Thr His Thr Cys Pro Pro Cys Pro Ala Pro Glu Ala Glu Gly Ala Pro
1               5                   10                  15

Ser Val Phe Leu Phe Pro Pro Lys Pro Lys Asp Thr Leu Met Ile Ser
            20                  25                  30

Arg Thr Pro Glu Val Thr Cys Val Val Val Asp Val Ser His Glu Asp
        35                  40                  45

Pro Glu Val Lys Phe Asn Trp Tyr Val Asp Gly Val Glu Val His Asn
    50                  55                  60

Ala Lys Thr Lys Pro Arg Glu Glu Gln Tyr Asn Ser Thr Tyr Arg Val
65                  70                  75                  80

Val Ser Val Leu Thr Val Leu His Gln Asp Trp Leu Asn Gly Lys Glu
                85                  90                  95

Tyr Lys Cys Lys Val Ser Asn Lys Ala Leu Pro Ala Ser Ile Glu Lys
            100                 105                 110

Thr Ile Ser Lys Ala Lys Gly Gln Pro Arg Glu Pro Gln Val Tyr Thr
        115                 120                 125

Leu Pro Pro Ser Arg Asp Glu Leu Thr Lys Asn Gln Val Ser Leu Thr
    130                 135                 140

Cys Leu Val Lys Gly Phe Tyr Pro Ser Asp Ile Ala Val Glu Trp Glu
145                 150                 155                 160

Ser Asn Gly Gln Pro Glu Asn Asn Tyr Lys Thr Thr Pro Pro Val Leu
                165                 170                 175

Asp Ser Asp Gly Ser Phe Phe Leu Tyr Ser Lys Leu Thr Val Asp Lys
            180                 185                 190

Ser Arg Trp Gln Gln Gly Asn Val Phe Ser Cys Ser Val Met His Glu
        195                 200                 205

Ala Leu His Asn His Tyr Thr Gln Lys Ser Leu Ser Leu Ser Pro Gly
    210                 215                 220

Lys
225

<210>  110
<211>  4613
<212>  DNA
<213>  artificial sequence

<220>
<223>  chemically synthesized

<400>  110
gagctggcta gcgccaccat ggcctgggct ctgctcctcc tcaccctcct cactcagggc      60

acagggtcct gggcccagtc tgagctcctg caggaattcg atatcctagg tcagcccaag     120

gctgccccct cggtcactct gttcccgccc tcctctgagg agcttcaagc caacaaggcc     180

acactggtgt gtctcataag tgacttctac ccgggagccg tgacagtggc ctggaaggca     240

gatagcagcc ccgtcaaggc gggagtggag accaccacac cctccaaaca aagcaacaac     300

aagtacgcgg ccagcagcta tctgagcctg acgcctgagc agtggaagtc ccacagaagc     360

tacagctgcc aggtcacgca tgaagggagc accgtggaga agacagtggc ccctacagaa     420

tgttcatagg tttaaacggt accaggtaag tgtacccaat tcgccctata gtgagtcgta     480

ttacaattca ctcgatcgcc cttcccaaca gttgcgcagc ctgaatggcg aatggagatc     540

caatttttaa gtgtataatg tgttaaacta ctgattctaa ttgtttgtgt attttagatt     600

cacagtccca aggctcattt caggcccctc agtcctcaca gtctgttcat gatcataatc     660

agccatacca catttgtaga ggttttactt gctttaaaaa acctcccaca cctccccctg 720

aacctgaaac ataaaatgaa tgcaattgtt gttgttaact tgtttattgc agcttataat 780

ggttacaaat aaagcaatag catcacaaat ttcacaaata aagcattttt ttcactgcat 840

tctagttgtg gtttgtccaa actcatcaat gtatcttaac gcgtaaattg taagcgttaa 900

tattttgtta aaattcgcgt taaatttttg ttaaatcagc tcatttttta accaataggc 960

cgaaatcggc aaaatccctt ataaatcaaa agaatagacc gagatagggt tgagtgttgt 1020

tccagtttgg aacaagagtc cactattaaa gaacgtggac tccaacgtca aagggcgaaa 1080

aaccgtctat cagggcgatg gcccactacg tgaaccatca ccctaatcaa gttttttggg 1140

gtcgaggtgc cgtaaagcac taaatcggaa ccctaaaggg agcccccgat ttagagcttg 1200

acggggaaag ccggcgaacg tggcgagaaa ggaagggaag aaagcgaaag gagcgggcgc 1260

tagggcgctg gcaagtgtag cggtcacgct gcgcgtaacc accacacccg ccgcgcttaa 1320

tgcgccgcta cagggcgcgt caggtggcac ttttcgggga aatgtgcgcg gaacccctat 1380

ttgtttattt ttctaaatac attcaaatat gtatccgctc atgagacaat aaccctgata 1440

aatgcttcaa taatattgaa aaaggaagaa tcctgaggcg gaaagaacca gctgtggaat 1500

gtgtgtcagt tagggtgtgg aaagtcccca ggctccccag caggcagaag tatgcaaagc 1560

atgcatctca attagtcagc aaccaggtgt ggaaagtccc caggctcccc agcaggcaga 1620

agtatgcaaa gcatgcatct caattagtca gcaaccatag tcccgcccct aactccgccc 1680

atcccgcccc taactccgcc cagttccgcc cattctccgc cccatggctg actaattttt 1740

tttatttatg cagaggccga ggccgcctcg gcctctgagc tattccagaa gtagtgagga 1800

ggcttttttg gaggcctagg cttttgcaaa gatcgatcaa gagacaggat gaggatcgtt 1860

tcgcatgatt gaacaagatg gattgcacgc aggttctccg gccgcttggg tggagaggct 1920

attcggctat gactgggcac aacagacaat cggctgctct gatgccgccg tgttccggct 1980

gtcagcgcag gggcgcccgg ttctttttgt caagaccgac ctgtccggtg ccctgaatga 2040

actgcaagac gaggcagcgc ggctatcgtg gctggccacg acgggcgttc cttgcgcagc 2100

tgtgctcgac gttgtcactg aagcgggaag ggactggctg ctattgggcg aagtgccggg 2160

gcaggatctc ctgtcatctc accttgctcc tgccgagaaa gtatccatca tggctgatgc 2220

aatgcggcgg ctgcatacgc ttgatccggc tacctgccca ttcgaccacc aagcgaaaca 2280

tcgcatcgag cgagcacgta ctcggatgga agccggtctt gtcgatcagg atgatctgga 2340

cgaagaacat caggggctcg cgccagccga actgttcgcc aggctcaagg cgagcatgcc 2400

cgacggcgag gatctcgtcg tgacccatgg cgatgcctgc ttgccgaata tcatggtgga 2460

aaatggccgc ttttctggat tcatcgactg tggccggctg ggtgtggcgg accgctatca 2520

ggacatagcg ttggctaccc gtgatattgc tgaagaactt ggcggcgaat gggctgaccg 2580

cttcctcgtg ctttacggta tcgccgctcc cgattcgcag cgcatcgcct tctatcgcct 2640

tcttgacgag ttcttctgag cgggactctg gggttcgaaa tgaccgacca agcgacgccc 2700

aacctgccat cacgagattt cgattccacc gccgccttct atgaaaggtt gggcttcgga 2760

atcgttttcc gggacgccgg ctggatgatc ctccagcgcg gggatctcat gctggagttc 2820

ttcgcccacc ctagggggag gctaactgaa acacggaagg agacaatacc ggaaggaacc 2880

cgcgctatga cggcaataaa aagacagaat aaaacgcacg gtgttgggtc gtttgttcat 2940

aaacgcgggg ttcggtccca gggctggcac tctgtcgata ccccaccgag accccattgg 3000

ggccaatacg cccgcgtttc ttccttttcc ccaccccacc ccccaagttc gggtgaaggc 3060

ccagggctcg cagccaacgt cggggcggca ggccctgcca tagcctcagg ttactcatat 3120

atactttaga ttgatttaaa acttcatttt taatttaaaa ggatctaggt gaagatcctt 3180

tttgataatc tcatgaccaa aatcccttaa cgtgagtttt cgttccactg agcgtcagac 3240

cccgtagaaa agatcaaagg atcttcttga gatccttttt ttctgcgcgt aatctgctgc 3300

ttgcaaacaa aaaaaccacc gctaccagcg gtggtttgtt tgccggatca agagctacca 3360

actctttttc cgaaggtaac tggcttcagc agagcgcaga taccaaatac tgtccttcta 3420

gtgtagccgt agttaggcca ccacttcaag aactctgtag caccgcctac atacctcgct 3480

ctgctaatcc tgttaccagt ggctgctgcc agtggcgata agtcgtgtct taccgggttg 3540

gactcaagac gatagttacc ggataaggcg cagcggtcgg gctgaacggg gggttcgtgc 3600

acacagccca gcttggagcg aacgacctac accgaactga gatacctaca gcgtgagcta 3660

tgagaaagcg ccacgcttcc cgaagggaga aaggcggaca ggtatccggt aagcggcagg 3720

gtcggaacag gagagcgcac gagggagctt ccagggggaa acgcctggta tctttatagt 3780

cctgtcgggt ttcgccacct ctgacttgag cgtcgatttt tgtgatgctc gtcagggggg 3840

cggagcctat ggaaaaacgc cagcaacgcg gcctttttac ggttcctggc cttttgctgg 3900

ccttttgctc acatgttctt tcctgcgtta tcccctgatt ctgtggataa ccgtattacc 3960

gccatgcatt agttattaat agtaatcaat tacggggtca ttagttcata gcccatatat 4020

ggagttccgc gttacataac ttacggtaaa tggcccgcct ggctgaccgc ccaacgaccc 4080

ccgcccattg acgtcaataa tgacgtatgt tcccatagta acgccaatag ggactttcca 4140

ttgacgtcaa tgggtggagt atttacggta aactgcccac ttggcagtac atcaagtgta 4200

tcatatgcca agtacgcccc ctattgacgt caatgacggt aaatggcccg cctggcatta 4260

tgcccagtac atgaccttat gggactttcc tacttggcag tacatctacg tattagtcat 4320

cgctattacc atggtgatgc ggttttggca gtacatcaat gggcgtggat agcggtttga 4380

ctcacgggga tttccaagtc tccaccccat tgacgtcaat gggagtttgt tttggcacca 4440

aaatcaacgg gactttccaa aatgtcgtaa caactccgcc ccattgacgc aaatgggcgg 4500

taggcgtgta cggtgggagg tctatataag cagagctggt ttagtgaacc gtcagatccg 4560

ctagcgatta cgccaagctc gaaattaacc ctcactaaag ggaacaaaag ctg 4613

<210> 111
<211> 45
<212> DNA
<213> artificial sequence

<220>
<223> chemically synthesized

<400> 111
ggctagcgcc accatggcct gggctctgct cctcctcacc ctcct 45

<210> 112
<211> 43
<212> DNA
<213> artificial sequence

<220>
<223> chemically synthesized

<400> 112
gtgaggagga gcagagccca ggccatggtg gcgctagcca gct 43

<210> 113
<211> 42
<212> DNA
<213> artificial sequence

<220>
<223> chemically synthesized

<400> 113
cactcagggc acagggtcct gggcccagtc tgagctcctg ca 42

<210> 114
<211> 44
<212> DNA
<213> artificial sequence

<220>
<223> chemically synthesized

<400> 114
ggagctcaga ctgggcccag gaccctgtgc cctgagtgag gagg 44

<210> 115
<211> 35

<212>  DNA
<213>  artificial sequence

<220>
<223>  chemically synthesized

<400>  115
gaggaggata tcctaggtca gcccaaggct gcccc                                35


<210>  116
<211>  41
<212>  DNA
<213>  artificial sequence

<220>
<223>  chemically synthesized

<400>  116
gaggagggta ccgtttaaac ctatgaacat tctgtagggg c                         41


<210>  117
<211>  1075
<212>  DNA
<213>  artificial sequence

<220>
<223>  chemically synthseized

<400>  117
ggcgcgccac catggactgg acctggagga tcctcttctt ggtggcagca gccacaggag      60

cccactccca gatgcaactg ctcgaggcct ccaccaaggg cccatcggtc ttccccctgg     120

cgccctgctc caggagcacc tccgagagca cagcggccct gggctgcctg gtcaaggact     180

acttccccga accggtgacg gtgtcgtgga actcaggcgc tctgaccagc ggcgtgcaca     240

ccttcccagc tgtcctacag tcctcaggac tctactccct cagcagcgtg gtgaccgtgc     300

cctccagcaa cttcggcacc cagacctaca cctgcaacgt agatcacaag cccagcaaca     360

ccaaggtgga caagacagtt gagcgcaaat gttgtgtcga gtgcccaccg tgcccagcac     420

cacctgtggc aggaccgtca gtcttcctct tccccccaaa acccaaggac accctcatga     480

tctcccggac ccctgaggtc acgtgcgtgg tggtggacgt gagccacgaa gaccccgagg     540

tccagttcaa ctggtacgtg gacggcgtgg aggtgcataa tgccaagaca aagccacggg     600

aggagcagtt caacagcacg ttccgtgtgg tcagcgtcct caccgttgtg caccaggact     660

ggctgaacgg caaggagtac aagtgcaagg tctccaacaa aggcctccca gcccccatcg     720

agaaaaccat ctccaaaacc aaagggcagc cccgagaacc acaggtgtac accctgcccc     780

catcccggga ggagatgacc aagaaccagg tcagcctgac ctgcctggtc aaaggcttct     840

accccagcga catcgccgtg gagtgggaga gcaatgggca gccggagaac aactacaaga     900

ccacacctcc catgctggac tccgacggct ccttcttcct ctacagcaag ctcaccgtgg 960

acaagagcag gtggcagcag gggaacgtct tctcatgctc cgtgatgcat gaggctctgc 1020

acaaccacta cacgcagaag agcctgtccc tgtctccggg taaatgatta attaa 1075

<210> 118
<211> 1087
<212> DNA
<213> artificial sequence

<220>
<223> chemically synthesized

<400> 118
ggcgcgccac catggactgg acctggagga tcctcttctt ggtggcagca gccacaggag 60

cccactccca gatgcaactg ctcgaggcct ccaccaaggg cccatcggtc ttccccctgg 120

caccctcctc caagagcacc tctgggggca cagcggccct gggctgcctg gtcaaggact 180

acttccccga accggtgacg gtgtcgtgga actcaggcgc cctgaccagc ggcgtgcaca 240

ccttcccggc tgtcctacag tcctcaggac tctactccct cagcagcgtg gtgaccgtgc 300

cctccagcag cttgggcacc cagacctaca tctgcaacgt gaatcacaag cccagcaaca 360

ccaaggtgga caagaaagtt gagcccaaat cttgtgacaa aactcacaca tgcccaccgt 420

gcccagcacc tgaactcctg gggggaccgt cagtcttcct cttcccccca aaacccaagg 480

acaccctcat gatctcccgg acccctgagg tcacatgcgt ggtggtggac gtgagccacg 540

aagaccctga ggtcaagttc aactggtacg tggacggcgt ggaggtgcat aatgccaaga 600

caaagccgcg ggaggagcag tacaacagca cgtaccgtgt ggtcagcgtc ctcaccgtcc 660

tgcaccagga ctggctgaat ggcaaggagt acaagtgcaa ggtctccaac aaagccctcc 720

cagcccccat cgagaaaacc atctccaaag ccaaagggca gccccgagaa ccacaggtgt 780

acaccctgcc cccatcccgg gatgagctga ccaagaacca ggtcagcctg acctgcctgg 840

tcaaaggctt ctatcccagc gacatcgccg tggagtggga gagcaatggg cagccggaga 900

acaactacaa gaccacgcct cccgtgctgg actccgacgg ctccttcttc ctctacagca 960

agctcaccgt ggacaagagc aggtggcagc aggggaacgt cttctcatgc tccgtgatgc 1020

atgaggctct gcacaaccac tacacgcaga agagcctctc cctgtctccg ggtaaatgat 1080

taattaa 1087

<210> 119
<211> 1091
<212> DNA
<213> artificial sequence

<220>

<223>  chemically synthesized

<400>  119
ggcgcgccac catggactgg acctggagga tcctcttctt ggtggcagca gccacaggag      60

cccactccca gatgcaactg ctcgaggcct ccaccaaggg cccatcggtc ttccccctgg     120

cgccctgctc caggagcacc tccgagagca cagcggccct gggctgcctg gtcaaggact     180

acttccccga accggtgacg gtgtcgtgga actcaggcgc cctgaccagc ggcgtgcaca     240

ccttcccggc tgtcctacag tcctcaggac tctactccct cagcagcgtg gtgaccgtgc     300

cctccagcag cttgggcacg aagacctaca cctgcaatgt agatcacaag cccagcaaca     360

ccaaggtgga caagagagtt gagtccaaat atggtccccc gtgcccatca tgcccagcac     420

ctgaattcct ggggggacca tcagtcttcc tgttcccccc aaaacccaag gacaccctca     480

tgatctcccg gacccctgag gtcacgtgcg tggtggtgga cgtgagccag gaagaccccg     540

aggtccagtt caactggtac gtggatggcg tggaggtgca taatgccaag acaaagccgc     600

gggaggagca gttcaacagc acgtaccgtg tggtcagcgt cctcaccgtc gtgcaccagg     660

actggctgaa cggcaaggag tacaagtgca aggtctccaa caaaggcctc ccgtcctcca     720

tcgagaaaac catctccaaa gccaaagggc agccccgaga gccacaggtg tacaccctgc     780

ccccatccca ggaggagatg accaagaacc aggtcagcct gacctgcctg gtcaaaggct     840

tctaccccag cgacatcgcc gtggagtggg agagcaatgg gcagccggag aacaactaca     900

agaccacgcc tcccgtgctg gactccgacg gctccttctt cctctacagc aggctaaccg     960

tggacaagag caggtggcag gaggggaatg tcttctcatg ctccgtgatg catgaggctc    1020

tgcacaacca ctacacgcag aagagcctct ccctgtctct gggtaaatga gtgccagggc    1080

cggttaatta a                                                         1091


<210>  120
<211>  400
<212>  DNA
<213>  artificial sequence

<220>
<223>  chemically synthesized

<400>  120
ggcgcgccac catggactgg acctggagga tcctcttctt ggtggcagca gccacaggag      60

cccactccca gatgcaactg ctcgaggcct ccaccaaggg cccatcggtc ttccccctgg     120

cgccctgctc caggagcacc tccgagagca cagcggccct gggctgcctg gtcaaggact     180

acttccccga accggtgacg gtgtcgtgga actcaggcgc tctgaccagc ggcgtgcaca     240

ccttcccagc tgtcctacag tcctcaggac tctactccct cagcagcgtg gtgaccgtgc     300

cctccagcaa cttcggcacc cagacctaca cctgcaacgt agatcacaag cccagcaaca 360

ccaaggtgga caagacagtt gagcgcaaat gattaattaa 400

<210> 121
<211> 443
<212> DNA
<213> artificial sequence

<220>
<223> chemically synthesized

<400> 121
gctagcgcca ccatggacat gagggtcccc gctcagctcc tggggctcct gctactctgg 60

ctccgaggtg ccagatgtga catcgagctc ctgcaggaat tcgatatcaa acgaactgtg 120

gctgcaccat ctgtcttcat cttcccgcca tctgatgagc agttgaaatc tggaactgcc 180

tctgttgtgt gcctgctgaa taacttctat cccagagagg ccaaagtaca gtggaaggtg 240

gataacgccc tccaatcggg taactcccag gagagtgtca cagagcagga cagcaaggac 300

agcacctaca gcctcagcag caccctgacg ctgagcaaag cagactacga gaaacacaaa 360

gtctacgcct gcgaagtcac ccatcagggc ctgagttcgc ccgtcacaaa gagcttcaac 420

aggggagagt gttaggttta aac 443

<210> 122
<211> 431
<212> DNA
<213> artificial sequence

<220>
<223> chemically synthesized

<400> 122
gctagcgcca ccatggcctg ggctctgctc ctcctcaccc tcctcactca gggcacaggg 60

tcctgggccc agtctgagct cctgcaggaa ttcgatatcc taggtcagcc caaggctgcc 120

ccctcggtca ctctgttccc gccctcctct gaggagcttc aagccaacaa ggccacactg 180

gtgtgtctca taagtgactt ctacccggga gccgtgacag tggcctggaa ggcagatagc 240

agccccgtca aggcgggagt ggagaccacc acaccctcca aacaaagcaa caacaagtac 300

gcggccagca gctatctgag cctgacgcct gagcagtgga agtcccacag aagctacagc 360

tgccaggtca cgcatgaagg gagcaccgtg gagaagacag tggcccctac agaatgttca 420

taggtttaaa c 431

<210> 123
<211> 21
<212> DNA
<213> artificial sequence

<220>
<223>  chemically synthesized

<400>  123
   agcgggggct tgccggccct g                                                    21


<210>  124
<211>  27
<212>  PRT
<213>  Hepatitis B virus Genotyp D

<400>  124

Pro Leu Gly Phe Phe Pro Asp His Gln Leu Asp Pro Ala Phe Arg Ala
1               5                   10                  15


Asn Thr Ala Asn Pro Asp Trp Asp Phe Asn Pro
            20                  25

## Description

### FIELD OF THE INVENTION

The present invention is related to the fields of vaccinology, monoclonal antibodies and medicine. The invention provides methods for generating and selecting a eukaryotic cell expressing and displaying on its surface an antibody, preferably a single chain monoclonal antibody (e.g. scFv) which is capable of specifically binding an antigen of interest. Said cell is selected from a populations of eukaryotic, preferably mammalian cells expressing a library of the variable regions of immunoglobulins derived from B-cells which were pre-selected for their specificity towards said antigen of interest. The variable regions of the antibody with the desired specificity can be (i) cloned from the selected eukaryotic cell, (ii) reassembled to a species specific, preferably to a fully human, recombinant monoclonal antibody (mAb), and (iii) produced in large scale by expression in vitro. Recombinant antibodies comprising said variable regions can be expressed in various forms, including scFv fusions, Fab fragments, and whole antibodies such as IgG, IgE, IgD, IgA and IgM. Monoclonal antibodies produced by the method of the invention may be used for research purposes, diagnostic purposes or the treatment of diseases.

### RELATED ART

Monoclonal antibodies (mAbs) have proven their usefulness as tools for a wide spectrum of research and diagnostic applications as well as in therapeutic applications. Monoclonal antibodies generated by the conventional hybridoma technology comprise mouse sequences, giving rise to an undesired immune response against the foreign sequence when administered to humans. Such an anti-immunoglobuline responses can interfere with therapy (Miller et al. 1983, Blood 62:988-995) or cause allergic or immune complex hypersensitivity (Ratner B., Allergy, Anaphylaxis and Immunotherapy, Basic Principles and Practice, Williams & Wilkins Company, Baltimore, 1943).

Humanized antibodies (GB 2188638 B, 1987; Riechmann et al. 1988 Nature 332:323-327; Foote and Winter 1992 Mol. Biol. 224:487-499) or fully human antibodies (Mendez 1997, Nat Genet. 15:146-156) are therefore becoming increasingly important for the treatment of a growing number of diseases, including cancer, heart disease, infection and immune disorders.

Given the usefulness of mAbs in general, and the enormous therapeutic and commercial potential of human mAbs in particular, a lot of effort has been put into the development of screening platforms allowing for the isolation of mAbs with predetermined selectivity.

The numerous strategies available for production of recombinant antibodies have been reviewed recently (Hoogenboom 2005, Nature Biotechnol. 23:1105-1116). In each case, a number of consecutive steps are involved: (1) cloning of the immunological diversity contained in the antibodies' variable regions by recombinant DNA technology; (2) expression of such antibody libraries using a suitable expression system, thereby coupling phenotype (i. e. the expressed antibody) with genotype (i. e. the nucleic acid encoding it); (3) application of an appropriate selective pressure, typically selection for binding to antigen; and (4) amplification of the selected antibody-encoding clones, leading to an enrichment of specific binders. Typically, antibody libraries are enriched by several such rounds of selection before individual clones are analyzed.

The most frequently used screening methods for the isolation of recombinant antibodies are phage display (Hogenboom 2002, Methods Mol. Biol. 178:1-37), ribosome/mRNA display (Lipovsek and Plückthun 2004, J. Immunol. Method 290:51-67) and microbial cell display (Boder and Wittrup 1997, Nat. Biotechnol. 15:553-557). While each of these screening platforms has its specific advantages, they share the same drawback: they are all based on expression of antibodies in an unnatural environment, namely in bacteria (phage display), in vitro in a test tube (ribosome/mRNA display), or in yeast (microbial cell display). It is important to remember that the chemical and physical properties of antibodies are very variable due to the sequence variability inherent to this class of proteins. Therefore, every screening method involving the expression of antibodies under such unnatural conditions is likely to lead to a strongly biased set of antibodies, by selecting not only for the desired binding properties, but also for chemical and physical properties advantageous under the respective screening conditions. In contrast, a selection platform based on the expression of antibodies in their natural environment, i. e. the secretory pathway of mammalian cells, ensures that all the cellular components normally involved in antibody synthesis and processing (folding, disulfide bond formation, glycosylation etc.) are available in a physiological form and concentration. Therefore, screening for antibodies in a mammalian expression/selection system is likely to yield a set of antibodies much less biased by properties other than binding to the desired antigen.

Currently, there are two reports of screening systems based on cell surface expression of antibodies in mammalian cells. One screening system is based on Vaccinia virus-mediated expression of whole antibodies in mammalian cells (US2002/0123057A1). With this method, antibody heavy and light chain libraries are expressed from separate vectors, by consecutive infection and transfection: the heavy chains are expressed in target cells using a high-titer vaccinia virus heavy chain library, such that each cell produces in average one heavy chain; the light chains are shortly after expressed in these infected cells by transfection of a light chain plasmid library. This leads to libraries of cells, each expressing one heavy chain paired with an undefined number of different light chains, which can be screened for binding to antigen. A second screening system based on cell surface expression of antibodies in mammalian cells has been described recently (Ho et al. 2006, Proc. Natl. Acad. Sci. USA 103:9637-9642). In this method, a scFv library is expressed in HEK 293T cells via transfection of plasmid DNA. This leads to pools of transfected cells expressing pools of scFv antibodies on their surface, which can be screened for binding to antigen. While only the enrichment of a high-affinity variant on an anti-CD22 scFv has been demonstrated, this method can in principle be used for the screening of all types of scFv libraries. Both of these methods profit from the above-mentioned advantages of a mammalian cell-based screening method. However, there are also some significant drawbacks to each of these methods. In case of the Vaccinia-based strategy, two separate libraries need to be constructed and transferred to target cells for expression and screening. In addition, the method initially selects only for a specific heavy chain, and the matching light chain has to be isolated in a second screen. Finally, similar to phage and ribosome/mRNA display, multiple selection rounds have to be carried out, both for the initial isolation of the heavy chain, as well as for the identification of the matching light chain. The scFv display method described by Ho et al. suffers from two main disadvantages. First, transfection is not the optimal method to introduce an antibody expression library into cells, since all transfection methods lead to the delivery of an undefined number of plasmid molecules to each cell. Thus, each transfected cell expresses an undefined number of different antibodies, further increasing the selective disadvantage of poorly expressed or otherwise problematic antibodies. Second, since the enrichment was reported to be only about 240-fold, also this method requires multiple rounds of selection to be carried out in order to isolate an antibody of interest from a complex library.

Here we describe a novel screening platform for the isolation human antibodies, that profits from all the advantages of a mammalian cell-based expression system, circumvents the disadvantages specific to the methods described above, and is completed in one single round of selection.

### SUMMARY OF THE INVENTION

The application provides a method of generating, selecting and isolating a cell expressing an antibody of desired specificity, preferably a monoclonal single chain antibody, most preferably a scFv. The application further provides methods which allow to clone the variable regions of said antibody from that isolated cell and to recombinantly produce antibodies comprising said variable regions as fusion protein with a purification tag, eg. as Fc-fusion, as Fab fragment, or as whole antibody, preferably as IgG1, IgG2 or IgG4. Said methods also allows to recombinantly produce antibodies with desired specificity in a fully species specific form, preferably as fully human antibodies.

Conventional selection of recombinant antibody libraries by phage display allows the screening of very large numbers (10¹²) of antibody clones in one single experiment. In contrast, screening of eukaryotic cell surface display libraries is limited to the screening of few million (10⁶ to 10⁷) antibody clones at a time but allows the expression and processing of antibodies in their natural environment. It is therefore advantageous to use antigen-specific B cells or B cell populations enriched in antigen-specific B cells for the generation of such libraries. This reduces the number of antibody clones that need to be screened and at the same time dramatically increases the fraction of functional antigen-specific combinations of heavy chain variable regions (HCVRs) and light chain variable regions (LCVRs) in said library.

Thus, one aspect of the invention is a method of isolating a cell expressing an antibody specifically binding an antigen of interest, said method comprising the steps of: (a) providing a population of B-cells; (b) selecting from said population of B-cells a sub-population of B-cells by selecting B-cells for their capability of specifically binding said antigen of interest; (c) generating an expression library, wherein each member of said expression library encodes an antibody comprising at least one variable region (VR), by (i) generating a multitude of DNA molecules, wherein said generating comprises the step of amplifying a pool of DNA molecules from said sub-population of B-cells, wherein each of said DNA molecules of said pool of DNA molecules encodes one of said at least one variable region (VR); and (ii) cloning said multitude of DNA molecules into an expression vector; (d) introducing said expression library into a first population of eukaryotic cells; (e) displaying antibodies of said expression library on the surface of said eukaryotic cells; and (f) isolating from said first population of eukaryotic cells a cell, wherein said cell is selected for the capability of the antibody displayed on its surface of specifically binding said antigen of interest or a fragment or antigenic determinant thereof.

One aspect of the invention is a method of isolating a cell expressing an antibody specifically binding an antigen of interest, said method comprising the steps of: (a) providing a population of B-cells; (b) selecting from said population of B-cells a sub-population of B-cells by selecting B-cells for their capability of specifically binding said antigen of interest; (c) generating an expression library, said generating comprising the steps of: (i) generating a multitude of DNA molecules encoding antibodies, said generating a multitude of DNA molecules comprising the steps of: (1) amplifying from said sub-population of B-cells a first pool of DNA molecules encoding HCVRs; (2) amplifying from said sub-population of B-cells a second pool of DNA molecules encoding LCVRs; and (3) linking specimens of said first and of said second pool of DNA molecules to each other by a DNA encoding a linker region (LR); (ii) cloning a specimen of said multitude of DNA molecules into an expression vector; wherein each member of said expression library encodes an antibody comprising a signal peptide, a HCVR, a LCVR and a transmembrane region, wherein said HCVR and said LCVR are linked to each other by said linker region; (d) introducing said expression library into a first population of eukaryotic cells; (e) displaying antibodies of said expression library on the surface of said eukaryotic cells; and (f) isolating from said first population of eukaryotic cells a cell, wherein said cell is selected for the capability of the antibody displayed on its surface of specifically binding said antigen of interest or a fragment or antigenic determinant thereof.

A further aspect of the invention is a method of isolating a cell expressing an antibody specifically binding an antigen of interest, said method comprising the steps of: (a) providing a population of B-cells; (b) selecting from said population of B-cells a sub-population of B-cells by selecting B-cells for their capability of specifically binding said antigen of interest; (c) generating an expression library, said generating comprising the steps of: (i) generating a multitude of DNA molecules encoding antibodies, said generating comprising the steps of: (1) isolating RNA from said sub-population of B-cells; (2) transcribing said RNA to cDNA; (3) amplifying from said cDNA said first pool of DNA molecules using a first mixture of oligonucleotides comprising at least two oligonucleotides capable of amplifying HCVR coding regions; (4) amplifying from said cDNA said second pool of DNA molecules using a second mixture of oligonucleotides comprising at least two oligonucleotides capable of amplifying LCVR coding regions; and (5) linking specimens of said first and said second pool of DNA molecules to each other by a DNA encoding said linker region; (ii) cloning a specimen of said multitude of DNA molecules into an expression vector; wherein each member of said expression library encodes an antibody comprising a signal peptide, a HCVR, a LCVR and a transmembrane region, wherein said HCVR and said LCVR are linked to each other by said linker region; (d) introducing said expression library into a first population of eukaryotic cells; (e) displaying antibodies of said expression library on the surface of said eukaryotic cells; and (f) isolating from said first population of eukaryotic cells a cell, wherein said cell is selected for the capability of the antibody displayed on its surface of specifically binding said antigen of interest or a fragment or antigenic determinant thereof.

A further aspect of the invention is a method of producing an antibody specifically binding an antigen of interest, said method comprising the steps of: (a) isolating a cell expressing an antibody according to the method above; (b) obtaining RNA from said isolated cell; (c) synthesizing cDNA encoding said antibody from said RNA; (d) cloning said cDNA into an expression vector; (e) generating a fusion construct encoding a fusion product comprising said antibody and said purification tag; (e) expressing said fusion product in a cell; and (f) purifying said fusion product.

A further aspect of the invention is a method of producing an antibody specifically binding an antigen of interest, said method comprising the steps of: (a) isolating a cell expressing an antibody according to the method above; (b) obtaining RNA from said cell; (c) synthesizing cDNA form said RNA; (d) amplifying from said cDNA a DNA encoding VRs of said antibody expressed by said cell; (e) generating an expression construct comprising said DNA, wherein said expression construct is encoding at least one VR of said antibody expressed by said cell; (h) expressing said expression construct in a cell.

A further aspect of the invention is an expression vector for displaying polypeptides, preferably antibodies on the surface of a eukaryotic cell. The invention thus relates to an expression vector, preferably an alphaviral expression vector, wherein said expression vector comprises DNA elements encoding a signal peptide, a transmembrane region and, preferably, a detection tag, and wherein further preferably said expression vector comprises a restriction site allowing the cloning, preferably the orientation specific cloning, of DNA molecules encoding said polypeptides, preferably said antibody variable regions, into said expression vector.

A further aspect of the invention is an expression library comprising said expression vector.

A further aspect of the invention is a eukaryotic cell comprising said expression vector or comprising at least one specimen of said expression library.

### DETAILED DESCRIPTION OF THE INVENTION

**"Animal":** As used herein, the term "animal" refers to any organism comprising an immune system capable of producing antibodies. Preferred animals in the context of the invention are fish, amphibians, birds, reptiles, and mammals, preferably artiodactyls, rodents and primates. In a preferred embodiment said animal is selected from the group consisting of sheep, elk, deer, donkey, mule deer, mink, horse, cattle, pig, goat, dog, cat, rat, hamster, guinea pig, and mouse. In a further preferred embodiment said animal is a mouse, a rat or, most preferably, a primate. In a further preferred embodiment said animal is a non-human primate or a human, most preferably a human. In a further preferred embodiment said animal is a humanized mouse, e.g. as described as a source for humanized antibodies in (Lonberg (2005), Nature Biotechnology 23(9):1117-1125). In a further preferred embodiment the animal is a humanized mouse or a human, preferably a human.

**"Antibody":** As used herein, the term "antibody" refers to a molecule, preferably a protein, which is capable of specifically binding an antigen, typically and preferably by binding an epitope or antigenic determinant or said antigen. The term antibody refers to whole antibodies, preferably of the IgG, IgA, IgE, IgM, or IgD class, more preferably of the IgG class, most preferably IgG1, IgG2, IgG3, and IgG4, and antigen-binding fragments thereof, including single chain antibodies, wherein further preferably said whole antibodies comprise either a kappa or a lambda light chain. The term "antibody" also refers to antigen binding antibody fragments, preferably to proteolytic fragments and their recombinant analogues, most preferably to Fab, Fab' and F(ab')2, Fd, and Fv. The term "antibody" further encompasses proteins comprising at least one, preferably two variable regions. Preferred antibodies are single chain antibodies, preferably scFvs, disulfide-linked Fvs (sdFv) and fragments comprising either a light chain variable region (LCVR) or a heavy chain variable region (HCVR). In the context of the invention the term "antibody" also refers to recombinant antibodies, preferably to recombinant proteins consisting of a single polypeptide, wherein said polypeptide comprises at least one variable region, preferably two variable regions, most preferably at least one, preferably one, HCVR and at least one, preferably one LCVR. In the context of the invention recombinant antibodies may further comprise functional elements, such as, for example, a linker region, a transmembrane region, a signal peptide or hydrophobic leader sequence, a detection tag and/or a purification tag.

**"Fv":** The term Fv refers to the smallest proteolytic fragment of an antibody capable of binding an antigen and to recombinant analogues of said fragment.

**"single chain antibody":** A single chain antibody is an antibody consisting of a single polypeptide. Preferred single chain antibodies consist of a polypeptide comprising a single VR, preferably a single HCVR. More preferred single chain antibodies are scFv, wherein said scFv consist of a single polypeptide comprising exactly one HCVR and exactly one LCVR, wherein said HCVR and said LCVR are linked to each other by a linker region, wherein preferably said linker region consists of at least 15, preferably of 15 to 20 amino acids (Bird et al. (1988) Science, 242(4877):423-426). Further preferred single chain antibodies are scFv, wherein said scFv are encoded by a coding region, wherein said coding region, in 5' to 3' direction, comprises in the following order: (1) a light chain variable region (LCVR) consisting of light chain framework (LFR) 1, complementary determining region (LCDR) 1, LFR 2, LCDR 2, LFR3, LCDR3 and LFR4 from a κ or λ light chain; (2) a flexible linker (L), and (3) a heavy chain variable region (HCVR) consisting of framework (HFR) 1, complementary determining region (HCDR) 1, HFR 2, HCDR 2, HFR3, HCDR3 and HFR4. Alternatively, single chain antibodies are scFv, wherein said scFv are encoded by a coding region, wherein said coding region, in 5' to 3' direction, comprises in the following order: (1) a heavy chain variable region (HCVR) consisting of framework (HFR) 1, complementary determining region (HCDR) 1, HFR 2, HCDR 2, HFR3, HCDR3 and HFR4; (2) a flexible linker (L), and (3) a light chain variable region (LCVR) consisting of light chain framework (LFR) 1, complementary determining region (LCDR) 1, LFR 2, LCDR 2, LFR3, LCDR3 and LFR4 from a κ or λ light chain.

"**diabody**": The term "diabody" refers to an antibody comprising two polypeptide chains, preferably two identical polypeptide chains, wherein each polypeptide chain comprises a HCVR and a LCVR, wherein said HCVR and said LCVR are linked to each other by a linker region, wherein preferably said linker region comprises at most 10 amino acids (Huston et al. (1988), PNAS 85(16):587958-83; Holliger et al. (1993), PNAS 90(14):6444-6448, Hollinger & Hudson, 2005, Nature Biotechnology 23(9):1126-1136; Arndt et al. (2004) FEBS Letters 578(3):257-261). Preferred linker regions of diabodies comprise 0, 1, 2, 3, 4, 5, 6, 7, 8, 9, or 10 amino acids.

**"species specific antibody":** The term "species specific antibody" refers to an antibody, preferably to a recombinant antibody, comprising variable and preferably also constant regions of only one single animal species. Preferred species specific antibodies are mouse antibodies, rat antibodies and human antibodies, most preferably human antibodies.

**"human antibodies":** As used herein, the term "human antibody" refers to an antibody, preferably a recombinant antibody, essentially having the amino acid sequence of a human immunoglobulin, or a fragment thereof, and includes antibodies isolated from human immunoglobulin libraries. In the context of the invention "human antibodies" may comprise a limited number of amino acid exchanges as compared to the sequence of a native human antibody. Such amino acid exchanges can, for example, be caused by cloning procedures. However, the number of such amino acid exchanges in human antibodies of the invention is preferably minimized; most preferably, the amino acid sequence of human antibodies is at least 95 %, more preferably at least 96 %, still more preferably 97 %, still more preferably 98 %, still more preferably 99 % and most preferably 100 % identical to that of native human antibodies. Preferred recombinant human antibodies differ from native human antibodies in at most 20, 15, 10, 9, 8, 7, 6, 5, 4, 3, 2, or 1 amino acid. Very preferably, differences in the amino acid sequence of recombinant human antibodies and native human antibodies are eliminated my means of molecular cloning, and thus, most preferably, the amino acid sequence of recombinant human antibodies and native human antibodies are identical. Such antibodies are also referred to as "fully human antibodies".

Preferred recombinant human antibodies comprise at least one, preferably one, heavy chain variable region and at least one, preferably one, heavy chain constant region, wherein said at least one heavy chain variable region is at least 95 %, more preferably at least 96 %, still more preferably 97 %, still more preferably 98 %, still more preferably 99 % and most preferably 100 % identical to a native human heavy chain variable region; and wherein said at least one heavy chain constant region is at least 95 %, more preferably at least 96 %, still more preferably 97 %, still more preferably 98 %, still more preferably 99 % and most preferably 100 % identical to a native human heavy chain constant region.

Further preferred recombinant human antibodies comprise at least one, preferably one, light chain variable region and at least one, preferably one, light chain constant region, wherein said at least one light chain variable region is at least 95 %, more preferably at least 96 %, still more preferably 97 %, still more preferably 98 %, still more preferably 99 % and most preferably 100 % identical to a native human light chain variable region; and wherein said at least one light chain constant region is at least 95 %, more preferably at least 96 %, still more preferably 97 %, still more preferably 98 %, still more preferably 99 % and most preferably 100 % identical to a native human light chain constant region.

Preferred human antibodies comprise least one, preferably one, heavy chain variable region and at least one, preferably one, heavy chain constant region, at least one, preferably one, light chain variable region and at least one, preferably one, light chain constant region, wherein said at least one light chain variable region is at least 95 %, more preferably at least 96 %, still more preferably 97 %, still more preferably 98 %, still more preferably 99 % and most preferably 100 % identical to a native human light chain variable region; and wherein said at least one heavy chain variable region, said at least one heavy chain constant region, said at least one light chain constant region and said at least one light chain constant region is at least 95 %, more preferably at least 96 %, still more preferably 97 %, still more preferably 98 %, still more preferably 99 % and most preferably 100 % identical to the respective native human regions.

**"humanized antibodies":** As used herein, the term "humanized antibody" refers to antibodies wherein the antigen-binding parts of the antibody are derived from a non-human species and the remaining parts of the humanized antibody comprise or preferably entirely consist of a human amino acid sequence. The generation of humanized antibodies is within the skill of the artisan. The basic technology for the generation of humanized antibodies is, for example, disclosed in GB 2188638 B, Riechmann et al. (1988) Nature 332:323-327, and Foote and Winter (1992) Mol. Biol. 224:487-499. Preferred humanized antibodies are mouse antibodies, wherein the constant regions, more preferably the constant regions and the VR framework regions are exchanged by the corresponding human sequences ("CDR grafting").

**"monoclonal antibody":** As used herein, the term "monoclonal antibody" refers to an antibody population comprising only one single antibody species, i.e. antibodies having an identical amino acid sequence.

"constant region (CR)": The term "constant region" refers to a light chain constant region (LCCR) or a heavy chain constant region (HCCR) of an antibody. Typically and preferably, said CR comprises one to four immunoglobulin domains characterized by disulfide stabilized loop structures. Preferred CRs are CRs, preferably kappa CRs or lambda CRs, of immunoglobulins, preferably of human immunoglobulins, wherein further preferably said immunoglobulins, preferably said human immunoglobulins, are selected from the group consisting of IgG1, IgG2, IgG3, IgG4, IgA, IgE, IgM, and IgD. Very preferred CRs are human CRs comprising or consisting of an amino acid sequence available from public databases, including, for example the Immunogenetic Information System (http://imgt.cines.fr/).

**light chain constant region (LCCR):** The LCCR, more specifically the kappa LCCR or the lambda LCCR, typically represents the C-terminal half of a native kappa or lambda light chain of an native antibody. A LCCR typically comprises about 110 amino acids representing one immunoglobulin domain.

**heavy chain constant region (HCCR):** The constant region of a heavy chain comprises about three quarters or more of the heavy chain of an antibody and is situated at its C-terminus. Typically the HCCR comprises either three or four immunoglobulin domains.

**"variable region (VR)"**: Refers to the variable region or variable domain of an antibody, more specifically to the heavy chain variable region (HCVR) or to the light chain variable region (LCVR). Typically and preferably, a VR comprises a single immunoglobulin domain. Preferred VRs are VRs of immunoglobulins, preferably of human immunoglobulins, wherein further preferably said immunoglobulins, preferably said human immunoglobulins, are selected from the group consisting of IgG1, IgG2, IgG3, IgG4, IgA, IgE, IgM, and IgD. VRs of various species are known in the art. Preferred VRs are human VRs, wherein said human VRs exhibit at least 80 %, preferably at least 90 %, more preferably at least 95 %, most preferably at least 99 % sequence identity with any known human VR sequence, preferably with any human VR sequence available from public databases, most preferably with any human VR available from the Immunogenetics Information System (http://imgt.cines.fr/).

**"light chain variable region (LCVR)"**: Light chain variable regions are encoded by rearranged nucleic acid molecules and are either a kappa LCVR or a lambda LCVR. In the context of the invention preferred kappa LCVRs are human kappa LCVRs, preferably human kappa LCVRs which are encoded by a DNA which can be amplified from human B cells using a primer combination of any one of SEQ ID NO:49 to 52 with any one of SEQ ID NO:53 to 56, and further preferably, PCR conditions described in Example 3.

In the context of the invention preferred lambda LCVRs are human lambda LCVRs, preferably human lambda LCVRs which are encoded by a DNA which can be amplified from human B cells using a primer combination of any one of SEQ ID NO:57 to 65 with any one of SEQ ID NO:66 to 68, and further preferably, PCR conditions described in Example 3.

**"heavy chain variable region (HCVR)"**: Heavy chain variable regions are encoded by rearranged nucleic acid molecules. In the context of the invention preferred HCVRs are human HCVRs, preferably human HCVRs which are encoded by a DNA which can be amplified from human B cells using a primer combination of any one of SEQ ID NO:42 to 47 with SEQ ID NO:48 and, further preferably, PCR conditions described in Example 3.

**"antibody coding region":** As used herein, the term "antibody coding region" refers to any DNA encoding an antibody or an element thereof. Preferably, "antibody coding regions" refers to a DNA encoding a CR, preferably a HCCR or LCCR, or a VR, preferably a HCVR or a LCVR, of an antibody. Very preferred antibody coding regions are DNA fragments representing human antibody coding regions, preferably human VR coding regions, most preferably human VR coding regions which can be amplified from human B cells using any combination of primers of SEQ ID NO:42 to 68 and, further preferably, PCR conditions described in Example 3.

Preferred antibody coding regions are human kappa LCVR coding regions, preferably human kappa LCVR coding regions which can be amplified from human B cells using a primer combination of any one of SEQ ID NO:49 to 52 with any one of SEQ ID NO:53 to 56, and further preferably, PCR conditions described in Example 3.

Further preferred antibody coding regions are human lambda LCVR coding regions, preferably human lambda LCVR coding regions which can be amplified from human B cells using a primer combination of any one of SEQ ID NO:57 to 65 with any one of SEQ ID NO:66 to 68, and further preferably, PCR conditions described in Example 3.

Further preferred antibody coding regions are human HCVR coding regions, preferably human HCVR coding regions which can be amplified from human B cells using a primer combination of any one of SEQ ID NO:42 to 47 with SEQ ID NO:48 and, further preferably, PCR conditions described in Example 3.

**"antigen":** As used herein, the term "antigen" refers to a molecule which is bound by an antibody. An antigen is recognized by the immune system and/or by a humoral immune response and can have one or more epitopes, preferably B-cell epitopes, or antigenic determinants.

**"antigen of interest":** The application provides methods for the selection of cells expressing antibodies with a desired specificity and to methods of producing such antibodies, i.e. the antibodies of the invention are capable of binding an antigen of interest. Typically and preferably said antigen of interest is a protein or a haptene. The antigen of interest is preferably selected from the group consisting of (a) antigen of a microorganism or of a pathogen, (b) tumor antigen, (c) self antigen, and (d) allergen.

**"fragment of the antigen of interest":** The term "fragment of an antigen of interest" refers to a fragment of an antigen, preferably of a polypeptide, comprising at least one antigenic determinant of said antigen. In a preferred embodiment a fragment of the antigen of interest is a polypeptide consisting of a stretch, preferably a consecutive stretch, of amino acids derived from said antigen of interest, wherein said polypeptide can be bound by an antibody. Typically and preferably, said fragment comprises at least 80, preferably at least 90, more preferably at least 95, still more preferably at least 99 and most preferably 100 % sequence identity with said antigen of interest. Typically and preferably, a fragment of the antigen of interest is a polypeptide consisting of 6 to 1000, preferably 6 to 500, more preferably 6 to 300, still more preferably 6 to 200, still more preferably 6 to 100 amino acids. Very preferred are fragments consisting of about 10, 20, 30, 40, 50, 60, 70, 80, 90 or 100 amino acids.

**"antigen of a microorganisms or pathogen":** An antigen of a microorganism or pathogen preferably is an antigen of infectious virus, infectious bacteria, parasites or infectious fungi. Such antigens include the intact microorganism or pathogen as well as natural isolates and fragments or derivatives thereof and also synthetic or recombinant compounds which are identical to or similar to natural microorganism antigens and induce an immune response specific for that microorganism. A compound is similar to an antigen of a microorganism or pathogen if it induces an immune response (humoral and/or cellular) to a natural microorganism antigen. Examples of infectious viruses, bacteria, and infectious fungi that are microbial antigen as used herein, are described in WO03/024481 (page 23 last paragraph to page 25 third paragraph), the disclosure of which is incorporated herein by reference.

**"tumor antigen":** A tumor antigen is a compound, such as a peptide, associated with a tumor or cancer and which can be bound by an antibody. Tumor antigens can be prepared from cancer cells either by preparing crude extracts of cancer cells, for example, as described in Cohen, et al., Cancer Research, 54:1055 (1994), by partially purifying the antigens, by recombinant technology or by de novo synthesis of known antigens. Tumor antigens include antigens that are antigenic portions of or are a whole tumor or cancer polypeptide. Such antigens can be isolated or prepared recombinantly or by any other means known in the art. Cancers or tumors include, but are not limited to, biliary tract cancer; brain cancer; breast cancer; cervical cancer; choriocarcinoma; colon cancer; endometrial cancer; esophageal cancer; gastric cancer; intraepithelial neoplasms; lymphomas; liver cancer; lung cancer (e.g. small cell and non-small cell); melanoma; neuroblastomas; oral cancer; ovarian cancer; pancreas cancer; prostate cancer; rectal cancer; sarcomas; skin cancer; testicular cancer; thyroid cancer; and renal cancer, as well as other carcinomas and sarcomas.

**"self antigen":** As used herein, the term "self antigen" refers, with respect to an animal, to proteins encoded by the DNA of said animal and products generated by proteins or RNA encoded by the DNA of said animal. Preferably, the tem "self antigen", as used herein, refers to proteins encoded by the human genome or DNA and products generated by proteins or RNA encoded by the human genome or DNA are defined as self. In one embodiment, self antigens are proteins that result from a combination of two or more self-molecules or fragments of self-molecules and proteins that have a sequence identity of at least 95 %, preferably at least 97 %, more preferably at least 99 % are also considered to be self antigens.

**"Allergens"**: The term "allergen", as used herein, also encompasses "allergen extracts" and "allergenic epitopes" which are capable of inducing an allergic reaction of the immune system of an animal. Preferred allergens are pollen (e.g. grass, ragweed, birch and mountain cedar); house dust and dust mites; mammalian epidermal allergens and animal danders; mold and fungus; insect bodies and insect venom; feathers; food; and drugs (e.g., penicillin).

**"antigenic determinant":** As used herein, the term "antigenic determinant" is meant to refer to that portion of an antigen that is specifically recognized by B-lymphocytes. B-lymphocytes respond to foreign antigenic determinants by antibody production.

**"specifically binding" (antibody / antigen):** The specificity of an antibody relates to the antibody's capability of specifically binding an antigen. The specificity of this interaction between the antibody and the antigen (affinity) is characterized by a binding constant or, inversly, by a dissociation constant (Kd). It is to be understood that the apparent affinity of an antibody to an antigen in a multivalent interaction depends on the structure of the antibody and of the antigen, and on the actual assay conditions. The apparent affinity of an antibody to an antigen in a multivalent interaction may be significantly higher than in a monovalent interaction due to avidity. Thus, affinity is preferably determined under conditions favouring monovalent interactions. Kd can be determined by methods known in the art. Kd of a given combination of antibody and antigen is preferably determined by ELISA, most preferably by an ELISA essentially as described in Example 7, wherein a constant amount of immobilized antigen is contacted with a serial dilution of a known concentration of a purified antibody, preferably a monovalent antibody, for example scFv or Fab fragment. Kd is then determined as the concentration of the antibody where half-maximal binding is observed. Alternatively, Kd of a monovalent interaction of an antibody and an antigen is determined by Biacore analysis as the ratio of on rate (kₒₙ) and off rate (k_{off}.). Lower values of Kd indicate a more specific binding of the antibody to the antigen than higher values. In the context of the application, an antibody is considered to be "specifically binding an antigen (of interest)", when the dissociation constant (Kd), preferably determined as described above, and further preferably determined in a monovalent interaction, is at most 1 mM (<= 10⁻³ M), preferably at most 1 µM (<= 10⁻⁶M), most preferably at most 1 nM (<= 10⁻⁹M). Very preferred are antibodies capable of binding an antigen with a Kd of less than 1 nM (< 10⁻⁹ M, "subnanomolar"), wherein further preferably Kd is determined in a monovalent interaction. Further preferred antibodies are capable of binding an antigen with a Kd of 0.01 to 10 nM, more preferably of 0.01 to 5 nM, still more preferably of 0.01 to 3 nM, most preferably of 0.1 to 2 nM, wherein further preferably Kd is determined in a monovalent interaction.

**"specifically binding"(antibody displayed on a cell / antigen):** With respect to an antibody displayed on a mammalian cell the specificity of the binding of an antigen is preferably determined in an fluorescence assay essentially as set forth herein in Example 4, wherein the intensity of a fluorescence signal is correlated with the amount of antigen bound by a cell displaying said antibody. Antibodies displayed on mammalian cells are regarded as specifically binding an antigen, when the intensity of the fluorescence signal is higher than the signal detected for control cells. Preferably, said signal is at least two times higher than that of control cells.

**"B-cell"**: As used herein, the term "B-cell" refers to a cell produced in the bone marrow of an animal expressing membrane-bound antibody specific for an antigen. Following interaction with the antigen it differentiates into a plasma cell producing antibodies specific for the antigen or into a memory B-cell.

**"Antigen-specific B cell"**: As used herein, the term "antigen-specific B cell" refers to a B cell which expresses antibodies that are able to distinguish between the antigen of interest and other antigens and which specifically bind to that antigen of interest with high or low affinity but which do not bind to other antigens.

**"Memory B-cell":** As used herein, "memory B-cell" refers to a B-cell sub-type that is formed following a primary contact with the antigen of interest. When a B-cell is activated, by specifically recognizing the antigen of interest, it proliferates to form antibody producing plasma cells and long-lived memory B-cells. These memory B-cells are specific for the antigen of interest that stimulated their production. If this antigen of interest is encountered again, memory B cells can recognize it and quickly proliferate.

**"immunizing":** As used herein the term immunizing means administering to an animal the antigen of interest, a fragment or antigenic determinant thereof, preferably together with an adjuvant in a dose capable of inducing a detectable immune response, preferably a B-cell response.

**"Tag":** The term tag, preferably a purification or detection tag, refers to a polypeptide segment that can be attached to a second polypeptide to provide for purification or detection of the second polypeptide or provides sites for attachment of the second polypeptide to a substrate. In principle, any peptide or protein for which an antibody or other specific binding agent is available can be used as an affinity tag. Tags include haemagglutinin tag, myc tag, poly-histidine tag, protein A, glutathione S transferase, Glu-Glu affinity tag, substance P, FLAG peptide, streptavidine binding peptide, or other antigenic epitope or binding domain (mostly taken from US06686168).

**"expression library"**: The term expression library refers to a multitude of expression vectors of the same type, wherein individual expression vectors expresses a different polypeptide, e.g. a different antibody. Preferred expression libraries are viral expression libraries, most preferably alpha viral expression libraries. Very preferred expression libraries are Sindbis-based libraries as described, for example, in WO1999/025876A1.

**"Multiplicity of infection (MOI)"**: The term multiplicity of infection refers to the ratio between the number of infectious virus particles in a viral, preferably alphaviral, expression library and the number of cells exposed to the virus.

The application provides a method of generating, selecting and isolating a cell expressing an antibody of desired specificity. In more detail, the application provides a method of isolating A method of isolating a cell expressing an antibody specifically binding an antigen of interest, said method comprising the steps of: (a) providing a population of B-cells; (b) selecting from said population of B-cells a sub-population of B-cells by selecting B-cells for their capability of specifically binding said antigen of interest; (c) generating an expression library, wherein each member of said expression library encodes an antibody comprising at least one variable region (VR), by (i) generating a multitude of DNA molecules, wherein said generating comprises the step of amplifying a pool of DNA molecules from said sub-population of B-cells, wherein each of said DNA molecules of said pool of DNA molecules encodes one of said at least one variable region (VR); and (ii) cloning a specimen of said multitude of DNA molecules into an expression vector; (d) introducing said expression library into a first population of eukaryotic cells; (e) displaying antibodies of said expression library on the surface of said eukaryotic cells; and (f) isolating from said first population of eukaryotic cells a cell, wherein said cell is selected for the capability of the antibody displayed on its surface of specifically binding said antigen of interest or a fragment or antigenic determinant thereof.

Furthermore, the application provides for a method of isolating a cell expressing an antibody specifically binding an antigen of interest, said method comprising the steps of: (a) providing a population of B-cells; (b) selecting from said population of B-cells a sub-population of B-cells by selecting B-cells for their capability of specifically binding said antigen of interest; (c) generating an expression library, said generating comprising the steps of: (i) generating a multitude of DNA molecules encoding antibodies, said generating a multitude of DNA molecules comprising the steps of: (1) amplifying from said sub-population of B-cells a first pool of DNA molecules encoding HCVRs; (2) amplifying from said sub-population of B-cells a second pool of DNA molecules encoding LCVRs; and (3) linking specimens of said first and of said second pool of DNA molecules to each other by a DNA encoding a linker region (LR); (ii) cloning said multitude of DNA molecules into an expression vector; wherein each member of said expression library encodes an antibody comprising a signal peptide, a HCVR, a LCVR and a transmembrane region, wherein said HCVR and said LCVR are linked to each other by said linker region; (d) introducing said expression library into a first population of eukaryotic cells; (e) displaying antibodies of said expression library on the surface of said eukaryotic cells; and (f) isolating from said first population of eukaryotic cells a cell, wherein said cell is selected for the capability of the antibody displayed on its surface of specifically binding said antigen of interest or a fragment or antigenic determinant thereof.

Moreover, the application provides for method of isolating a cell expressing an antibody specifically binding an antigen of interest, said method comprising the steps of: (a) providing a population of B-cells; (b) selecting from said population of B-cells a sub-population of B-cells by selecting B-cells for their capability of specifically binding said antigen of interest; (c) generating an expression library, said generating comprising the steps of: (i) generating a multitude of DNA molecules encoding antibodies, said generating comprising the steps of: (1) isolating RNA from said sub-population of B-cells; (2) transcribing said RNA to cDNA; (3) amplifying from said cDNA said first pool of DNA molecules using a first mixture of oligonucleotides comprising at least two oligonucleotides capable of amplifying HCVR coding regions; (4) amplifying from said cDNA said second pool of DNA molecules using a second mixture of oligonucleotides comprising at least two oligonucleotides capable of amplifying LCVR coding regions; and (5) linking specimens of said first and said second pool of DNA molecules to each other by a DNA encoding said linker region; (ii) cloning said multitude of DNA molecules into an expression vector; wherein each member of said expression library encodes an antibody comprising a signal peptide, a HCVR, a LCVR and a transmembrane region, wherein said HCVR and said LCVR are linked to each other by said linker region; (d) introducing said expression library into a first population of eukaryotic cells; (e) displaying antibodies of said expression library on the surface of said eukaryotic cells; and (f) isolating from said first population of eukaryotic cells a cell, wherein said cell is selected for the capability of the antibody displayed on its surface of specifically binding said antigen of interest or a fragment or antigenic determinant thereof.

In a preferred embodiment each antibody encoded by said expression library further comprises a signal peptide and a transmembrane region.

In a further preferred embodiment said antibody specifically binding said antigen of interest is a humanized or human antibody, preferably a human antibody. In a further preferred embodiment said antibody specifically binding said antigen of interest is a single chain antibody, preferably a scFv. Thus, the antibody displayed on the surface of said cell is preferably expressed as a scFv comprising a transmembrane region. In a preferred embodiment said at least one VR comprised by said antibody is a heavy chain variable region (HCVR) and alight chain variable region (LCVR). Thus, in a further preferred embodiment each member of said expression library encodes an antibody, wherein said antibody is expressed as fusion protein consisting of a single polypeptide, wherein said polypeptide comprises a signal peptide, a HCVR, a LCVR, and a transmembrane region. Typically and preferably, cDNA encoding variable regions is synthesized from RNA obtained from said sub-population of antigen specific B-cells, cloned and expressed in an expression vector, wherein the variability of antigen-specific antibodies is increased by randomly linking different light and heavy chain variable regions. This is achieved by separately amplifying DNA molecules encoding HCVRs and LCVRs and linking them together by a DNA encoding a linker region (LR). Therefore, in a preferred embodiment said generating an expression library comprises the steps of: (a) generating a multitude of DNA molecules encoding antibodies, said generating comprising the steps of: (i) amplifying from said sub-population of B-cells a first pool of DNA molecules encoding HCVRs; (ii) amplifying from said sub-population of B-cells a second pool of DNA molecules encoding LCVRs; and (iii) linking specimens of said first and of said second pool of DNA molecules to each other by a DNA encoding a linker region (LR); (b) cloning a specimen of said multitude of DNA molecules into an expression vector; wherein each member of said expression library encodes an antibody comprising a signal peptide, a HCVR, a LCVR and a transmembrane region, wherein said HCVR and said LCVR are linked to each other by said linker region.

In a further preferred embodiment said generating a multitude of DNA molecules comprises the steps of: (a) isolating RNA from said sub-population of B-cells; (b) transcribing said RNA to cDNA; and (c) amplifying from said cDNA a pool of DNA molecules using a mixture of oligonucleotides comprising at least two oligonucleotides capable of amplifying VR coding regions.

In a further preferred embodiment said generating a multitude of DNA molecules comprises the steps of: (a) isolating RNA from said sub-population of B-cells; (b) transcribing said RNA to cDNA; (c) amplifying from said cDNA said first pool of DNA molecules using a first mixture of oligonucleotides comprising at least two oligonucleotides capable of amplifying HCVR coding regions; (d) amplifying from said cDNA said second pool of DNA molecules using a second mixture of oligonucleotides comprising at least two oligonucleotides capable of amplifying LCVR coding regions; and (e) linking specimens of said first and said second pool of DNA molecules to each other by a DNA encoding said linker region (LR). In a preferred embodiment the order of these elements from N- to C-terminus of said antibody is LCVR-LR-HCVR or HCVR-LR-LCVR, most preferably said order is HCVR-LR-LCVR.

The cloning of variable regions is a standard procedure generally known in the art and has been described for various species, including humans, non-human primates, mouse, rabbit, and chicken. For review see Barbas III et al. (eds.), Phage Display - A Laboratory manual, Cold Spring Habour Press, 2001, in particular the chapter Andris-Widhopf et al., Generation of Antibody Libraries: PCR Amplification and Assembly of Light- and Heavy-chain Coding Sequences, therein. Andris-Widhopf et al. discloses sequences of oligonucleotides capable of amplifying variable region coding regions (VR coding regions), preferably HCVR coding regions or LCVR coding regions, of the afore mentioned species which sequences are incorporated herein by reference. Furthermore, oligonucleotides capable of amplifying HCVR coding regions or LCVR coding regions, preferably human HCVR coding regions or LCVR coding regions, can be designed by the artisan by comparing known sequences of antibody coding regions which are available from databases such as, for example, Immunogenetics (http://imgt.cines.fr/), Kabat (www.kabatdatabase.com), and Vbase (http://vbase.mrc-cpe.cam.ac.uk/), and by identifying consensus sequences suitable for primer design. Based on general knowledge in molecular biology, on the afore mentioned manual (Barbas III et al. (eds.) Phage Display - A Laboratory manual, Cold Spring Habour Press, 2001) and the references cited therein, the artisan is able to design oligonucleotides capable of amplifying HCVR coding regions or LCVR coding regions, wherein preferably said primers comprise suitable restriction sites for the cloning of the amplified products and wherein preferably said oligonucleotides also encode said linker region. Further Strategies for amplifying and cloning VRs are described in Sblattero and Bradbury (1998) Immunotechnology 3:271-278 and Weitkamp et al. (2003), J. Immunol. Meth. 275:223-237.

Preferred oligonucleotides encode restriction sites (RS1 and RS2) to allow for cloning of the assembled coding regions in the orientation RS1-LCVR-LR-HCVR-RS2 or RS1-HCVR-LR-LCVR-RS2, preferably RS1-LCVR-LR-HCVR-RS2. In a preferred embodiment, said restriction sites are distinct from one another and at least one of them generates a single-stranded overhang ("sticky end"), thus allowing for directional cloning. More preferably, said RS are eight or more base pairs long and recognized by "rare cutting" restriction enzymes selected from but not limited to the list of Asc1, Fse1, Not1, Pac1, Pme1, Sfi1 and Swa1. Most preferably, the RS are recognition sequences for Sfi1 (which cuts the sequence 5'-GGCCNNNNNGGCC-3'), and the sequences of RS1 and RS2 are, respectively, 5'-GGCCCAGGCGGCC-3' and 5'-GGCCAGGCCGGCC-3'. Primers suitable for the generation of libraries of scFv cDNAs of the format Sfil-LCVR-GGSSRSSSSGGGGSGGGG-HCVR-Sfil have been described (Barbas, C.F., III, Burton, D. R., Scott, J. K. and Silverman, G. J. (2001) Phage Display. A Laboratory Manual. Cold Spring Harbor Laboratory Press, 9.24-9.26) and are listed below.

The human HCVR, human kappa LCVR and human lambda LCVR coding regions are amplified by PCR with mixtures of specific sense and antisense primers annealing in the framework 1 and 4 regions, respectively. The principal set of primers is described here: Sblattero D, Bradbury A. (1998) A definitive set of oligonucleotide primers for amplifying human V regions. Immunotechnology. 3, 271-278. As an alternative to the use of a specific mix of antisense primers for the amplification of HCVR, kappa LCVR and lambda LCVR coding sequences, one antisense primer annealing in the gamma, kappa and lambda constant region can be used, respectively.

It has surprisingly been found that the efficiency of the subsequent cloning of specific VR coding regions can be enhanced by pre-amplifying the transcriptome of said sub-population of B-cells, preferably by using the template switch protocol as described by Zhu et al. 2001, Biotechniques 30(4):892-897, wherein single stranded cDNA is synthesized with the CDS oligonucleotide SEQ ID NO:32 and the SMART II oligonucleotide SEQ ID NO:33 as switch template. However, the pre-amplification of the transcriptome needs to be balanced against the possible loss of certain rare cDNA species and the possible accumulation of sequence errors.

Thus, in a preferred embodiment, said transcribing of said RNA to cDNA comprises the steps of pre-amplifying the transcriptome of said sub-population of B-cells, wherein preferably said pre-amplifying comprises the steps of: (a) selectively transcribing polyadenylated mRNA contained in said RNA to single stranded cDNA; and (b) amplifying double stranded cDNA from said single stranded cDNA. In a further preferred embodiment said selectively transcribing is performed using the oligonucleotides of SEQ ID NO:32 and SEQ ID NO:33. In a further preferred embodiment said amplifying double stranded cDNA is performed using the oligonucleotides of SEQ ID NO:33 and SEQ ID NO:34, wherein preferably the number of PCR cycles is less than 20, more preferably less than 15, still more preferably 10 to 14, and most preferably 14.

In principle said linker region may consist of any polypeptide comprising suitable length and flexibility to accommodate appropriate folding and assembly of the heavy and light chain variable regions. In a preferred embodiment said linker region consists of 5 to 30, preferably 5 to 22, more preferably 5 to 20, and most preferably of 18 amino acid. It is known to the artisan, that the length of the linker regions influences the structure and, thus, the immunological characteristics of the resulting antibody, in particular of the resulting single chain antibody. Linker regions of less than 15 amino acids in length typically lead to the formation of so called "diabodies", whereas linker regions comprising at least 15 amino acid residues typically lead to the formation of scFv (Huston et al. (1988), PNAS 85(16):587958-83; Holliger et al. (1993), PNAS 90(14):6444-6448, Hollinger & Hudson, 2005, Nature Biotechnology 23(9):1126-1136). Thus, in a further preferred embodiment said linker region consists of 15 to 20, most preferably of 18 amino acid residues. In a very preferred embodiment said linker region comprises or further preferably consists of SEQ ID NO: 107.

In a further preferred embodiment said linker region is encoded by an oligonucleotide contained in said mixture of oligonucleitodes, preferably in said first mixture of oligonucleotides, and/or in said second mixture of oligonucleotides.

Said linking specimens of said first and said second pool of DNA molecules to each other by a DNA encoding said linker region may be performed by ligating said DNA molecules with said DNA encoding said linker region. Typically and preferably, said linking is performed by PCR overlap extension using an overlap in the sequence of the oligonucleotides encoding said linker region. Thus, in a further preferred embodiment a first part of said linker region is encoded by an oligonucleotide contained in said first mixture of oligonucleotides and a second part of said linker region is encoded by an oligonucleotide contained in said second mixture of oligonucleotides, wherein preferably said oligonucleotide encoding said first part of said linker region and said oligonucleotide encoding said second part of said linker region comprise an overlap, wherein further preferably said overlap is at least 3, preferably at least 10, more preferably at least 20, and most preferably 24 nucleotides in length, wherein still further preferably said overlap is at most 50, preferably at most 40, more preferably at most 30, and most preferably at most 24 nucleotides in length.

In a further preferred embodiment said linking of said specimens of said first pool of DNA molecules and of said second pool of DNA molecules to each other is performed by PCR using the oligonucleotides depicted in SEQ ID NO:69 and SEQ ID NO:70 as primers.

Typically and preferably, the resulting multitude of DNA molecules encoding antibodies, preferably human single chain antibodies, most preferably human scFv, are about 750-800 bp in length and further preferably flanked by two Sfi1 restriction sites.

In one embodiment said pool of DNA molecules, preferably said first and/or said second pool of DNA molecules is either generated by pooling DNA molecules obtained in independent PCR reactions, each reaction performed with a different pair of oligonucleotides capable of amplifying VR coding regions, wherein preferably the oligonucleotides contained in an individual reaction are in an equimolar ratio. Thus, said mixture of oligonucleotides, said first mixture of oligonucleotides and/or said second mixture of oligonucleotides comprises or preferably consists of exactly one pair of oligonucleotides capable of amplifying VR coding regions, preferably HCVR coding regions or LCVR coding regions. The artisan may consider to standardize the concentration of DNA molecules generated in different reactions and with different pairs of oligonucleotides in said pool of DNA molecules, preferably said first and/or said second pool of DNA molecules, to the same concentration. The artisan may further consider to adapt in said pool of DNA molecules, preferably said first and/or said second pool of DNA molecules the ratio of DNA molecules encoding certain VR to the frequency of the corresponding VR coding regions in the genome of said B-cells.

Typically and preferably said generating of said pool of DNA molecules, preferably said first and/or said second pool of DNA molecules is performed in a single reaction using more than one pair of oligonucleotides in said reaction. In a preferred embodiment said mixture of oligonucleotides, preferably said first mixture of oligonucleotides, comprises at least two oligonucleotides capable of amplifying human HCVR coding regions. In a further preferred embodiment said mixture of oligonucleotides, preferably said first mixture of oligonucleotides, comprises at least two, preferably all, oligonucleotides selected from the group consisting of SEQ ID NO:42 to 48. In a further preferred embodiment said mixture of oligonucleotides, preferably said second mixture of oligonucleotides, comprises at least two oligonucleotides capable of amplifying kappa LCVR coding regions, preferably human LCVR coding regions. In a further preferred embodiment said mixture of oligonucleotides, preferably said second mixture of oligonucleotides, comprises at least two oligonucleotides capable of amplifying kappa LCVR coding regions, wherein preferably said mixture of oligonucleotides, preferably said second mixture of oligonucleotides, comprises at least two, preferably all, oligonucleotides selected from the group consisting of SEQ ID NO:49 to 56.

In a preferred embodiment said mixture of oligonucleotides, preferably said second mixture of oligonucleotides, comprises at least two oligonucleotides capable of amplifying lambda LCVR coding regions, preferably human lambda LCVR coding regions. In a preferred embodiment said mixture of oligonucleotides, preferably said second mixture of oligonucleotides, comprises at least two oligonucleotides capable of amplifying lambda LCVR coding regions, wherein further preferably said mixture of oligonucleotides, preferably said second mixture of oligonucleotides, comprises at least two, preferably all, oligonucleotides selected from the group consisting of SEQ ID NO:57 to 68.

In a further preferred embodiment said mixture of oligonucleotides, said first mixture of oligonucleotides or said second mixture of oligonucleotides comprise a total amount of primers capable of amplifying VR coding regions, wherein all forward primers and all reverse primers contained in said total amount are in an equimolar ratio.

In a further preferred embodiment said antibody encoded by said expression library comprises exactly one VR and a transmembrane region, wherein preferably said exactly one VR is a HCVR.

In a further preferred embodiment said antibody encoded by said expression library comprises said HCVR, said LCVR and said linker region (LR), in an order selected from: (a) LCVR-LR-HCVR; and (b) HCVR-LR-LCVR; wherein preferably said order is LCVR-LR-HCVR.

To ensure cell surface display of said antibody, said antibody is expressed with a signal peptide directing said antibody to the secretory pathway through the endoplasmic reticulum of said cell, wherein preferably said signal peptide is located at the N-terminus of said antibody, and wherein further preferably said signal peptide is cleaved off said antibody during the processing and transport in said cell. Furthermore, said antibody is expressed with a transmembrane region anchoring said antibody in the cell membrane. Very preferably, said transmembrane region is located at the C-terminus of said antibody and causes said antibody to remain attached to the outer surface of said cell. The anchoring of said antibody in the cell membrane can be achieved by GPI-linking (Moran & Caras 1991, The Journal of Cell Biology 115(6):1595-1600) or, preferably, by directly targeting said transmembrane region to the cell membrane.

Thus, in a preferred embodiment said cloning a specimen of said multitude of DNA molecules into an expression vector comprises the steps of: (a) generating a DNA construct encoding said antibody comprising a signal peptide, a HCVR, a LCVR and a transmembrane region, by linking a specimen of said multitude of DNA molecules to a first DNA element encoding said transmembrane region; and (b) functionally linking said DNA construct to a second DNA element encoding said signal peptide directing said antibody to the secretory pathway, wherein preferably said functionally linking is performed in such a way that said signal peptide is linked to the N-terminus of said antibody.

Signal peptides directing a protein to the secretory pathway of a eukaryotic cell are generally known in the art and are disclosed, for example, in Nielsen et al. (1997), Protein Engineering, 10:1-6. In one embodiment, the signal peptide is derived from a secretory or type I transmembrane protein. In a preferred embodiment, the signal peptide is derived from a secretory protein such as member of the serum protein family (albumin, transferrin, lipoproteins, immunoglobulins), an extracellular matrix protein (collagen, fibronectin, proteoglycans), a peptide hormone (insulin, glucagon, endorphins, enkephalins, ACTH), a digestive enzyme (trypsin, chymotrypsin, amylase, ribonuclease, deoxyribonuclease) or a milk protein (casein, lactalbumin). In a more preferred embodiment, the signal peptide is derived from an immunoglobulin, preferably a light chain variable region. In a further preferred embodiment said signal peptide is a mouse Ig kappa light chain signal peptide, and wherein preferably said signal peptide comprises or further preferably consists of SEQ ID NO:105.

In one embodiment, said transmembrane region is derived from an integral membrane protein. In a preferred embodiment, said transmembrane region is an internal stop-transfer membrane-anchor sequence derived from a type I transmembrane protein (Do et al. (1996), Cell 85:369-78; Mothes et al. (1997), Cell 89:523-533) such as a cell adhesion molecule (integrins, mucins, cadherins), a lectin (Sialoadhesin, CD22, CD33), or a receptor tyrosin kinase (insulin receptor, EGF receptor, FGF receptor, PDGF receptor). In a more preferred embodiment, said transmembrane region is derived from a receptor tyrosine kinase, more preferably from human platelet-derived growth factor receptor (hPDGFR), most preferably from hPDGFR B chain (accession number NP_002600). In a very preferred embodiment said transmembrane region is derived from human PDGFR beta chain, wherein preferably said transmembrane region comprises or further preferably consists of SEQ ID NO:106.

It is advantageous to express said antibody as a polypeptide further comprising a tag allowing the detection of cells expressing said antibody and the quantification of the expression level. Thus, in a further preferred embodiment said antibody further comprises a detection tag, wherein preferably said detection tag is HA, and wherein further preferably said detection tag comprises or still further preferably consists of SEQ ID NO:108.

In a very preferred embodiment said antibody encoded by said expression library comprises a signal peptide (SP), a HCVR, a LCVR, a linker region (LR) and a transmembrane region (TM), wherein the order of said elements from the N- to the C-terminus of said antibody is: SP-LCVR-LR-HCVR-TM. In a further preferred embodiment said antibody encoded by said expression library comprises a signal peptide (SP), a HCVR, a LCVR, a linker region (LR), a transmembrane region (TM) and a detection tag (TAG), wherein the order of said elements from the N- to the C-terminus of said antibody is: SP-LCVR-LR-HCVR-TAG-TM.

The multiplicity of assembled VR coding regions, preferably in the format Sfil-LCVR-GGSSRSSSSGGGGSGGGG-HCVR-Sfi1, are then cloned into an expression vector, preferably a viral expression vector, creating a antibody expression library. In a preferred embodiment said expression library is a viral expression library, preferably a viral expression library derived from an RNA virus, wherein further preferably said RNA virus is a member of the Togaviridae, wherein still further preferably said RNA virus is an alpha virus. In a more preferred embodiment said expression library is an alphaviral expression library, wherein preferably said alphaviral expression library is derived from an alphavirus selected from the group of: (a) Sindbis virus; (b) Semliki forest virus; and (c) Venezuelan equine encephalitis virus. In a very preferred embodiment said alphaviral expression library is derived from Sindbis virus.

Alphaviruses, including Sindbis virus, can function in a broad range of host cells, including mammalian, avian, amphibian, reptilian and insect cells. Their genome comprises elements capable of directing expression of proteins, including heterologous proteins, encoded by nucleic acids of said viral genome in large amounts.

In one embodiment, said expression library is based on a single Sindbis RNA replicon. However, expression of structural and non-structural viral proteins can also be separated, and the structural proteins can be provided either by a packaging cell line or by a helper virus replicon (Bredenbeek PJ, Frolov I, Rice CM, Schlesinger S. (1993) Sindbis virus expression vectors: packaging of RNA replicons by using defective helper RNAs. J Virol. 67, 6439-6446). In a preferred embodiment, said expression library is based on two separate Sindbis RNA replicons, one encoding the nonstructural proteins plus said antibody, the other encoding the structural proteins. A Sindbis based alphaviral expression systems useful in the context of the invention has been described in detail in WO1999/025876A1 which is incorporated herein by reference.

In one embodiment said expression library comprises an expression vector, wherein said expression vector is a viral expression vector, wherein preferably said viral expression vector is an alphaviral expression vector, wherein further preferably said alphaviral expression vector is derived from Sindbis virus. In a preferred embodiment said expression vector comprises DNA elements encoding said signal peptide, said transmembrane region and, optionally, said detection tag in the desired order and further comprises a restriction site allowing the cloning, preferably the orientation specific cloning, of said multitude of DNA molecules into said expression vector. In a preferred embodiment said expression vector comprises a DNA encoding a signal peptide, preferably mouse Ig kappa light chain signal peptide and a transmembrane region, preferably a transmembrane region derived from human PDGFR beta chain. In a very preferred embodiment said expression vector comprises nucleotides 4 to 282 of SEQ ID NO:1. In a still more preferred embodiment said expression vector is an alphaviral expression vector derived from Sindbis virus, wherein said alphaviral expression vector comprises or preferably consists of pDe1-SP-TM (SEQ ID NO:38).

In a further preferred embodiment said expression vector comprises a DNA encoding a signal peptide, preferably mouse Ig kappa light chain signal peptide, a transmembrane region, preferably a transmembrane region derived from human PDGFR beta chain, and a detection tag, preferably HA. In a very preferred embodiment said expression vector comprises 4 to 312 of SEQ ID NO:40. In a still further preferred embodiment said expression vector is an alphaviral expression vector derived from Sindbis virus, wherein said alphaviral expression vector comprises or preferably consists of pDel-SP-HA-TM (SEQ ID NO:39).

In a further preferred embodiment said population of B-cells is derived from an animal exhibiting an increased titer of antibodies specifically binding said antigen of interest. The titer of antibodies binding an antigen of interest in the blood of an animal can be determined by methods generally known in the art, e.g. by ELISA. Thus in a preferred embodiment said titer, preferably said titer in the blood of said animal, is at least 5 times, preferably at least 10 times, most preferably at least 20 times higher than in the average population of said animal, and wherein further preferably said titer can be competed away by said antigen of interest or fragment or antigenic determinant thereof.

In a further preferred embodiment said animal said animal is or has been exposed to said antigen of interest or to a fragment or antigenic determinant thereof, wherein preferably said exposure is by way of natural exposure, infection with a pathogen or immunization. In a further preferred embodiment said animal is or has been infected by a pathogen, wherein said pathogen comprises said antigen of interest or a fragment or antigenic determinant thereof.

In a further preferred embodiment said population of B-cells is derived from an animal immunized with an immunogenic composition, wherein said immunogenic composition comprises or alternatively consists of: (a) said antigen of interest; (b) a fragment of said antigen of interest; and (c) an antigenic determinant of said antigen of interest.. Any immunogenic composition known in the art may be used in the context of the invention. Generally preferred are compositions generating a strong immune response. Preferred immunogenic compositions are compositions comprising a virus-like particle (VLP), preferably a VLP of a RNA bacteriophage, more preferably a VLP of RNA bacteriophages Qbeta, AP205 or fr, most preferably a VLP of RNA bacteriophage Qbeta, and said antigen of interest or an antigenic determinant thereof. Immunogenic compositions useful in the context of the invention are disclosed in WO2006/097530A2, WO2006/097530A2, WO2006/045796A2, WO2006/032674A1, WO2006/027300A2, WO2005/117963A1, WO2006/063974A2, WO2004/084939A2, WO2004/085635A1, WO2005/068639A2, WO2005/108425A1, WO2005/117983A2, WO2005/004907A1, WO2004/096272A2, WO2004/016282A1, WO2004/009124A2, WO2003/039225A2, WO2004/007538A2, WO2003/040164A2, WO2003/031466A2, WO2004/009116A2, and WO2003/024481A2, which are incorporated herein by reference.

In a further preferred embodiment, said immunizing of said animal is performed with an immunogenic composition, wherein the immunogenicity of said immunogenic composition is enhanced by an immunostimulatory substance, preferably by an immunostimulatory oligonucleotide, most preferably by an unmethylated CpG-containing oligonucleotide as disclosed, for example, in WO2003/024481A2, WO2005/004907A1 and WO2004/084940A1, which are incorporated herein by reference. In a very preferred embodiment said unmethylated CpG-containing oligonucleotide is G10 (SEQ ID NO:54 of WO2005/004907A1) which is incorporated herein by reference.

It is within the skill of the artisan to find a dosage and a mode of administration of said immunogenic compositions resulting in high antibody titers. In a preferred embodiment said immunizing of said animal with said immunogenic composition is performed by administering said immunogenic compositions to said animal at least three times, preferably three to six times, in intervals of at least one week, preferably in intervals of two weeks up to three months. In a further preferred embodiment said immunizing of said animal is performed by administering at least 100 µg, preferably 200 to 1000 µg of said immunogenic composition to said animal per single administration. In a further preferred embodiment said immunogenic composition comprises an adjuvant, preferably Freund's complete or incomplete adjuvant or alum.

In a further preferred embodiment said population of B-cells is derived from a source selected from: (a) blood; (b) secondary lymphoid organs, preferably spleen or lymph node; (c) bone marrow; and (d) tissue comprising memory B-cells. Most preferably said source is blood. In a further preferred embodiment said population of B-cells comprises or preferably consists of peripheral blood mononuclear cells (PBMCs).

In a preferred embodiment, said animal is a mammal or a bird. In a preferred embodiment, said animal is selected from the group consisting of: (a) human; (b) mouse; (c) rabbit; and (d) chicken. In a very preferred embodiment, said animal is a mammal, preferably a rat, a mouse or a human. In a further preferred embodiment said animal a humanized mouse or a human, most preferably a human.

The efficiency of the screening for and cloning of antigen specific antibodies can be significantly increased by enriching antigen specific B-cells. Methods for selecting from said population of B-cells a sub-population of B-cells by selecting B-cells for their capability of specifically binding said antigen of interest are generally known in the art. These methods are based on the interaction of antigen-specific B-cells contained in said population of B-cells with the antigen of interest. In a preferred embodiment said selecting from said population of B-cells a sub-population of B-cells comprises the steps of: (a) contacting said population of B-cells with said antigen of interest or a fragment or antigenic determinant thereof; and (b) selecting B-cells specifically binding said antigen of interest or fragment or antigenic determinant thereof.

Preferred methods for selecting from said population of B-cells a sub-population of B-cells are the binding of B-cells to an antigen-covered carrier and FACS sorting and as described in WO2004/102198A2, which is incorporated herein by reference. Thus, in one embodiment said selecting from said population of B-cells a sub-population of B-cells comprises the steps of: (a) coating a carrier with said antigen of interest or fragment or antigenic determinant thereof; (b) contacting said population of B-cells with said carrier and allowing said B-cells to bind to said carrier via said antigen of interest or fragment or antigenic determinant thereof; and (c) removing unbound B-cells, wherein preferably said carrier comprises or further preferably consists of beads, wherein still further preferably said beads are paramagnetic beads.

In a preferred embodiment, said selecting from said population of B-cells a sub-population of B-cells comprises is performed by FACS sorting, wherein preferably said selecting from said population of B-cells a sub-population of B-cells comprises the steps of: (a) contacting said population of B-cells with said antigen of interest or fragment or antigenic determinant thereof, wherein said antigen of interest or fragment or antigenic determinant thereof is labeled with a fluorescence dye; and (b) separating B-cells bound to said antigen of interest or fragment or antigenic determinant thereof by FACS sorting.

In a further preferred embodiment said fluorescence dye is selected from the group consisting of (a) PerCP, allophycocyanin (APC), (b) texas red, (c) rhodamine, (d) Cy3, (e) Cy5, (f) Cy5.5, (f) Cy7, (g) Alexa Fluor Dyes, preferably Alexa 647 nm or Alexa 546 nm (h) phycoerythrin (PE), (i) green fluorescent protein (GFP), (j) a tandem dye (e.g. PE-Cy5), and (k) fluorescein isothiocyanate (FITC). In a very preferred embodiment said fluorescence dye is Alexa 647 nm or Alexa 546 nm. In the context of the invention labeling of a compound, preferably of said antigen of interest or fragment or antigenic determinant thereof, with said fluorescence dye is performed by any method known in the art, preferably by direct labeling said compound by coupling said fluorescence dye to said compound, wherein said coupling may be effected via a covalent as well as a non-covalent bound. Alternatively, labeling of a compound, preferably of said antigen of interest or fragment or antigenic determinant thereof, with said fluorescence dye is performed indirectly by binding to said compound a second compound, preferably an antibody, wherein said second compound comprises said fluorescence dye.

In one preferred embodiment said antigen of interest or fragment or antigenic determinant thereof is coupled to a VLP, preferably to a VLP of a RNA bacteriophage, most preferably to a VLP of bacteriophage Qbeta, wherein said antigen of interest or fragment or antigenic determinant thereof is labeled with said fluorescence dye by binding an anti-VLP antibody to said VLP, wherein said anti-VLP antibody is labeled with said fluorescence dye, wherein preferably said anti-VLP antibody is directly labeled by said fluorescence dye or biotin/steptavidin-fluorescence-labeled.

In one preferred embodiment said antigen of interest or fragment or antigenic determinant thereof is coupled to a VLP, preferably to a VLP of a RNA bacteriophage, most preferably to a VLP of bacteriophage Qbeta, wherein said antigen of interest or fragment or antigenic determinant thereof is labeled with said fluorescence dye by binding an antibody directed against said antigen of interest or fragment or antigenic determinant thereof to said antigen of interest or fragment or antigenic determinant thereof, wherein said antibody directed against said antigen of interest or fragment or antigenic determinant thereof is labeled with said fluorescence dye, wherein preferably said antibody directed against said antigen of interest or fragment or antigenic determinant thereof is directly labeled by said fluorescence dye or biotin/steptavidin-fluorescence-labeled.

If the cloning of a certain type of immunoglobulin is intended, said sub-population of B-cells may, besides the capability of said cells of specifically binding said antigen of interest, be further selected for additional markers which are specific for those types of B-cells expressing immunoglobulins the cloning of which is intended. Alternatively, certain undesired types of B-cells predominantly expressing undesired types of immunoglobulins may be excluded. Additionally, vitality markers such as, for example, PI (propidium iodide) oder 7-AAD (7-Amino-actinomycin) may be applied to select for vital cells. Further additionally or alternatively, cell death or apoptosis markers, such as, for example, YO-PRO-1 or Annexin V may be applied to sort out dead or apoptotic cells.

Furthermore, it is advantageous to include in said selecting from said population of B-cells a sub-population of B-cells a positive selection for the presence of a B-cell specific marker, preferably for CD19 or B220.

In a further embodiment said selecting from said population of B-cells comprises the steps of: (a) contacting said population of B-cells with said antigen of interest or a fragment or antigenic determinant thereof; (b) selecting B-cells specifically binding said antigen of interest or fragment or antigenic determinant thereof; and (c) selecting said B-cells for at least one additional parameter, wherein preferably said selection for said at least one additional parameter is (i) a positive selection for a parameter selected from presence of a B-cell specific marker, preferably CD19 or B220, and vitality of said B-cells; and/or (ii) a negative selection for a parameter selected from: presence of IgM antibodies; presence of IgD antibodies, presence of cell death markers, and presence of apoptosis markers.

Typically and preferably, the cloning of immunoglobulins of the IgG class is intended and, thus, said selecting from said population of B-cells a sub-population of B-cells further comprises the step of selecting for class switched B-cells, preferably for IgM- and/or IgD-negative B-cells, most preferably for IgM- and IgD-negative B-cells.

In a preferred embodiment, said selecting from said population of B-cells a sub-population of B-cells comprises the steps of: (a) contacting said population of B-cells with said antigen of interest or fragment or antigenic determinant thereof, wherein said antigen of interest or fragment or antigenic determinant thereof is labeled with a first fluorescence dye, wherein preferably said fluorescence dye is Alexa 647 nm, Alexa 488 or Alexa 546 nm; (b) contacting the cells of said population of B-cells with anti-IgM and/or anti-IgD antibodies, wherein said anti-IgM and/or anti-IgD antibodies are labeled with a second and/or a third fluorescence dye, wherein said second and/or said third fluorescence dye emits fluorescence at a wavelength which is different from the wavelength of the fluorescence emitted by said first fluorescence dye; and (c) separating B-cells bound to said antigen of interest or fragment or antigenic determinant thereof but not bound to said anti-IgM and/or not bound to said anti-IgD antibodies by FACS sorting.

For the efficiency of the subsequent screening process it is very advantageous though not absolutely essential, that each cell expressing and displaying an antibody on its surface comprises exactly one single antibody species. In general, this can be achieved by choosing a low ratio of expression vectors per number of eukaryotic cells, when introducing said expression library into said first population of eukaryotic cells. Thus, in one embodiment said introducing said expression library into a first population of eukaryotic cells is performed by transfecting said expression vectors to said eukaryotic cells, wherein the ration between the number of said expression vectors and eukaryotic cells is chosen to result in approximately one transfected expression vector per eukaryotic cell, wherein preferably the transfection rate is maintained at a high level by co-transfecting a second plasmid which is not expressed in said eukaryotic cell.

In a preferred embodiment said expression library is a viral expression library, preferably an alphaviral expression library, most preferably an alphaviral expression library derived from Sindbis virus, and said introducing said expression library into a first population of eukaryotic cells is performed by infecting said eukaryotic cell with said viral expression library, wherein preferably said infecting is performed at a multiplicity of infection of at most 10, preferably at most 1, more preferably at most 0.2, and most preferably at most 0.1. In a very preferred embodiment said multiplicity of infection is 0.1.

In a further embodiment said isolating of said cell is performed by FACS sorting. In a preferred embodiment said isolating of said cell comprises the steps of: (a) staining said first population of eukaryotic cells with said antigen of interest or fragment or antigenic determinant thereof, wherein said antigen of interest or fragment or antigenic determinant thereof is labeled with a fluorescence dye; and (b) separating an individual cell specifically binding said antigen of interest, or fragment or antigenic determinant thereof, by means of FACS sorting. The use of said detection tag as a component of said antibody displayed on the surface of said eukaryotic cells allows to further select only cells expressing and/or displaying an antibody. Thus, in a preferred embodiment said antibody further comprises a detection tag, wherein preferably said detection tag is HA, and said isolating of said individual cell comprises the steps of: (a) staining said first population of eukaryotic cells with a compound specifically binding to said detection tag, wherein said compound is labeled with a first fluorescence dye; (b) staining said first population of eukaryotic cells with said antigen of interest or fragment or antigenic determinant thereof, wherein said antigen of interest or fragment or antigenic determinant thereof is labeled with a second fluorescence dye, wherein said second fluorescence dye emits fluorescence at a wavelength which is different from the wavelength of the fluorescence emitted by said first fluorescence dye; and (c) separating an individual cell specifically binding said detection tag and said antigen of interest, or fragment or antigenic determinant thereof, by means of FACS sorting.

In a further embodiment said separating an individual cell specifically binding said antigen of interest, or fragment or antigenic determinant thereof, by means of FACS sorting comprises the step of further selecting said cell at least one additional parameter, wherein preferably said at least one additional parameter is selected from (i) a positive selection for vitality of said cell or the presence of a detection tag; and/or (ii) a negative selection for a parameter selected from: presence of IgM antibodies; presence of IgD antibodies, presence of cell death markers, and presence of apoptosis markers.

In a further preferred embodiment said method further comprises the steps of: (a) cultivating at least one, preferably exactly one, of said individual cells in the presence of a second population of eukaryotic cells; (b) verifying the capability of said second population of eukaryotic cells of specifically binding said antigen of interest, or fragment or antigenic determinant thereof. In a further preferred embodiment said verifying comprises the steps of: (a) staining said second population of eukaryotic cells with said antigen of interest, or fragment or antigenic determinant thereof, wherein said antigen of interest, or fragment or antigenic determinant thereof, is labeled with a fluorescence dye; and (b) detecting cells specifically binding said antigen of interest, or fragment or antigenic determinant thereof, by FACS analysis.

In a further preferred embodiment said first population of eukaryotic cells and/or, preferably and, said second population of eukaryotic cells comprises or preferably consists of cells selected from: (a) BHK 21 cells; (b) Neuro-2a cells; and (c) 293T cells. In a very preferred embodiment said first population of eukaryotic cells and/or, preferably and, said second population of eukaryotic cells comprises or preferably consists of BHK 21 cells. The method of the invention is by no means limited to the nature of the antigen of interest. Therefore said antigen of interest used in the method of the invention may be any antigen of known or yet unknown provenance. In one embodiment, the antigen of interest is a recombinant antigen or a synthetic peptide. In another embodiment, the antigen or antigenic determinant is isolated from a natural source. Preferred antigens of interest used in the present invention can be synthesized or recombinantly expressed and coupled to VLPs, or fused to VLPs using recombinant DNA techniques. Exemplary procedures describing the attachment of antigens to virus-like particles are disclosed in WO00/32227, in WO01/85208 and in WO02/056905, the disclosures of which are herewith incorporated by reference in its entirety.

In a preferred embodiment said antigen of interest is selected from the group consisting of: (a) allergen; (b) self-antigen; (c) tumor antigen; (d) antigen of a pathogen; and (e) haptene.

In a further preferred embodiment said antigen of interest is an allergen, preferably an allergen selected from the group consisting of: (a) pollen allergen, preferably Bet v I (birch pollen allergen); (b) house dust allergen, preferably Der p I (House dust mite allergen); (c) cat allergen, preferably Fel d1; (d) bee venom phospholipase A2; (e) 5 Dol m V (white-faced hornet venom allergen); (f) and an immunogenic fragments of (a) to (e).

In a further preferred embodiment said antigen of interest is a self-antigen, preferably a self-antigen selected from the group consisting of: (a) IL-6; (b) granulocyte macrophages colony stimulating factor (GMCSF); (c) IL-1 alpha; (d) IL-1 beta; (e) IL-5; (f) IL-15; (g) IL-23; (h) tumor necrosis factor (TNF) alpha; (i) receptor activator of nuclear factor kappaB ligand (RANKL); (j) Ghrelin; (k) GIP; (1) adiponectin receptor; (m) amyloid beta, preferably amyloid beta peptide (Aβ1-42); (n) lymphotoxins, preferably Lymphotoxin α (LT α), or Lymphotoxin β (LT β); (o) vascular endothelial growth factor (VEGF) and vascular endothelial growth factor receptor (VEGF-R); (p) MIF; (q) MCP-1; (r) SDF-1; (s) Rank-L; (t) M-CSF; (u) Angiotensin II; (v) Endoglin; (w) Eotaxin; (x) BLC; (y) CCL21; (z) IL-13; (aa) IL-17; (bb) IL-8; (cc) Bradykinin; (dd) Resistin; (ee) LHRH; (ff) GHRH; (gg) GIH; (hh) CRH; (ii) TRH; (jj) Gastrin; (kk) Interferon α; (ll) Interferon γ; (mm) EGF-R; and (nn) fragments of (a) to (mm) which can be used to elicit immunological responses.

In a further preferred embodiment said antigen of interest is a tumor antigen, wherein preferably said tumor antigen is selected from the group consisting of: (a) MelanA; (b) HER2 / ErbB-2 (breast cancer); (c) GD2 (neuroblastoma); (d) EGF-R (malignant glioblastoma); (e) CEA (medullary thyroid cancer); (f) CD52 (leukemia); (g) human melanoma protein gp100; (h) tyrosinase and tyrosinase related proteins, preferably TRP-1 and TRP-2; (i) NA17-A nt protein; (j) MAGE-3 protein; and (k) NY-ESO-1.

In a further preferred embodiment said antigen of interest is an antigen of a pathogen, wherein preferably said pathogen is selected from the group consisting of: (a) hepatitis B virus; (b) influenza A virus; (c) HIV; (d) Hepatitis C virus; (e) rotavirus; (f) polio virus; (g) encephalitis virus; (h) West-Nile virus; (i) SARS virus; (j) Ebola virus; (k) Measles virus; (1) RSV; (m) Toxoplasma; (n) Plasmodium falciparum; (o) Plasmodium ovale; (p) Plasmodium malariae; and (q) Chlamydia.

In a further preferred embodiment said antigen of interest is an antigen of a pathogen, wherein preferably said antigen of interest is selected from the group consisting of: (a) hepatitis B virus preS1 protein; (b) influenza A virus M2 protein; and (b) influenza A virus HA protein.

In a further preferred embodiment said antigen of interest is a haptene, preferably a hapten selected from the group consisting of haptens of: (a) opioids; (b) morphine derivatives, preferably selected from codeine, fentanyl, heroin, morphium and opium; (c) stimulants, preferably selected from amphetamine, cocaine, MDMA (methylenedioxymethamphetamine), methamphetamine, methylphenidate and nicotine; (d) hallucinogens, preferably LSD, mescaline, psilocybin, and cannabinoids. In a very preferred embodiment said antigen of interest is a hapten of nicotine or of a nicotine derivative.

Said individual cell displaying said antibody of interest can then be used to clone and to recombinantly express antibodies comprising the variable regions of said antibody displayed on said cell using methods generally known in the art (see for example Weitkamp et al., 2003, J. Immunol. Meth. 275, 223-237). In principle, it is possible to express said antibodies in any know form (for different forms of antibodies see Hollinger & Hudson (2005), Nature Biotechnology 23(9)), preferably as IgG, most preferably as fully human IgG.

One possibility of producing recombinant antibodies specifically binding said antigen of interest is to express said antibody as a fusion product comprising a purification tag. For example, the expression of single chain antibodies as an Fc-fusion has been described in Ray et al. (2001), Clin. Exp. Immunol. 125(1):94-101 and Ono et al. (2003), J. Biosci. Bioeng. 95(3):231-238). The invention therefore provides for a method of producing an antibody specifically binding an antigen of interest said method comprising the steps of: (a) isolating a cell expressing an antibody according to the method described above; (b) obtaining RNA from said isolated cell; (c) synthesizing cDNA encoding said antibody from said RNA; (d) cloning said cDNA into an expression vector; (e) generating a fusion construct encoding a fusion product comprising said antibody and said purification tag; (e) expressing said fusion product in a cell; and (f) purifying said fusion product. In a further preferred embodiment said antibody comprises at least one VR, preferably a LCVR and a HCVR, and a purification tag, wherein preferably said at least one VR, more preferably said LCVR and said LCVR, are derived from the same of said individual cell. In a preferred embodiment said synthesizing of said cDNA comprises the step of synthesizing single stranded cDNA from said RNA, wherein preferably said single stranded cDNA is synthesized using SEQ ID NO:35 as a primer. In a further preferred embodiment said synthesizing of said cDNA further comprises the step of amplifying said cDNA from said single stranded cDNA, wherein preferably said amplifying is performed using the oligonucleotides of SEQ ID NO:35 and SEQ ID NO:36 as primers. In a still further preferred embodiment said purification tag is Fc, preferably human Fc, and wherein further preferably said purification tag comprises or still further preferably consists of SEQ ID NO:109. In a very preferred embodiment said expression vector is pCEP-SP-Sfi-Fc (SEQ ID NO:37). In a further preferred embodiment said expressing of said fusion product is performed in HEK-293T cells. Antibodies comprising a purification tag can be expressed an purified using standard procedures and are preferably used to test the specificity of said antibody for said antigen of interest of fragment or antigenic determinant thereof by determining the binding constant of said antibody to said antigen of interest or fragment or antigenic determinant thereof, wherein said testing is preferably performed by ELISA, most preferably by ELISA essentially as described in Example 7.

The invention further provides a method of producing an antibody specifically binding an antigen of interest by expressing said antibody as an immunoglobulin, preferably as a species specific immunoglobulin, most preferably as a mouse, rat, rabbit chicken or human immunoglobulin, most preferably as a fully human immunoglobulin.One embodiment of the invention is a method of producing an antibody specifically binding an antigen of interest, said method comprising the steps of: (a) isolating a cell expressing an antibody according to the method described above; (b) obtaining RNA from said cell; (c) synthesizing cDNA form said RNA; (d) amplifying from said cDNA a DNA encoding VRs of said antibody expressed by said cell; (e) generating an expression construct comprising said DNA, wherein said expression construct is encoding at least one VR of said antibody expressed by said cell; (h) expressing said expression construct in a cell. In a preferred embodiment, said method comprising the steps of: (a) according to the method described above; (b) obtaining RNA from said cell; (c) synthesizing cDNA form said RNA; (d) amplifying from said cDNA a first DNA encoding a HCVR of said antibody expressed by said cell; (e) generating a first expression construct comprising said first DNA, wherein said first expression construct is encoding a heavy chain immunoglobulin comprising a heavy chain constant region (HCCR) and said HCVR; (f) amplifying from said cDNA a second DNA encoding a LCVR of said antibody expressed by said cell; (g) generating a second expression construct comprising said second DNA, wherein said second expression construct is encoding a light chain immunoglobulin comprising a light chain constant region (LCCR) and said LCVR; (h) expressing said first expression construct and said second expression construct in a cell. In a further preferred embodiment said HCCR, said HCVR, said LCCR and said LCVR are derived from human.

In a further preferred embodiment said expression construct, said first expression construct and/or said second expression construct are further encoding a hydrophobic leader sequence, preferably a species specific hydrophobic leader sequence, most preferably a human hydrophobic leader sequence. In a further preferred embodiment said first expression construct is further encoding a human heavy chain hydrophobic leader sequence. In a further preferred embodiment said second expression construct is further encoding a human light chain hydrophobic leader sequence, wherein said human light chain hydrophobic leader sequence is selected from the group consisting of (a) human kappa light chain hydrophobic leader sequence; and (b) human lambda light chain hydrophobic leader sequence.

In a further preferred embodiment said synthesizing of said cDNA comprises the step of synthesizing single stranded cDNA from said RNA, wherein preferably said single stranded cDNA is synthesized using SEQ ID NO:35 as a primer. In a further preferred embodiment said synthesizing of said cDNA further comprises the step of amplifying said cDNA from said single stranded cDNA, wherein preferably said amplifying is performed using the oligonucleotides of SEQ ID NO:35 and SEQ ID NO:36 as primers.

In a further preferred embodiment said HCCR is a human HCCR, preferably a human HCCR selected from the group consisting of: (a) human gamma 1 HCCR; (b) human gamma 2 HCCR; (c) human gamma 4 HCCR; and (d) human heavy chain Fd regions, preferably gamma 2 Fd region. In a further preferred embodiment said LCCR is a human LCCR, preferably a human LCCR selected from the group consisting of: (a) human kappa LCCR; and (b) human lambda LCCR.

In a further preferred embodiment said amplifying of said first DNA is performed with HCVR specific primers, wherein preferably said HCVR specific primers are SEQ ID NO:102 and SEQ ID NO:103.

In a further preferred embodiment said amplifying of said second DNA is performed with LCVR specific primers, wherein preferably said LCVR specific primers are selected from kappa LCVR specific primers and lambda LCVR specific primers. In a further preferred embodiment said LCVR specific primers are kappa LCVR specific primers, wherein preferably said kappa LCVR specific primers are a combination of any one selected from SEQ ID NO:92 or 93 with SEQ ID NO:94. In a further preferred embodiment said LCVR specific primers are lambda LCVR specific primers, wherein preferably said lambda LCVR specific primers are a combination of any one selected from SEQ ID NO:95 to 99 with any one of SEQ ID NO:100 or 101.

In a further preferred embodiment said LCCR is a human kappa LCCR and wherein said LCVR is a human kappa LCVR. In a further preferred embodiment said LCCR is a human lambda LCCR and wherein said LCVR is a human lambda LCVR.

In principle, immunoglobulins comprising a heavy and a light chain can be recombinantly produced by expressing two different expression vectors in the same cell. Alternatively, expression constructs encoding said light chain and said heavy chain can be cloned into a single expression vector. Thus, in one embodiment said expressing of said first expression construct and of said second expression construct comprises expressing said first expression construct as part of a first expression vector and expressing said second expression construct as part of a second expression vector, wherein said first expression vector and said second expression vector are co-transfected to said cell. In a preferred embodiment said expressing of said first expression construct and of said second expression construct comprises expressing said first expression construct and said second expression construct as part of the same expression vector, wherein preferably said expression vector is pCB15 (SEQ ID NO:104).

For the expression of species specific, preferably human, antibodies expression cassettes are produced encoding HCCRs or LCCRs of said species, preferably of humans, and the corresponding leader sequences and comprising a restriction site allowing to insert the corresponding VR coding regions. In a preferred embodiment said generating said first expression construct comprises the step of cloning said first DNA into a first expression cassette, wherein said first expression cassette is encoding said HCCR, and, preferably, said HCCR hydrophobic leader sequence, wherein further preferably said first expression cassette comprises or still more preferably consists of a sequence selected from SEQ ID NO:117 to 120. In a further preferred embodiment said generating said second expression construct comprises the step of cloning said second DNA into a second expression cassette, wherein said second expression cassette is encoding said LCCR, and, preferably, said LCCR hydrophobic leader sequence, and wherein further preferably said second expression cassette comprises or still more preferably consists of a sequence selected from SEQ ID NO:121 or 122.

In one embodiment said antibody is expressed in a form selected from: (a) single chain antibody, preferably scFv; (b) diabody; (c) Fab fragment; (d) F(ab')2 fragment; and (e) whole antibody, preferably selected from IgG, IgA, IgE, IgM, and IgD; wherein preferably said antibody is a human antibody, most preferably a fully human antibody.

In a preferred embodiment said antibody is a Fab fragment, wherein preferably said first expression cassette comprises or preferably consists of SEQ ID NO:120 and wherein further preferably said second expression cassette comprises or preferably consists of SEQ ID NO:121. In a further preferred embodiment said antibody is a Fab fragment, wherein said first expression vector comprises or preferably consists of SEQ ID NO:85 and wherein said second expression vector comprises or preferably consists of said SEQ ID NO:71.

In a further preferred embodiment said antibody is a Fab fragment, wherein preferably said first expression cassette comprises or preferably consists of SEQ ID NO:120 and wherein further preferably said second expression cassette comprises or preferably consists of SEQ ID NO:122. In a further preferred embodiment said antibody is a Fab fragment and said first expression vector comprises or preferably consists of SEQ ID NO:85 and said second expression vector comprises or preferably consists of said SEQ ID NO:110.

In another embodiment said antibody is expressed as a whole antibody of the IgG class, preferably as IgG1, IgG2, IgG3, or IgG4; wherein preferably said antibody is a human antibody, most preferably a fully human antibody.

In a preferred embodiment said antibody is a IgG1, and wherein preferably said first expression cassette comprises or preferably consists of SEQ ID NO:118 and wherein further preferably said second expression cassette comprises or preferably consists of SEQ ID NO:121. In a further preferred embodiment said first expression vector comprises or preferably consists of SEQ ID NO:88 and wherein said second expression vector comprises or preferably consists of said SEQ ID NO:71. In a further preferred embodiment said antibody is a IgG1, and wherein preferably said first expression cassette comprises or preferably consists of SEQ ID NO:118 and wherein further preferably said second expression cassette comprises or preferably consists of SEQ ID NO:122. In a further embodiment said first expression vector comprises or preferably consists of SEQ ID NO:88 and wherein said second expression vector comprises or preferably consists of said SEQ ID NO:110.

In a further preferred embodiment said antibody is a IgG2, and wherein preferably said first expression cassette comprises or preferably consists of SEQ ID NO:117 and wherein further preferably said second expression cassette comprises or preferably consists of SEQ ID NO:121. In a further preferred embodiment said first expression vector comprises or preferably consists of SEQ ID NO:78 and wherein said second expression vector comprises or preferably consists of said SEQ ID NO:71. In a further preferred embodiment said antibody is a IgG2, and wherein preferably said first expression cassette comprises or preferably consists of SEQ ID NO:117 and wherein further preferably said second expression cassette comprises or preferably consists of SEQ ID NO:122. In a further preferred embodiment said first expression vector comprises or preferably consists of SEQ ID NO:78 and wherein said second expression vector comprises or preferably consists of said SEQ ID NO:110.

In a further preferred embodiment said antibody is a IgG4, and wherein preferably said first expression cassette comprises or preferably consists of SEQ ID NO:119 and wherein further preferably said second expression cassette comprises or preferably consists of SEQ ID NO:121. In a further preferred embodiment said first expression vector comprises or preferably consists of SEQ ID NO:90 and wherein said second expression vector comprises or preferably consists of said SEQ ID NO:71. In a further preferred embodiment said antibody is a IgG4, and wherein preferably said first expression cassette comprises or preferably consists of SEQ ID NO:119 and wherein further preferably said second expression cassette comprises or preferably consists of SEQ ID NO:122. In a further preferred embodiment said first expression vector comprises or preferably consists of SEQ ID NO:90 and wherein said second expression vector comprises or preferably consists of said SEQ ID NO:71.

Said expressing of said antibody may be performed in any eukaryotic expression system known in the art. Typically and preferably, said expressing of said antibody is performed in eukaryotic cells, wherein further preferably said eukaryotic cells are selected from yeast cells, insect cells and mammalian cells. In a preferred embodiment said expressing of said antibody is performed in mammalian cells, wherein preferably said mammalian cells are selected from HEK-293T cells, CHO cells, COS cells. Very preferably said mammalian cells are HEK-293T cells.

The invention further relates to an expression vector for displaying polypeptides, preferably antibodies, most preferably single chain antibodies, on the surface of a eukaryotic cell. The invention thus relates to an expression vector, preferably a viral expression vector, more preferably alphaviral expression vector, most preferably an expression vector derived from Sindbis virus, wherein said expression vector comprises DNA elements encoding a signal peptide, a transmembrane region and, preferably, a detection tag, and wherein further preferably said expression vector comprises a restriction site allowing the cloning, preferably the orientation specific cloning, of DNA molecules encoding said polypeptides, preferably said antibody variable regions, into said expression vector. In a further preferred embodiment said expression vector comprises said DNA elements and said restriction site in an orientation allowing the expression of a fusion protein comprising from the N- to the C-terminus said signal peptide, said polypeptide, preferably said detection tag, and said transmembrane region.

In a preferred embodiment said signal peptide is mouse Ig kappa light chain signal peptide. In a further preferred embodiment said transmembrane region is derived from human PDGFR beta chain. In a further preferred embodiment said signal peptide is mouse Ig kappa light chain signal peptide and said transmembrane region is derived from human PDGFR beta chain. In a very preferred embodiment said expression vector comprises nucleotides 4 to 282 of SEQ ID NO:1. In a still more preferred embodiment said expression vector is an alphaviral expression vector derived from Sindbis virus, wherein said alphaviral expression vector comprises or preferably consists of pDe1-SP-TM (SEQ ID NO:38).

In a further preferred embodiment said detection tag is HA. In a further preferred embodiment said signal peptide is mouse Ig kappa light chain signal peptide, said transmembrane region is derived from human PDGFR beta chain and said detection tag is HA. In a very preferred embodiment said expression vector comprises nucleotides 4 to 312 of SEQ ID NO:40. In a still further preferred embodiment said expression vector is an alphaviral expression vector derived from Sindbis virus, wherein said alphaviral expression vector comprises or preferably consists of pDe1-SP-HA-TM (SEQ ID NO:39).

The invention further relates to an expression library, preferably to an expression library expressing antibodies, wherein further preferably said antibodies are single chain antibodies, wherein still further preferably said single chain antibodies are human single chain antibodies, said expression library comprising said expression vector. In a preferred embodiment, said expression library comprises nucleotides 4 to 282 of SEQ ID NO:1 or, further preferably, said expression library comprises SEQ ID NO:38. In a further preferred embodiment, said expression library comprises nucleotides 4 to 312 of SEQ ID NO:40 or, further preferably, said expression library comprises SEQ ID NO:39.

The invention further relates to an eukaryotic cell comprising said expression vector or comprising at least one specimen of said expression library.

### EXAMPLES

### EXAMPLE 1

### Construction of pDel-SP-TM, a Sindbis-based viral vector allowing cell surface display of single-chain antibodies

A DNA fragment (SEQ ID NO:1) encoding a mouse Ig kappa signal peptide (SEQ ID NO:105), two SfiI restriction sites and the transmembrane region of the human platelet-derived growth factor receptor beta chain (PDGFR, SEQ ID NO:106) was assembled from six overlapping oligonucleotides. Briefly, the oligonucleotides SPTM-2 (5'-CCT GCT ATG GGT ACT GCT GCT CTG GGT TCC AGG TTC CAC TGG TGA CTA TGA GGC CCA GGC GGC CGG TAC-3', SEQ ID NO:26), SPTM-3 (5'-CCT CCT GCG TGT CCT GGC CCA CAG CAT TGC GGC CGG CCT GGC CGC TAG CGG TAC CGG CCG CCT GGG CCT C-3', SEQ ID NO:27), SPTM-4 (5'-GGC CAG GAC ACG CAG GAG GTC ATC GTG GTG CCA CAC TCC TTG CCC TTT AAG GTG GTG GTG ATC TCA GCC-3', SEQ ID NO:28) and SPTM-5 (5'-CAT GAT GAG GAT GAT AAG GGA GAT GAT GGT GAG CAC CAC CAG GGC CAG GAT GGC TGA GAT CAC CAC CAC C-3' SEQ ID NO:29) were mixed at a final concentration of 0.1 µM each in a 100 µl polymerase chain reaction (PCR) and cycled 20 times (20 sec at 94 °C; 20 sec at 60 °C; 40 sec at 72 °C) in the presence of 2.5 units Taq DNA polymerase (Invitrogen) under the manufacturer's recommended reaction conditions. 1 µl of this reaction was then mixed with the oligonucleotides SPTM-1 (5'-GAG TCT AGA GCC ACC ATG GAG ACA GAC ACA CTC CTG CTA TGG GTA CTG CT GCT C-3', SEQ ID NO:30) and SPTM-6 (5'-CTC GGG CCC CTA ACG TGG CTT CTT CTG CCA AAG CAT GAT GAG GAT GAT AAG GGA G-3', SEQ ID N0:31) at a final concentration of 0.1 µM each in a second 100 µl PCR reaction and cycled for another 20 cycles as above. The resulting 285 bp DNA fragment was digested with the restriction endonucleases XbaI and ApaI, purified by agarose gel electrophoresis, and ligated into the Xbal/Apal digested Sindbis virus expression vector pDelSfi, yielding the scFv display vector pDel-SP-TM (SEQ ID NO:38).

For the construction of pDe1-SP-HA-TM (SEQ ID NO:39), a 315 bp DNA fragment (SP-HA-TM Linker, SEQ ID NO:40), which in addition encodes a haemagglutinin (HA) tag between the SfiI sites and the TM region, was assembled and cloned. The whole procedure was identical to the one for pDe1-SP-TM (SEQ ID NO:38), except that the oligo SPTM-3 (SEQ ID NO:27) was replaced by the oligo SPTM-3HA (5'-CCT CCT GCG TGT CCT GGC CCA CAG CAT TAG AGG CAT AAT CTG GCA CGT CGT AAG GAT AGC GGC CGG CCT GGC CGC TAG CGG TAC CGG CCG CCT GGG CCT C-3', SEQ ID NO:41).

### EXAMPLE 2

### Isolation of Qβ-specific human memory B cells from peripheral blood mononuclear cells

Peripheral blood mononuclear cells (PBMC) were isolated from 20 ml of heparinized blood of a Qβ-vaccinated volunteer by a standard Ficoll-HypaqueTM Plus (Amersham Biosciences) gradient method. PBMC were stained with Alexa 647 nm-labeled Qβ (4 µg/ml), FITC-labeled mouse anti-human IgM (1.5 µg/ml) (Jackson ImmunoResearch Laboratories), FITC-labeled mouse anti-human IgD (diluted 1:50) (BD Biosciences Pharmingen), and PE-labeled mouse anti-human CD19 (diluted 1:100) (BD Biosciences Pharmingen). After 30 min cells were washed, filtered and stained with propidium iodide (PI) to exclude dead cells. 230 Qβ-specific memory B cells (Qβ-, CD19-positive, IgM-, IgD-, PI-negative) were sorted on a FACSVantage SE flow cytometer (Becton Dickinson) and used for library construction.

### EXAMPLE 3

### Construction of a single-chain antibody cell surface display library from Qβ-specific human memory B cells

Total RNA was isolated from 230 Qβ-specific human memory B cells using TRI reagent (Molecular Research, Inc.). Single-stranded cDNA was produced with PowerScriptTM reverse transcriptase (Clontech) using the template switch protocol (Zhu et al. 2001 Biotechniques 30(4):892-7), with the CDS oligonucleotide (5'-AAG CAG TGG TAA CAA CGC AGA GTA CTT TTT TTT TTT TTT TTT TTT TTT TTT TTT TVN-3', SEQ ID NO:32) as primer, and the SMART II oligonucleotide (5'-d[AAG CAG TGG TAA CAA CGC AGA GTA CGC] r[GGG]-3', SEQ ID NO:33) as switch template. The cDNA was bulk-amplified by 14 cycles of PCR, using the Advantage2 polymerase mix (Clontech) and an anchor primer (5'-AAG CAG TGG TAT CAA CGC AGA GT-3', SEQ ID NO:34) in a total volume of 200 µl. Double-stranded cDNA was purified with the Qiaquick PCR purification kit (Qiagen).

A single-chain antibody library was then produced essentially as described (Phage Display: A Laboratory Manual, Cold Spring Harbor Laboratory Press, 2001) using the pre-amplified ds-cDNA as template. Briefly, heavy chain variable region coding sequences were amplified with an equimolar mix of 6 sense primers (HSCVH1-FL, SEQ ID NO:42; HSCVH2-FL, SEQ ID NO:43; HSCVH3a-FL, SEQ ID NO:44; HSCVH4a-FL, SEQ ID NO:45; HSCVH4-FL, SEQ ID NO:46; and HSCVH35-FL, SEQ ID NO:47) plus an antisense constant region primer (HSCG1234-B; SEQ ID NO:48); the _{K} light chain variable region coding sequences were amplified with an equimolar mix of 4 sense primers (HSCK1-F, SEQ ID NO:49; HSCK24-F, SEQ ID NO:50; HSCK3-F, SEQ ID NO:51; and HSCK5-F, SEQ ID NO:52) plus an equimolar mix of 4 antisense primers (HSCJK14o-B, SEQ ID NO:53; HSCJK2o-B, SEQ ID NO:54; HSCJK3o-B SEQ ID NO:55; and HSCJK5o-B, SEQ ID NO:56); and the λ light chain variable region coding sequences were amplified with an equimolar mix of 9 sense primers (HSCLamla, SEQ ID NO:57; HSCLamlb, SEQ ID NO:58; HSCLam2, SEQ ID NO:59; HSCLam3, SEQ ID NO:60; HSCLam4, SEQ ID NO:61; HSCLam6, SEQ ID NO:62; HSCLam78, SEQ ID NO:63; HSCLam9, SEQ ID NO:64; and HSCLam10 SEQ ID NO:65) plus an equimolar mix of 3 antisense primers (HSCJLam1236, SEQ ID NO:66; HSCJLam4, SEQ ID NO:67 and HSCJLam57, SEQ ID NO:68).

The scFv coding regions were assembled by PCR overlap extension of the VH PCR product with either the the Vκ PCR product or the Vλ PCR product using the primers RSC-F (SEQ ID NO:69) and RSC-B (SEQ ID NO:70). The resulting ~750-800 bp PCR products encoded a 5' light chain variable region (either κ or λ) and a 3' heavy chain variable region, linked by an 18 amino acid flexible linker, and flanked by two SfiI restriction sites. The κ-and λ-containing scFv fragments were pooled in equimolar ratio, digested with the restriction endonuclease SfiI, purified by agarose gel electrophoresis and cloned into SfiI-digested pDel-SP-TM (SEQ ID NO:38). The resulting library consisted of approximately 106 independent transformands. DNA was isolated from pooled colonies using the HiSpeed Plasmid Maxi Kit (Qiagen).

As a measure of library quality, individual clones were sequenced to ascertain diversity and overall structural organization of the single-chain antibodies, as well as their in frame fusion to the N-terminal Ig _{K} signal peptide and C-terminal PDGFR transmembrane region. Of the six scFv clones that were sequenced each corresponded to a different scFv, indicating that the library is diverse (SEQ ID NOs:2-7). Further, all six clones were fused in-frame to both signal peptide and transmembrane region. In addition, most of the clones displayed an intact open reading frame, with only one clone having an in-frame stop codon in the heavy chain variable region as a result of a point mutation. This is likely to be a PCR mutation resulting from the extensive amplification during library construction. In conclusion, the scFv cell surface display library was diverse and predominantly consisted of functional antibodies that can be expected to be displayed on the cell surface.

The plasmid library was converted into a Sindbis virus library as follows. For in vitro transcription, 5 µg of the library plasmid was linearized, half with the restriction endonuclease NotI (Roche), the other half with PacI (New England Biolabs). 5 µg of the helper plasmid pDHEB (Bredenbeek et al. 1993 J Virol. 67(11):6439-6446), encoding the Sindbis virus structural proteins, was linearized with the restriction endonuclease EcoRI. All restriction digests were then extracted with phenol-chloroform, ethanol precipitated, and resuspended in RNase-free H₂O at a concentration of 0.5 µg/µl. 1 µg of the linearized library and of the helper plasmid were subjected to SP6 RNA polymerase-mediated in vitro transcription in a volume of 20 µl, using the mMessage mMachineTM kit (Ambion). The transcribed library RNA was co-electroporated with an equimolar amount of helper RNA into 10⁷ BHK cells. 18 hours post transfection, cell supernatant was harvested and the viral titer determined to be approximately 10⁷ per ml. This Sindbis virus based cell surface display library was then used to isolate Qβ-specific single-chain antibodies.

### EXAMPLE 4

### Identification of cells displaying Qβ-specific single-chain antibodies by fluorescence-activated cell sorting

Sixty million subconfluent (80 %) baby hamster kidney (BHK) cells were infected with the single-chain antibody library derived from Qβ-specific variable domains or an empty viral vector as a negative control at a multiplicity of infection (MOI) of 0.2. After 5 hours, cells were detached with cell dissociation buffer (Sigma), washed and stained. Half of the cells were stained with Alexa 647 nm-labeled Qβ (4 µg/ml) for 30 min. The remaining cells were stained with Alexa 546 nm-labeled Qβ (4 µg/ml) and an anti-sindbis serum from rabbit (diluted 1:6000) for 30 min, followed by staining with Cy5-labeled donkey anti-rabbit IgG (1 µg/ml) (Jackson ImmunoResearch Laboratories) for 20 min. All cells were then washed, filtered and stained with propidium iodide (PI) to exclude dead cells. Single cell sorting was performed on a FACS Vantage SE flow cytometer (Becton Dickinson) for, respectively, Alexa 647 nm-positive, PI-negative and, Alexa 546 nm-positive, sindibis-positive, PI-negative cells. In total, 480 cells were sorted , 264 from the Alexa 647 nm sorting, and 216 from the Alexa 546 nm sorting.

Each cell was sorted into a well of a 24-well plate containing 50 % confluent BHK feeder cells. Upon virus spread (2-3 days post sorting), the infected cells were tested by FACS analysis for Qβ binding. On day 2 post sorting, 228 wells showed typical signs of viral infection, 199 of which bound Qβ. On day 3 post sorting, another 48 wells showed clear viral infection with 39 of them binding Qβ.

### EXAMPLE 5

### Rescue of cDNA encoding Qβ-specific single-chain antibodies

To obtain cDNAs encoding Qβ-specific single-chain antibodies, RT-PCR was performed using supernatants from BHK cells, each containing monoclonal recombinant Sindbis virus. For the viral RNA isolation, 140 µl of viral supernatant and the QIAamp Viral RNA Kit (Qiagen) were used. The procedure was performed according to manufacturer's protocol and the RNA was dissolved in 30 µl RNase-free H₂O. For the cDNA synthesis 8 µl of the viral RNA were used per reaction. The 1st strand cDNA was synthesized in a 20 µl reaction containing 20 pmoles LPP2 primer (5'- ACA AAT TGG ACT AAT CGA TGG C-3', SEQ ID NO:35), using PowerScriptTM reverse transcriptase (Clontech) according to the manufacturer's recommendations.

Single-chain antibody cDNAs were PCR amplified from 2 µl 1st strand cDNA in 100 µl reactions with the primers pDel-seq (5'-GAG CAA AAG AGC ATT CCA AG-3', SEQ ID NO:36) and LPP2 (SEQ ID NO:35), using the Advantage 2 Polymerase mix (Clontech) according to the manufacturer's recommendations. The PCR reaction was performed with one cycle of 1 min at 95 °C followed by 30 cycles of 20 sec at 95 °C, 20 sec at 56 °C, 90 sec at 72 °C. The resulting PCR products were analyzed on an agarose gel and the ~750-800 bp bands isolated using the QIAquick gel extraction Kit (Qiagen) according to manufacturer's protocol. Each gel-purified PCR product was then subjected to sequencing using the primers pDe1-seq and LPP2 (~100-200 ng per sequencing reaction).

A total of 14 PCR products were sequenced, the scFv coding regions assembled and the sequences predicted for the displayed scFvs determined (SEQ ID NOs:8-21). With the exception of one clone, each of the single-chain antibodies had an open reading frame and was fused in-frame to both signal peptide and transmembrane region, as was to be expected. ScFv-Qb#18 (SEQ ID NO:18) had a frame shift at the beginning of the heavy chain variable region followed by an early termination, leading to a protein lacking not only most of the heavy chain V region, but also the transmembrane region. Such a protein is expected to be secreted and should not be selected by our cell surface display strategy. Thus, it seems likely that the mutation was introduced during the gene rescue PCR amplification.

The sequence diversity was significantly reduced compared to prior to the screen. While there were no two scFvs with identical sequence, many were clearly closely related. Significantly, there were several scFvs where one of the two variable regions were identical. For instance, scFv-Qb#2 (SEQ ID NO:8), scFv-Qb#3 (SEQ ID NO:9), scFv-Qb#4 (SEQ ID NO:10) and scFv-Qb#6 (SEQ ID NO:12) share the same heavy chain variable region. Similarly, the light chain variable regions of scFv-Qb#2 (SEQ ID NO:8), scFv-Qb#5 (SEQ ID NO:11) and scFv-Qb#7 (SEQ ID NO:13) are almost identical and differ by only one or a few amino acids.

### EXAMPLE 6

### Construction, expression, and purification of the Qβ-specific scFv-Fc fusion proteins

Synthetic constructs were produced allowing for the eukaryotic expression of fusion proteins carrying an N-terminal human scFv fused to a C-terminal human Fc-γ1 domain. Thus, PCR products corresponding to scFv-Qb#2 (SEQ ID NO:8), scFv-Qb#3 (SEQ ID NO:9), scFv-Qb#5 (SEQ ID NO:11) and scFv-Qb#8 (SEQ ID NO:14) were digested with the restriction endonuclease SfiI (New England Biolabs) and cloned into the expression vector pCEP-SP-Sfi-Fc (SEQ ID NO:37). This vector is a derivative of the episomal mammalian expression vector pCEP4 (Invitrogen, cat. no. V044-50), carrying the Epstein-Barr Virus replication origin (oriP) and nuclear antigen (encoded by the EBNA-1 gene) to permit extrachromosomal replication, and contains a puromycin selection marker in place of the original hygromycin B resistance gene. The resulting plasmids, pCEP/scFvQb#2-Fc, pCEP/scFvQb#3-Fc, pCEP/scFvQb#5-Fc and pCEP/scFvQb#8-Fc drive expression of scFv-Fc domain fusion proteins (SEQ ID NOs:22-25) under the control of a CMV promoter.

Expression of the fusion constructs was done in HEK-293T cells. One day before transfection, 10⁷ 293T cells were plated onto a 14 cm tissue culture plate for each protein to be expressed. Cells were then transfected with the respective scFv-Fc fusion construct using Lipofectamin Plus (Invitrogen) according to the manufacturer's recommendations, incubated one day, and replated on three 14 cm dishes in the presence of 1 µg/ml puromycin. After 3 days of selection, puromycin-resistant cells were transferred to six Poly-L-Lysine coated 14 cm plates and grown to confluency. Medium was then replaced by serum-free medium and supernatants containing the respective scFv-Fc fusion protein was collected every 3 days and filtered through a 0.22 µM Millex GV sterile filter (Millipore).

For each of the scFv-Fc fusion proteins, the consecutive harvests were pooled and applied to a protein A-sepharose column. The column was washed with 10 column volumes of phosphate-buffered saline (PBS), and bound protein eluted with 0.1 M Glycine pH 3.6. 1 ml fractions were collected in tubes containing 0.1 ml of 1 M Tris pH 7.5 for neutralization. Protein-containing fractions were analyzed by SDS-PAGE and pooled. The buffer was exchanged with PBS by dialysis using 10'000 MWCO Slide-A-Lyzer dialysis cassettes (Pierce). The purified proteins in PBS were then filtered through 0.22 µM Millex GV sterile filters (Millipore) and aliquotted. Working stocks were kept at 4°C, whereas aliquots for long-term storage were flash-frozen in liquid nitrogen and kept -80°C.

### EXAMPLE 7

### Verification of Qβ-specific binding of scFv-Fc fusion proteins by ELISA

ELISA plates (96 well MAXIsorb, NUNC immuno plate. 442404) were coated with Qβ at a concentration of 2 µg/ml in coating buffer (0.1 M NaHCO₃, pH 9.6), over night at 4 °C. Alternatively, ELISA plates were coated with 2 µg/ml of an irrelevant control protein. The plates were then washed with wash buffer (PBS/0.05 % Tween) and blocked for 1 h at 37 °C with 3 % BSA in wash buffer. The plates were then washed again and incubated with serially diluted scFv-Qb#2-Fc (SEQ ID NO:8), scFv-Qb#3-Fc (SEQ ID NO:9), scFv-Qb#5-Fc (SEQ ID NO:11) and scFv-Qb#8-Fc (SEQ ID NO:14) (either serum-free tissue culture supernatant or purified scFv-Fc fusion proteins). Plates were incubated at 37 °C for 1 h and then extensively washed with wash buffer. Bound specific scFv-Fc fusion proteins were then detected by a 30 minute incubation with a HRPO-labeled, Fcγ-specific, goat anti-human IgG antibody (Jackson ImmunoResearch Laboratories 109-035-098). After extensive washing with wash buffer, plates were developed with OPD solution (1 OPD tablet, 25 µl OPD buffer and 8ul H2O2) for 5 to 10 minutes and the reaction was stopped with 5 % H₂SO₄ solution. Plates were then read at OD 450 nm on an ELISA reader (Biorad Benchmark). Half-maximal binding of purified scFv-Fc fusion proteins was observed at picomolar concentrations (scFv-Qb#2-Fc, 51 pM; scFv-Qb#3-Fc, 35 pM; scFv-Qb#5-Fc, 52 pM; scFv-Qb#8-Fc, 163 pM), suggesting that the antibodies are of very high affinity.

### EXAMPLE 8.

### Construction of vectors allowing for expression of human antibodies as whole IgG or Fab.

pCMV-LC (SEQ ID NO:71), a vector allowing for the expression of natural human antibody _{K} light chains, was generated as follows. First, a DNA segment encoding an Ig _{K} light chain signal peptide was assembled from the 4 oligonucleotides SP-kappa-1 (5'-GGC TAG CGC CAC CAT GGA CAT GAG GGT CCC CGC TCA GCT CCT GGG GCT C-3', SEQ ID NO:72), SP-kappa-2 (5'-CAG GAG CTG AGC GGG GAC CCT CAT GTC CAT GGT GGC GCT AGC CAG CT-3', SEQ ID NO:73), SP-kappa-3 (5'-CTG CTA CTC TGG CTC CGA GGT GCC AGA TGT GAC ATC GAG CTC CTG CA-3', SEQ ID NO:74) and SP-kappa-4 (5'-GGA GCT CGA TGT CAC ATC TGG CAC CTC GGA GCC AGA GTA GCA GGA GCC C-3', SEQ ID NO:75), by annealing the complementary oligonucleotides SP-kappa-1 and -2, and SP-kappa-3 and -4, respectively. The two resulting double stranded DNA fragments SP-kappa-1/2 and SP-kappa-3/4 were cloned into the vector pCMV-Script (Stratagene) digested with the restriction endonucleases SacI and PstI, yielding pCMV-kappa-leader. Second, the human κ light chain constant region was amplified from human spleen cDNA using the primers C-kappa-F (5'-GAG GAG GAT ATC AAA CGA ACT GTG GCT GCA CCA TC-3', SEQ ID NO:76) and C-kappa-B (5'-GAG GAG GGT ACC GTT TAA ACC TAA CAC TCT CCC CTG TTG AAG CTC TTT GTG ACG GGC GAA CTC AGG CC-3', SEQ ID NO:77). The resulting 359 bp PCR product was digested with the restriction endonucleases EcoRV and KpnI and cloned into the vector pCMV-Script, yielding pCMV-C-kappa. Third, after the correct sequence of both plasmids was verified, pCMV-kappa-leader and pCMV-C-kappa were digested with the restriction endonucleases EcoRV and KpnI. The 343 bp fragment excised from pCMV-C-kappa, corresponding to the _{K} light chain constant region, was then ligated into the 4282 bp pCMV-kappa-leader vector fragment, yielding the light chain expression vector pCMV-LC (SEQ ID NO:71). DNA fragments encoding light chain variable regions can be cloned into pCMV-LC via the restriction endonucleases SacI and EcoRV and expressed as part of natural κ light chains.

pCMV-LC-lambda (SEQ ID NO:110), a vector allowing for the expression of natural human antibody λ light chains, was generated as follows. First, a DNA segment encoding an Ig λ light chain signal peptide was assembled from the 4 oligonucleotides SP-lambda-1 (5'-GGC TAG CGC CAC CAT GGC CTG GGC TCT GCT CCT CCT CAC CCT CCT-3', SEQ ID NO:111), SP-lambda-2 (5'-GTG AGG AGG AGC AGA GCC CAG GCC ATG GTG GCG CTA GCC AGC T-3', SEQ ID NO:112), SP-lambda-3 (5'-CAC TCA GGG CAC AGG GTC CTG GGC CCA GTC TGA GCT CCT GCA-3', SEQ ID NO:113) and SP-lambda-4 (5'-GGA GCT CAG ACT GGG CCC AGG ACC CTG TGC CCT GAG TGA GGA GG-3', SEQ ID NO:114), by annealing the complementary oligonucleotides SP-lambda-1 and -2, and SP-lambda-3 and -4, respectively. The two resulting double stranded DNA fragments SP-lambda-1/2 and SP-lambda-3/4 were cloned into the vector pCMV-Script (Stratagene) digested with the restriction endonucleases SacI and PstI, yielding pCMV-lambda-leader. Second, the human λ light chain constant region was amplified from human spleen cDNA using the primers C-lambda-F (5'-GAG GAG GAT ATC CTA GGT CAG CCC AAG GCT GCC CC-3', SEQ ID NO:115) and C-lambda-B (5'-GAG GAG GGT ACC GTT TAA ACC TAT GAA CAT TCT GTA GGG GC-3', SEQ ID NO:116). The resulting 356 bp PCR product was digested with the restriction endonucleases EcoRV and Kpn1 and cloned into the vector pCMV-Script, yielding pCMV-C-lambda. Third, after the correct sequence of both plasmids was verified, pCMV-lambda-leader and pCMV-C-lambda were digested with the restriction endonucleases EcoRV and KpnI. The 340 bp fragment excised from pCMV-C-lambda, corresponding to the λ light chain constant region, was then ligated into the 4273 bp pCMV-lambda-leader vector fragment, yielding the light chain expression vector pCMV-LC-lambda. DNA fragments encoding lambda light chain variable regions can be cloned into pCMV-LC-lambda via the restriction endonucleases SacI and EcoRV and expressed as part of natural λ light chains.

pCMV-HC (SEQ ID NO:78), a vector allowing for the expression of natural human antibody γ2 heavy chains, was generated as follows. First, a DNA segment encoding an Ig heavy chain signal peptide was assembled from the 4 oligonucleotides SP-heavy-1 (5'-CGG CGC GCC ACC ATG GAC TGG ACC TGG AGG ATC CTC TT-3' SEQ ID NO:79), SP-heavy-2 (5'-ACC AAG AAG AGG ATC CTC CAG GTC CAG TCC ATG GTG GCG CGC CGA GCT-3' SEQ ID NO:80), SP-heavy-3 (5'-CTT GGT GGC AGC AGC CAC AGG AGC CCA CTC CCA GAT GCA ACT GC-3' SEQ ID NO:81) and SP-heavy-4 (5'-TCG AGC AGT TGC ATC TGG GAG TGG GCT CCT GTG GCT GCT GCC-3' SEQ ID NO:82), by annealing the complementary oligonucleotides SP-heavy-1 and -2, and SP-heavy-3 and -4, respectively. The two resulting double stranded DNA fragments SP-heavy-1/2 and SP-heavy-3/4 were cloned into the vector pCMV-Script (Stratagene) digested with the restriction endonucleases SacI and XhoI, yielding pCMV-heavy-leader. Second, the human γ2 heavy chain constant region was amplified from human spleen cDNA using the primers C-gamma2-FL (5'-GAG GAG CTC GAG GCC TCC ACC AAG GGC CCA TCG GTC TTC CCC CTG GCG CCC TGC TCC AGG AGC ACC TCC-3' SEQ ID NO:83) and C-gamma2-B (5'-GAG GAG GGT ACC TTA ATT AAT CAT TTA CCC GGA GAC AGG GAG-3' SEQ ID NO:84). The resulting 1013 bp PCR product was digested with the restriction endonucleases XhoI and KpnI and cloned into the vector pCMV-Script, yielding pCMV-C-gamma2. Third, after the correct sequence of both plasmids was verified, pCMV-heavy-leader and pCMV-C-gamma2 were digested with the restriction endonucleases XhoI and KpnI. The 999 bp fragment excised from pCMV-C-gamma2, corresponding to the γ2 heavy chain constant region, was then ligated into the 4258 bp pCMV-gamma2-leader vector fragment, yielding the heavy chain expression vector pCMV-HC. DNA fragments encoding heavy chain variable regions can be cloned into pCMV-HC via the restriction endonucleases XhoI and ApaI and expressed as part of natural γ2 heavy chains. Cotransfection of a pCMV-LC (SEQ ID NO:71) with a pCMV-HC (SEQ ID NO:78) expression construct will allow for the production of whole IgG2.

pCMV-Fd (SEQ ID NO:85), a vector allowing for the expression of human γ2 heavy chain Fd regions, was generated as follows. The human γ2 heavy chain Fd region was amplified from the plasmid pCMV-C-gamma2 using the primers C-gamma2-F (5'-GAG GAG CTC GAG GCC TCC ACC AAG GGC CCA TCG-3', SEQ ID NO:86) and Fd-gamma2-B (5'-GAG GAG GGT ACC TTA ATT AAT CAT TTG CGC TCA ACT GTC TTG TC-3', SEQ ID NO:87). The resulting 338 bp PCR product was digested with the restriction endonucleases XhoI and KpnI and cloned into the vector pCMV-gamma2-leader, yielding pCMV-Fd (SEQ ID NO:85). DNA fragments encoding heavy chain variable regions can be cloned into pCMV-Fd via the restriction endonucleases XhoI and ApaI and expressed as part of γ2 heavy chain Fd regions. Cotransfection of a pCMV-LC (SEQ ID NO:71) with a pCMV-Fd (SEQ ID NO:85) expression construct will allow for the production of Fab fragments.

pCMV-HC-gl (SEQ ID NO:88), a vector allowing for the expression of natural human antibody γ1 heavy chains, was generated as follows. The human γ1 heavy chain constant region was amplified from human bone marrow cDNA using the primers C-gamma1-F (5'-CAA GGG CCC ATC GGT CTT CCC CCT GGC ACC CTC-3', SEQ ID NO:89) and C-gamma2-B (SEQ ID NO:84). The resulting 1005 bp PCR product was digested with the restriction endonucleases ApaI and KpnI and used to replace the Fd coding region in pCMV-Fd (SEQ ID NO:85), yielding pCMV-HC-g1 (SEQ ID NO:88). DNA fragments encoding heavy chain variable regions can be cloned into pCMV-HC-g1 (SEQ ID NO:88) via the restriction endonucleases XhoI and ApaI and expressed as part of natural γ1 heavy chains. Cotransfection of a pCMV-LC (SEQ ID NO:71) with a pCMV-HC-g1 (SEQ ID NO:88) expression construct will allow for the production of whole IgG1.

pCMV-HC-g4 (SEQ ID NO:90), a vector allowing for the expression of natural human antibody γ4 heavy chains, was generated by nested PCR as follows. The human γ4 heavy chain constant region was pre-amplified from human spleen cDNA using the primers C-gamma2-F (SEQ ID NO:86) and C-gamma4-B2 (5'-AGC GGG GGC TTG CCG GCC CTG-3', SEQ ID NO:123). The resulting 1021bp PCR product was then reamplified with the primers C-gamma2-FL (SEQ ID NO:83) and C-gamma4-B (5'-GAG GAG GGT ACC TTA ATT AAC CGG CCC TGG CAC TCA TTT ACC CA-3', SEQ ID NO:91). The resulting 1029bp PCR product was digested with the restriction endonucleases Xho1 and Pac1 and used to replace the Fd coding region in pCMV-Fd (SEQ ID NO:85), yielding pCMV-HC-g4 (SEQ ID NO:90). DNA fragments encoding heavy chain variable regions can be cloned into pCMV-HC-g4 (SEQ ID NO:90) via the restriction endonucleases Xho1 and Apal and expressed as part of natural γ4 heavy chains. Cotransfection of a pCMV-LC (SEQ ID NO:71) or pCMV-LC-lambda (SEQ ID NO:110) with a pCMV-HC-g4 (SEQ ID NO:90) expression construct will allow for the production of whole IgG4.

### EXAMPLE 9

### Construction, expression, and purification of fully human Qβ-specific IgG and Fab.

The heavy and light chain variable region coding segments of scFv-Qb#2 (SEQ ID NO:8), scFv-Qb#3 (SEQ ID NO:9), scFv-Qb#5 (SEQ ID NO:11) and scFv-Qb#8 (SEQ ID NO:14) were amplified by PCR using variable region-specific transfer primers (SEQ ID NO:92-103). Specifically, the light chain variable regions were amplified as follows, wherein VL stands for lambda light chain variable region, VK stands for kappa light chain variable region, and VH stands for heavy chain variable region: VL-Qb#2 was amplified with the primers VL-SacI-F (SEQ ID NO:95) and VL-EcoR5-B1 (SEQ ID NO:100); VK-Qb#3 with the primers VK-SacI-F (SEQ ID NO:92) and VK-EcoR5-B2 (SEQ ID NO:94); VL-Qb#5 with the primers VL-SacI-F (SEQ ID NO:95) and VL-EcoR5-B2 (SEQ ID NO:101); VL-Qb#8 with the primers VL-SacI-F3 (SEQ ID NO:98) and VL-EcoR5-B2 (SEQ ID NO:101). The heavy chain variable region coding segments VH-Qb#2, VH-Qb#3, VH-Qb#5 and VH-Qb#8 were all amplified with the primers VH-XhoI-F (SEQ ID NO:102) and VH-ApaI-B (SEQ ID NO:103).

The resulting light chain variable region PCR products were digested with the restriction enzymes SacI and EcoR5, purified by agarose gel electrophoresis, and ligated into SacI-EcoR5 digested pCMV-LC (SEQ ID NO:71), yielding the light chain expression vectors pCMV-Qb#2-LC, pCMV-Qb#3-LC, pCMV-Qb#5-LC and pCMV-Qb#8-LC. Similarly, the heavy chain variable region PCR products were digested with the restriction enzymes XhoI and ApaI, gel purified, and ligated into Xhol-Apal digested pCMV-HC (SEQ ID NO:78), yielding the γ2 heavy chain expression vectors pCMV-Qb#2-HC, pCMV-Qb#3-HC, pCMV-Qb#5-HC and pCMV-Qb#8-HC, as well as into XhoI-ApaI digested pCMV-Fd (SEQ ID NO:85), yielding the γ2 Fd region expression vectors pCMV-Qb#2-Fd, pCMV-Qb#3-Fd, pCMV-Qb#5-Fd and pCMV-Qb#8-Fd.

As demonstrated in Example 8, co-expression of each of the pCMV-LC expression constructs with the corresponding pCMV-HC or pCMV-Fd expression construct in principle allows for the production of, respectively, whole IgG or Fab fragments. However, to increase yields and facilitate large-scale production of antibodies, heavy and light chain coding regions were first combined into a single, EBNA-based expression vector, pCB15 (SEQ ID NO:104). For instance, for expression of antibody Qb#2 as a whole IgG, the light chain coding region was excised from pCMV-Qb#2-LC by digestion with the restriction enzymes NheI and PmeI, the resulting 735 bp fragment purified by agarose gel electrophoresis, and then ligated into NheI-PmeI digested pCB15, yielding pCB15-Qb#2-LC. The Qb#2 heavy chain coding region was then excised from pCMV-Qb#2-HC by digestion with AscI and PacI, the resulting 1433 bp fragment gel-purified, and then ligated into AscI-PacI digested pCB15-Qb#2-LC, yielding the whole IgG expression vector pCB15-Qb#2-IgG2.

For expression as a Fab fragment, the Qb#2 Fd coding region was excised from pCMV-Qb#2-Fd by digestion with AscI and PacI, the resulting 758 bp fragment gel-purified, and then ligated into AscI-PacI digested pCB15-Qb#2-LC, yielding the Fab expression vector pCB15-Qb#2-Fab. The whole IgG expression vectors pCB15-Qb#3-IgG2, pCB15-Qb#5-IgG2 and pCB15-Qb#8-IgG2, and the Fab expression vectors pCB15-Qb#3-Fab, pCB15-Qb#5-Fab and pCB15-Qb#8-Fab were generated in exactly the same way as the Qb#2 expression vectors.

Expression of whole IgG and Fab fragments was done in HEK-293T cells, exactly as described for the scFv-Fc fusion proteins (Example 6), with expression levels in the range of 20 to 50 mg/L. Both whole IgG and Fab fragments were purified by applying protein-containing cell supernatants to affinity columns (IgG: protein G; Fab: goat anti-human F(ab')2). The columns were washed with 10 column volumes of phosphate-buffered saline (PBS), and bound protein eluted with 0.1 M Glycine pH 3.6. 1 ml fractions were collected in tubes containing 0.1 ml of 1 M Tris pH 7.5 for neutralization. Protein-containing fractions were analyzed by SDS-PAGE and pooled. The buffer was exchanged with PBS by dialysis using 10'000 MWCO Slide-A-Lyzer dialysis cassettes (Pierce). The purified proteins in PBS were then filtered through 0.22 µM Millex GV sterile filters (Millipore) and aliquoted. Working stocks were kept at 4 °C, whereas aliquots for long-term storage were flash-frozen in liquid nitrogen and kept -80 °C.

The binding properties of purified Qβ-specific IgG2 and Fab immunoglobulins were analyzed by ELISA essentially as described in Example 7. For most of the immunoglobulins, half-maximal binding was observed at picomolar concentrations, suggesting that they are of very high affinity (see Table 1).

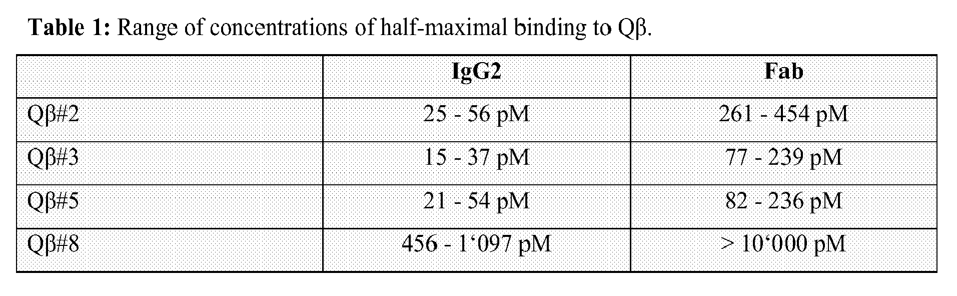

### EXAMPLE 10

### Isolation of human pathogen-specific human monoclonal antibodies: anti-preS1 antibodies

Peripheral blood mononuclear cells (PBMC) were isolated from 20 ml of heparinized blood of a fr-preS1 (21-47) (SEQ ID NO:124) vaccinated volunteer by a standard Ficoll-Hypaque^{™} Plus (Amersham Biosciences) gradient method. PBMC were stained with: (1) Qβ-preS1 (21-47) (1 µg/ml) in combination with a Alexa 488 nm-labeled Qβ-specific mouse mAb; (2) Alexa 647 nm-labeled Qβ (3 µg/ml); (3) PE-labeled mouse anti-human IgM (diluted 1:50; BD Biosciences Pharmingen), mouse anti-human IgD (diluted 1:100; BD Biosciences Pharmingen), mouse anti-human CD14 (diluted 1:50; BD Biosciences Pharmingen), and mouse anti-human CD3 (diluted 1:50; BD Biosciences Pharmingen) antibodies; and (4) PE-TexasRed-labeled mouse anti-human CD19 antibody (diluted 1:50; Caltag Laboratories). After staining, cells were washed, filtered and preS1 (21-47)-specific B cells (FL1-positive, FL2-negative, FL3-positive, FL4-negative) were sorted on a FACSVantage SE flow cytometer (Becton Dickinson).

Construction of a Sindbis-based scFv cell surface display library was done exactly as described in Example 3. Cells displaying preS1 (21-47)-specific scFv antibodies were isolated essentially as described in Example 4, using Qβ-preS1 (21-47) as a bait. A total of six preS1 (21-47)-specific antibodies were identified: A124, C032, E040, J058, L023 and L025. ScFv-Fc fusion proteins were cloned, expressed and purified as described in Examples 5 and 6. The binding properties of purified scFv-Fc fusion proteins were analyzed by ELISA essentially as described in Example 7. Half-maximal binding was observed at concentrations in the low nanomolar range (A124, 6.9 nM; C032, 5.6 nM; E040, 1.2 - 1.9 nM; J058, 7.5 nM; L023, 2.2 nM; and L025, 1.0 nM), suggesting that the antibodies are of high affinity. Antibodies A124, E040, J058, L023, and L025 were converted to whole human IgG2, and expressed and purified as described in Example 9.

### EXAMPLE 11

### Isolation of hapten-specific human monoclonal antibodies: anti-Nicotin antibodies

Peripheral blood mononuclear cells (PBMC) are isolated from 20 ml of heparinized blood of a Qβ-Nicotin-vaccinated volunteer by a standard Ficoll-HypaqueTM Plus (Amersham Biosciences) gradient method. PBMC are stained with: (1) Qβ-Nicotin (4 µg/ml) in combination with a Nicotin-specific mouse mAb and FITC-labeled goat anti-mouse antibody (Jackson ImmunoResearch Laboratories); (2) Alexa 647nm-labeled Qβ (4 µg/ml); (3) PE-labeled mouse anti-human IgM, IgD, CD14 and CD3 antibodies as described in Example 10; and (4) PE-TexasRed-labeled mouse anti-human CD19 antibody as described in Example 10. After staining, cells are washed, filtered and Nicotin-specific B cells (FL1-positive, FL2-negative, FL3-positive, FL4-negative) are sorted on a FACSVantage SE flow cytometer (Becton Dickinson).

Construction of a Sindbis-based scFv cell surface display library is done exactly as described in Example 3. Cells displaying Nicotin-specific scFv antibodies are isolated essentially as described in Example 4, using Qβ-Nicotin as a bait. Nicotin-specific antibodies are cloned, expressed and purified as described in Examples 5, 6 and 9.

### EXAMPLE 12

### Isolation of self antigen-specific human monoclonal antibodies: anti-ghrelin antibodies

Peripheral blood mononuclear cells (PBMC) are isolated from 20 ml of heparinized blood of a Qβ-ghrelin (24-31)-vaccinated volunteer by a standard Ficoll-HypaqueTM Plus (Amersham Biosciences) gradient method. PBMC are stained with: (1) Qβ-ghrelin (24-31) (4 µg/ml) in combination with a Alexa 488nm-labeled Qβ-specific mouse mAb; (2) Alexa 647nm-labeled Qβ (4µg/ml); (3) PE-labeled mouse anti-human IgM, IgD, CD14 and CD3 antibodies as described in Example 10; and (4) PE-TexasRed-labeled mouse anti-human CD19 antibody as described in Example 10. After staining, cells are washed, filtered and ghrelin (24-31)-specific B cells (FL1-positive, FL2-negative, FL3-positive, FL4-negative) are sorted on a FACSVantage SE flow cytometer (Becton Dickinson).

Construction of a Sindbis-based scFv cell surface display library is done exactly as described in Example 3. Cells displaying ghrelin (24-31)-specific scFv antibodies are isolated essentially as described in Example 4, using Qβ-ghrelin (24-31) as a bait. Ghrelin (24-31)-specific antibodies are cloned, expressed and purified as described in Examples 5, 6 and 9.

### EXAMPLE 13

### Isolation of allergen-specific human monoclonal antibodies: anti-Fel d1 antibodies

In principle, Fel d1-specific B cells can be isolated from a cat-allergic individual. Alternatively, Fel d1-specific B cells are isolated from a Fel d1-vaccinated volunteer. Thus, peripheral blood mononuclear cells (PBMC) are isolated from 20 ml of heparinized blood by a standard Ficoll-Hypaque^{™} Plus (Amersham Biosciences) gradient method. PBMC are stained with: (1) Qβ-Fe1 d1 (4 µg/ml) in combination with a Alexa 488nm-labeled Qβ-specific mouse mAb; (2) Alexa 647nm-labeled Qβ (3 µg/ml); (3) PE-labeled mouse anti-human IgM, IgD, CD 14 and CD3 antibodies as described in Example 10; and (4) PE-TexasRed-labeled mouse anti-human CD19 antibody as described in Example 10. After staining, cells are washed, filtered and Fel d1-specific B cells (FL1-positive, FL2-negative, FL3-positive, FL4-negative) are sorted on a FACSVantage SE flow cytometer (Becton Dickinson).

Construction of a Sindbis-based scFv cell surface display library is done exactly as described in Example 3. Cells displaying Fel d1-specific scFv antibodies are isolated essentially as described in Example 4, using Qβ-Fel d1 as a bait. Fel d1-specific antibodies are cloned, expressed and purified as described in Examples 5, 6 and 9.

## Claims

1. A method of isolating a cell expressing an antibody specifically binding an antigen of interest, said method comprising the steps of:
(a) providing a population of B-cells;
(b) selecting from said population of B-cells a sub-population of B-cells by selecting B-cells for their capability of specifically binding said antigen of interest;
(c) generating an expression library, wherein each member of said expression library encodes an antibody comprising at least one variable region (VR), by
(i) generating a multitude of DNA molecules, wherein said generating comprises the step of amplifying a pool of DNA molecules from said sub-population of B-cells, wherein each of said DNA molecules of said pool of DNA molecules encodes one of said at least one variable region (VR); and
(ii) cloning a specimen of said multitude of DNA molecules into an expression vector;
(d) introducing said expression library into a first population of eukaryotic cells;
(e) displaying antibodies of said expression library on the surface of said eukaryotic cells; and
(f) isolating from said first population of eukaryotic cells a cell, wherein said cell is selected for the capability of the antibody displayed on its surface of specifically binding said antigen of interest or a fragment or antigenic determinant thereof.

2. The method of claims 1, wherein each antibody encoded by said expression library further comprises a signal peptide and a transmembrane region.

3. The method of any one of claims 1 or 2, wherein said at least one VR comprised by said antibody is a heavy chain variable region (HCVR) and alight chain variable region (LCVR).

4. The method of any one of claims 1 to 3, wherein said generating an expression library comprises the steps of:
(a) generating a multitude of DNA molecules encoding antibodies, said generating comprising the steps of:
(i) amplifying from said sub-population of B-cells a first pool of DNA molecules encoding HCVRs;
(ii) amplifying from said sub-population of B-cells a second pool of DNA molecules encoding LCVRs; and
(iii) linking specimens of said first and of said second pool of DNA molecules to each other by a DNA encoding a linker region (LR);
(b) cloning a specimen of said multitude of DNA molecules into an expression vector;
wherein each member of said expression library encodes an antibody comprising a signal peptide, a HCVR, a LCVR and a transmembrane region, wherein said HCVR and said LCVR are linked to each other by said linker region.

5. The method of any one of claims 1 to 4, wherein said antibody specifically binding said antigen of interest is a single chain antibody.

6. The method of any one of claims 1 to 5, wherein said antibody specifically binding said antigen of interest is a humanized or human antibody, preferably a human antibody.

7. The method of any one of claims 1 to 6, wherein said generating a multitude of DNA molecules comprises the steps of:
(a) isolating RNA from said sub-population of B-cells;
(b) transcribing said RNA to cDNA; and
(c) amplifying from said cDNA a pool of DNA molecules using a mixture of oligonucleotides comprising at least two oligonucleotides capable of amplifying VR coding regions.

8. The method of any one of claims 1 to 7, wherein said generating a multitude of DNA molecules comprises the steps of:
(a) isolating RNA from said sub-population of B-cells;
(b) transcribing said RNA to cDNA;
(c) amplifying from said cDNA said first pool of DNA molecules using a first mixture of oligonucleotides comprising at least two oligonucleotides capable of amplifying HCVR coding regions;
(d) amplifying from said cDNA said second pool of DNA molecules using a second mixture of oligonucleotides comprising at least two oligonucleotides capable of amplifying LCVR coding regions; and
(e) linking specimens of said first and said second pool of DNA molecules to each other by a DNA encoding said linker region.

9. The method of claim 8 wherein a first part of said linker region is encoded by an oligonucleotide contained in said first mixture of oligonucleotides and wherein a second part of said linker region is encoded by an oligonucleotide contained in said second mixture of oligonucleotides, wherein preferably said oligonucleotide encoding said first part of said linker region and said oligonucleotide encoding said second part of said linker region comprise an overlap, wherein further preferably said overlap is at least 3, preferably at least 10, more preferably at least 20, and most preferably 24 nucleotides in length, wherein still further preferably said overlap is at most 50, preferably at most 40, more preferably at most 30, and most preferably at most 24 nucleotides in length.

10. The method of any one of claims 7 to 9, wherein said transcribing of said RNA to cDNA comprises the steps of pre-amplifying the transcriptome of said sub-population of B-cells, wherein preferably said pre-amplifying comprises the steps of:
(a) selectively transcribing polyadenylated mRNA contained in said RNA to single stranded cDNA; and
(b) amplifying double stranded cDNA from said single stranded cDNA.

11. The method of claim 10, wherein (a) said selectively transcribing is performed using the oligonucleotides of SEQ ID NO:32 and SEQ ID NO:33; and (b) wherein said amplifying double stranded cDNA is performed using the oligonucleotide of SEQ ID NO:34.

12. The method of any one of claims 4 to 11, wherein said linker region consists of 5 to 30, preferably 5 to 22, more preferably 5 to 20, and most preferably of 18 amino acids.

13. The method of any one of claims 4 to 12, wherein said linker region comprises or preferably consists of SEQ ID NO:107.

14. The method of any one of claims 7 to 13, wherein said mixture of oligonucleotides, preferably said first mixture of oligonucleotides comprises at least two, preferably all, oligonucleotides selected from the group consisting of SEQ ID NO:42 to 48.

15. The method of any one claims 7 to 14, wherein said mixture of oligonucleotides, preferably said second mixture of oligonucleotides, comprises at least two oligonucleotides capable of amplifying kappa LCVR coding regions, wherein further preferably said mixture of oligonucleotides, preferably said second mixture of oligonucleotides, comprises at least two, preferably all, oligonucleotides selected from the group consisting of SEQ ID NO:49 to 56.

16. The method of any one claims 7 to 14, wherein said mixture of oligonucleotides, preferably said second mixture of oligonucleotides, comprises at least two oligonucleotides capable of amplifying lambda LCVR coding regions, wherein further preferably said mixture of oligonucleotides, preferably said second mixture of oligonucleotides, comprises at least two, preferably all, oligonucleotides selected from the group consisting of SEQ ID NO:57 to 68.

17. The method of any one of claims 7 to 16, wherein said mixture of oligonucleotides, said first mixture of oligonucleotides or said second mixture of oligonucleotides comprise a total amount of primers capable of amplifying VR coding regions, wherein all forward primers and all reverse primers contained in said total amount are in an equimolar ratio.

18. The method of any one of claims 1 or 2, wherein each member of said expression library encodes an antibody comprising exactly one VR and a transmembrane region, wherein preferably said exactly one VR is a HCVR.

19. The method of any one of claims 1 to 18 wherein said antibody encoded by said expression library comprises said HCVR, said LCVR and said linker region (LR), in an order selected from:
(a) LCVR-LR-HCVR; and
(b) HCVR-LR-LCVR;
wherein preferably said order is LCVR-LR-HCVR.

20. The method of any of claims 4 to 19, wherein said linking of said specimens of said first pool of DNA molecules and of said second pool of DNA molecules to each other is performed by PCR using the oligonucleotides depicted in SEQ ID NO:69 and SEQ ID NO:70 as primers.

21. The method of any one of claims 1 to 20, wherein said cloning a specimen of said multitude of DNA molecules into an expression vector comprises the steps of:
(a) generating a DNA construct encoding said antibody comprising a HCVR, a LCVR and a transmembrane region by linking a specimen of said multitude of DNA molecules to a first DNA element encoding said transmembrane region; and
(b) functionally linking said DNA construct to a second DNA element encoding a signal peptide directing said antibody to the secretory pathway, wherein preferably said functionally linking is performed in such a way that said signal peptide is linked to the N-terminus of said antibody.

22. The method of any one of claims 1 to 21, wherein said transmembrane region is derived from human PDGFR beta chain, and wherein preferably said transmembrane region comprises or further preferably consists of SEQ ID NO:106.

23. The method of any one of claims 21 or 22, wherein said signal peptide is a mouse Ig kappa light chain signal peptide, and wherein preferably said signal peptide comprises or further preferably consists of SEQ ID NO:105.

24. The method of any one of claims 1 to 23, wherein said antibody further comprises a detection tag, wherein preferably said detection tag is HA, and wherein further preferably said detection tag comprises or still further preferably consists of SEQ ID NO:108.

25. The method of any one of claims 1 to 24, wherein said expression library is a viral expression library, preferably an alphaviral expression library, more preferably an alphaviral expression library, wherein still further preferably said alphaviral expression library is derived from an alphavirus selected from the group of:
(a) Sindbis virus;
(b) Semliki forest virus; and
(c) Venezuelan equine encephalitis virus.

26. The method of claim 25, wherein said alphaviral expression library is derived from Sindbis virus.

27. The method of any one of claims 1 to 26, wherein said expression vector is a viral expression vector, wherein preferably said viral expression vector is an alphaviral expression vector, wherein further preferably said alphaviral expression vector is derived from Sindbis virus.

28. The method of any one of claims 1 to 27, wherein said expression vector comprises nucleotides 4 to 282 of SEQ ID NO:1.

29. The method of any one of claims 1 to 27, wherein said expression vector comprises nucleotides 4 to 312 of SEQ ID NO:40.

30. The method of any one of claims 1 to 29, wherein said expression vector is an alphaviral expression vector derived from Sindbis virus, wherein said alphaviral expression vector comprises or preferably consists of pDe1-SP-TM (SEQ ID NO:38).

31. The method of any one of claims 1 to 29, wherein said expression vector is an alphaviral expression vector derived from Sindbis virus, wherein said alphaviral expression vector comprises or preferably consists of pDe1-SP-HA-TM (SEQ ID NO:39).

32. The method of any one of claims 1 to 29, wherein said population of B-cells is derived from an animal exhibiting an increased titer of antibodies specifically binding said antigen of interest.

33. The method of claim 32, wherein said titer is at least 5 times, preferably at least 10 times, most preferably at least 20 times higher than in the average population of said animal.

34. The method of any one of claims 32 or 33, wherein said animal is or has been exposed to said antigen of interest or to a fragment or antigenic determinant thereof.

35. The method of any one of claims 32 to 34, wherein said animal is or has been infected by a pathogen, wherein said pathogen comprises said antigen of interest or a fragment or antigenic determinant thereof.

36. The method of any one claims 1 to 35, wherein said population of B-cells is derived from an animal immunized with an immunogenic composition, wherein said immunogenic composition comprises or alternatively consists of:
(a) said antigen of interest;
(b) a fragment of said antigen of interest; and
(c) an antigenic determinant of said antigen of interest.

37. The method of any one of claims 1 to 36, wherein said population of B-cells is derived from a source selected from:
(a) blood;
(b) secondary lymphoid organs, preferably spleen or lymph node;
(c) bone marrow; and
(d) tissue comprising memory B-cells.

38. The method of claim 37, wherein said source is blood.

39. The method of any one of claims 32 to 38, wherein said animal is selected from the group consisting of:
(a) human;
(b) mouse;
(c) rabbit; and
(d) chicken.

40. The method of any one of claims 32 to 38, wherein said animal is a mammal, preferably a humanized mouse or a human, most preferably a human.

41. The method of any one claims 1 to 40, wherein said population of B-cells comprises or preferably consists of peripheral blood mononuclear cells (PBMCs).

42. The method of any one of claims 1 to 41, wherein said selecting from said population of B-cells a sub-population of B-cells comprises the steps of:
(a) contacting said population of B-cells with said antigen of interest or a fragment or antigenic determinant thereof; and
(b) selecting B-cells specifically binding said antigen of interest or fragment or antigenic determinant thereof.

43. The method of any one of claims 1 to 42, wherein said selecting from said population of B-cells a sub-population of B-cells comprises the steps of:
(a) coating a carrier with said antigen of interest or fragment or antigenic determinant thereof;
(b) contacting said population of B-cells with said carrier and allowing said B-cells to bind to said carrier via said antigen of interest or fragment or antigenic determinant thereof; and
(c) removing unbound B-cells.

44. The method of claim 43, wherein said carrier comprises or preferably consists of beads, wherein further preferably said beads are paramagnetic beads.

45. The method of any one of claims 1 to 42, wherein said selecting from said population of B-cells a sub-population of B-cells comprises the steps of:
(a) contacting said population of B-cells with said antigen of interest or fragment or antigenic determinant thereof, wherein said antigen of interest or fragment or antigenic determinant thereof is labeled with a fluorescence dye; and
(b) separating B-cells bound to said antigen of interest or fragment or antigenic determinant thereof by FACS sorting.

46. The method of any one of claims 1 to 42, wherein said selecting from said population of B-cells a sub-population of B-cells comprises the steps of:
(a) contacting said population of B-cells with said antigen of interest or fragment or antigenic determinant thereof, wherein said antigen of interest or fragment or antigenic determinant thereof is labeled with a first fluorescence dye;
(b) contacting the cells of said population of B-cells with anti-IgM and/or anti-IgD antibodies, wherein said anti-IgM and/or anti-IgD antibodies are labeled with a second fluorescence dye; and
(c) selecting B-cells bound to said antigen of interest or fragment or antigenic determinant thereof but not bound to said anti-IgM and/or not bound to said anti-IgD antibodies by FACS sorting.

47. The method of any one of claims 1 to 46, wherein said expression library is a viral expression library, preferably an alphaviral expression library, and wherein said introducing said expression library into a first population of eukaryotic cells is performed by infecting said eukaryotic cell with said expression library, wherein preferably said infecting is performed at a multiplicity of infection of at most 10, preferably at most 1, more preferably at most 0.2, and most preferably at most 0.1.

48. The method of any one of claims 1 to 47, wherein said isolating of said individual cell is performed by FACS sorting.

49. The method of any one of claims 1 to 48, wherein said isolating of said cell comprises the steps of:
(a) staining said first population of eukaryotic cells with said antigen of interest or fragment or antigenic determinant thereof, wherein said antigen of interest or fragment or antigenic determinant thereof is labeled with a fluorescence dye; and
(b) separating an individual cell specifically binding said antigen of interest, or fragment or antigenic determinant thereof, by means of FACS sorting.

50. The method of claim 49, further comprising the steps of:
(a) cultivating at least one, preferably exactly one, of said individual cell in the presence of a second population of eukaryotic cells;
(b) verifying the capability of said second population of eukaryotic cells of specifically binding said antigen of interest, or fragment or antigenic determinant thereof.

51. The method of claim 50, wherein said verifying comprises the steps of:
(a) staining said second population of eukaryotic cells with said antigen of interest, or fragment or antigenic determinant thereof, wherein said antigen of interest, or fragment or antigenic determinant thereof, is labeled with a fluorescence dye; and
(b) detecting cells specifically binding said antigen of interest, or fragment or antigenic determinant thereof, by FACS analysis.

52. The method of any one of claims 1 to 51, wherein said first population of eukaryotic cells and/or, preferably and, said second population of eukaryotic cells comprises or preferably consists of cells selected from:
(a) BHK 21 cells;
(b) Neuro-2a cells; and
(c) 293T cells.

53. The method of any one of claims 1 to 52, wherein said first population of eukaryotic cells and/or, preferably and, said second population of eukaryotic cells comprises or preferably consists of BHK 21 cells.

54. The method of any one of claims 1 to 53 wherein said antigen of interest is selected from the group consisting of:
(a) allergen;
(b) self-antigen;
(c) tumor antigen;
(d) antigen of a pathogen; and
(e) haptene.

55. The method of any one of claims 1 to 54, wherein said antigen of interest is an allergen, preferably an allergen selected from the group consisting of:
(a) pollen allergen, preferably Bet v I (birch pollen allergen);
(b) house dust allergen, preferably Der p I (House dust mite allergen);
(c) cat allergen, preferably Fel d1;
(d) bee venom phospholipase A2;
(e) 5 Dol m V (white-faced hornet venom allergen); and
(f) and an immunogenic fragments of (a) to (e).

56. The method of any one of claims 1 to 54, wherein said antigen of interest is a self-antigen, preferably a self-antigen selected from the group consisting of:
(a) IL-6;
(b) granulocyte macrophages colony stimulating factor (GMCSF);
(c) IL-1 alpha;
(d) IL-1 beta;
(e) IL-5;
(f) IL-15;
(g) IL-23;
(h) TNF alpha;
(i) RANKL;
(j) Ghrelin;
(k) GIP;
(l) adiponectin receptor; and
(m) amyloid beta.

57. The method of any one of claims 1 to 54, wherein said antigen of interest is a tumor antigen, wherein preferably said tumor antigen is selected from the group consisting of:
(a) MelanA;
(b) HER2 / ErbB-2 (breast cancer);
(c) GD2 (neuroblastoma);
(d) EGF-R (malignant glioblastoma);
(e) CEA (medullary thyroid cancer);
(f) CD52 (leukemia);
(g) human melanoma protein gp 100;
(h) tyrosinase and tyrosinase related proteins, preferably TRP-1 and TRP-2;
(i) NA 17-A nt protein;
(j) MAGE-3 protein; and
(k) NY-ESO-1.

58. The method of any one of claims 1 to 54, wherein said antigen of interest is an antigen of a pathogen, wherein preferably said pathogen is selected from the group consisting of:
(a) hepatitis B virus;
(b) influenza A virus;
(c) HIV;
(d) Hepatitis C virus;
(e) rotavirus;
(f) polio virus;
(g) encephalitis virus;
(h) West-Nile virus;
(i) SARS virus;
(j) Ebola virus;
(k) Measles virus;
(l) RSV;
(m) Toxoplasma;
(n) Plasmodium falciparum;
(o) Plasmodium ovale;
(p) Plasmodium malariae; and
(q) Chlamydia.

59. The method of claim 58, wherein said antigen of interest is selected from the group consisting of:
(a) hepatitis B virus preS1;
(b) influenza A virus M2 protein; and
(b) influenza A virus HA protein.

60. A method of producing an antibody specifically binding an antigen of interest said method comprising the steps of:
(a) isolating a cell expressing an antibody according to the method of claims 1-59;
(b) obtaining RNA from said isolated cell;
(c) synthesizing cDNA encoding said antibody from said RNA;
(d) cloning said cDNA into an expression vector;
(e) generating a fusion construct encoding a fusion product comprising said antibody and said purification tag;
(e) expressing said fusion construct in a cell; and
(f) purifying said fusion protein.

61. The method of claim 60, wherein said synthesizing of said cDNA comprises the step of synthesizing single stranded cDNA from said RNA, wherein preferably said single stranded cDNA is synthesized using SEQ ID NO:35 as a primer.

62. The method of claim 61, wherein said synthesizing of said cDNA further comprises the step of amplifying said cDNA from said single stranded cDNA, wherein preferably said amplifying is performed using the oligonucleotides of SEQ ID NO:35 and SEQ ID NO:36 as primers.

63. The method of any one of claims 60 to 62, wherein said purification tag is Fc, preferably human Fc, and wherein further preferably said purification tag comprises or still further preferably consists of SEQ ID NO:109.

64. The method of any one of claims 60 to 63, wherein said expression vector is pCEP-SP-Sfi-Fc (SEQ ID NO:37).

65. The method of any one of claims 60 to 64, wherein said expressing of said fusion product is performed in HEK-293T cells.

66. A method of producing an antibody specifically binding an antigen of interest, said method comprising the steps of:
(a) isolating a cell expressing an antibody according to the method of any one of claims 1 to 59;
(b) obtaining RNA from said cell;
(c) synthesizing cDNA form said RNA;
(d) amplifying from said cDNA a DNA encoding VRs of said antibody expressed by said cell;
(e) generating an expression construct comprising said DNA, wherein said expression construct is encoding at least one VR of said antibody expressed by said cell;
(h) expressing said expression construct in a cell.

67. The method of claim 66, said method comprising the steps of:
(a) isolating a cell expressing an antibody according to the method of any one of claims 1 to 59;
(b) obtaining RNA from said cell;
(c) synthesizing cDNA form said RNA;
(d) amplifying from said cDNA a first DNA encoding a HCVR of said antibody expressed by said cell;
(e) generating a first expression construct comprising said first DNA, wherein said first expression construct is encoding a heavy chain immunoglobulin comprising a heavy chain constant region (HCCR) and said HCVR;
(f) amplifying from said cDNA a second DNA encoding a LCVR of said antibody expressed by said cell;
(g) generating a second expression construct comprising said second DNA, wherein said second expression construct is encoding a light chain immunoglobulin comprising a light chain constant region (LCCR) and said LCVR;
(h) expressing said first expression construct and said second expression construct in a cell.

68. The method of any one of claim 66 or 67, wherein said expression construct, said first expression construct and/or said second expression construct are further encoding a hydrophobic leader sequence, preferably a human hydrophobic leader sequence.

69. The method of claim 67, wherein said first expression construct is further encoding a human heavy chain hydrophobic leader sequence.

70. The method of any one of claims 67 or 69, wherein said second expression construct is further encoding a human light chain hydrophobic leader sequence, wherein said human light chain hydrophobic leader sequence is selected from the group consisting of (a) human kappa light chain hydrophobic leader sequence; and (b) human lambda light chain hydrophobic leader sequence.

71. The method of any one of claims 66 to 70, wherein said synthesizing of said cDNA comprises the step of synthesizing single stranded cDNA from said RNA, wherein preferably said single stranded cDNA is synthesized using SEQ ID NO:35 as a primer.

72. The method of any one of claims 66 to 71, wherein said synthesizing of said cDNA further comprises the step of amplifying said cDNA from said single stranded cDNA, wherein preferably said amplifying is performed using the oligonucleotides of SEQ ID NO:35 and SEQ ID NO:36 as primers.

73. The method of any one of claims 67 to 72, wherein said HCCR is a human HCCR, preferably a human HCCR selected from the group consisting of:
(a) human gamma 1 HCCR;
(b) human gamma 2 HCCR;
(c) human gamma 4 HCCR; and
(d) human heavy chain Fd regions, preferably gamma 2 Fd region.

74. The method of any one of claims 67 to 73, wherein said LCCR is a human LCCR, preferably a human LCCR selected from the group consisting of:
(a) human kappa LCCR; and
(b) human lambda LCCR.

75. The method of any one of claims 67 to 74, wherein said amplifying of said first DNA is performed with HCVR specific primers, wherein preferably said HCVR specific primers are SEQ ID NO:102 and SEQ ID NO:103.

76. The method of any one of claims 67 to 75, wherein said amplifying of said second DNA is performed with LCVR specific primers, wherein preferably said LCVR specific primers are selected from kappa LCVR specific primers and lambda LCVR specific primers.

77. The method of claim 76, wherein said LCVR specific primers are kappa LCVR specific primers, wherein preferably said kappa LCVR specific primers are a combination of SEQ ID NO:92 with any one of SEQ ID NO:93 or 94.

78. The method of claim 77, wherein said LCVR specific primers are lambda LCVR specific primers, wherein preferably said lambda LCVR specific primers are a combination of any one selected from SEQ ID NO:95 to 99 with any one of SEQ ID NO:100 or 101.

79. The method of any one of claims 67 to 78, wherein said LCCR is a human kappa LCCR and wherein said LCVR is a human kappa LCVR.

80. The method of any one of claims 67 to 78, wherein said LCCR is a human lambda LCCR and wherein said LCVR is a human lambda LCVR.

81. The method of any one of claims 67 to 80, wherein said expressing of said first expression construct and of said second expression construct comprises expressing said first expression construct as part of a first expression vector and expressing said second expression construct as part of a second expression vector, wherein said first expression vector and said second expression vector are co-transfected to said cell.

82. The method of any one of claims 67 to 81, wherein said expressing of said first expression construct and of said second expression construct comprises expressing said first expression construct and said second expression construct as part of the same expression vector.

83. The method of claim 82, wherein said expression vector is pCB15 (SEQ ID NO:104).

84. The method of any one of claims 67 to 83, wherein said generating said first expression construct comprises the step of cloning said first DNA into a first expression cassette, wherein said first expression cassette is encoding said HCCR, and, preferably, said HCCR hydrophobic leader sequence, wherein further preferably said first expression cassette comprises or still more preferably consists of a sequence selected from SEQ ID NO:117 to 120.

85. The method of any one of claims 67 to 84, wherein said generating said second expression construct comprises the step of cloning said second DNA into a second expression cassette, wherein said second expression cassette is encoding said LCCR, and, preferably, said LCCR hydrophobic leader sequence, and wherein further preferably said second expression cassette comprises or still more preferably consists of a sequence selected from SEQ ID NO:121 or 122.

86. The method of claim 85, wherein said antibody is a Fab fragment and wherein preferably said first expression cassette comprises or preferably consists of SEQ ID NO:120 and wherein further preferably said second expression cassette comprises or preferably consists of SEQ ID NO:121.

87. The method of claim 86, wherein said first expression vector comprises or preferably consists of SEQ ID NO:85 and wherein said second expression vector comprises or preferably consists of said SEQ ID NO:71.

88. The method of claim 85, wherein said antibody is a Fab fragment and wherein preferably said first expression cassette comprises or preferably consists of SEQ ID NO:120 and wherein further preferably said second expression cassette comprises or preferably consists of SEQ ID NO:122.

89. The method of claim 88, wherein said first expression vector comprises or preferably consists of SEQ ID NO:85 and wherein said second expression vector comprises or preferably consists of said SEQ ID NO:110.

90. The method of claim 85, wherein said antibody is a whole antibody selected from the group consisting of (a) IgG, preferably selected from IgG1, IgG2, IgG3 and IgG4; (b) IgA, (c) IgE, (d) IgM, and (e) IgD.

91. The method of claim 90, wherein said antibody is a IgG1, and wherein preferably said first expression cassette comprises or preferably consists of SEQ ID NO:118 and wherein further preferably said second expression cassette comprises or preferably consists of SEQ ID NO:121.

92. The method of claim 91 wherein said first expression vector comprises or preferably consists of SEQ ID NO:88 and wherein said second expression vector comprises or preferably consists of said SEQ ID NO:71.

93. The method of claim 90, wherein said antibody is a IgG1, and wherein preferably said first expression cassette comprises or preferably consists of SEQ ID NO:118 and wherein further preferably said second expression cassette comprises or preferably consists of SEQ ID NO:122.

94. The method of claim 93, wherein said first expression vector comprises or preferably consists of SEQ ID NO:88 and wherein said second expression vector comprises or preferably consists of said SEQ ID NO:110.

95. The method of claim 90, wherein said antibody is a IgG2, and wherein preferably said first expression cassette comprises or preferably consists of SEQ ID NO:117 and wherein further preferably said second expression cassette comprises or preferably consists of SEQ ID NO:121.

96. The method of claim 95, wherein said first expression vector comprises or preferably consists of SEQ ID NO:78 and wherein said second expression vector comprises or preferably consists of said SEQ ID NO:71.

97. The method of claim 90, wherein said antibody is a IgG2, and wherein preferably said first expression cassette comprises or preferably consists of SEQ ID NO:117 and wherein further preferably said second expression cassette comprises or preferably consists of SEQ ID NO:122.

98. The method of claim 97, wherein said first expression vector comprises or preferably consists of SEQ ID NO:78 and wherein said second expression vector comprises or preferably consists of said SEQ ID NO:110.

99. The method of claim 90, wherein said antibody is a IgG4, and wherein preferably said first expression cassette comprises or preferably consists of SEQ ID NO:119 and wherein further preferably said second expression cassette comprises or preferably consists of SEQ ID NO:121.

100. The method of claim 99, wherein said first expression vector comprises or preferably consists of SEQ ID NO:90 and wherein said second expression vector comprises or preferably consists of said SEQ ID NO:71.

101. The method of claim 90, wherein said antibody is a IgG4, and wherein preferably said first expression cassette comprises or preferably consists of SEQ ID NO:119 and wherein further preferably said second expression cassette comprises or preferably consists of SEQ ID NO:122.

102. The method of claim 101, wherein said first expression vector comprises or preferably consists of SEQ ID NO:90 and wherein said second expression vector comprises or preferably consists of said SEQ ID NO:71.

103. The method of any one of claims 66 to 102, wherein said expressing of said antibody is performed in HEK-293T cells.
